# EUROPEAN PATENT APPLICATION

(11) **EP 4 653 444 A1**
(43) Date of publication of application: **26.11.2025**
(21) Application number: 24744417.7
(22) Date of filing: 19.01.2024
(51) Int. Cl.: C07D 498/04, A61K 31/5383, A61P 35/00, A61P 31/12

(54) **FUSED TRICYCLIC COMPOUND AS KINASE INHIBITOR**

(30) Priority: 19.01.2023 CN 202310060105
(71) Applicant: Hangzhou Innogate Pharma Co., Ltd., Hangzhou, Zhejiang 311121 (CN)
(72) Inventor: ZHANG, Hancheng, Hangzhou, Zhejiang 311121 (CN); CAI, Congcong, Hangzhou, Zhejiang 311121 (CN)
(74) Representative: Lavoix
(86) International application number: PCT/CN2024/073374
(87) International publication number: WO 2024/153248

(57) **Abstract**

Provided in the present ivention is a class of compounds containing a fused tricyclic structure Specifically, provided in the present invention is a compound having a structure as shown in formula (I), or anoptical isomer, a pharmaceutically acceptable salt, a prodrug, a deuterated derivative, a hydrate or a solvate thereofThe compound can effectively inhibit protein kinase (including ULK, FAK, ALK, CDK7, HPKI, AXL, FLT3TNK1, etc.), and is used for treating or preventing diseases or conditionSR^{e}lated to the activity or expression levelof kinase.

## Description

### Technical Field

The present invention relates to the field of medicinal chemistry. Specifically, the present invention relates to a class of novel compounds, their synthesis methods, and their application as kinase inhibitors in the preparation of medicaments for treating multiple diseases such as tumors and related diseases.

### Background

Autophagy is a lysosome-mediated process for the clearance of protein aggregates and damaged organelles, playing a critical role in intracellular macromolecular and organelle quality control. Kinases are key components involved in the initiation of autophagy. Currently, targeting specific kinases to modulate autophagy has emerged as a therapeutic strategy for various human diseases, particularly cancer, as autophagy is often upregulated in tumor cells and tissues. Notably, cancers harboring RAS mutations are highly dependent on autophagy. ULK1 (Unc-51 Like Autophagy Activating Kinase 1) is a serine/threonine kinase that serves as a key initiator of autophagy. Under nutrient-rich conditions, mTORC1 is activated and phosphorylates ULK1 and ATG13, thereby suppressing autophagy. In contrast, under nutrient deprivation, mTORC1 activity at the lysosomal membrane is inhibited, leading to rapid dephosphorylation of ULK1 and ATG13. ULK1 subsequently forms an autophagy-initiating complex with ATG13, FIP300, and ATG101 to promote autophagy initiation. Additionally, ULK1 phosphorylates ATG9 at serine 14 to facilitate the translocation of ATG9-containing vesicles to the autophagosome formation site. ULK1 also modulates autophagy by phosphorylating other substrates such as AMBRA1 and Raptor. Inactive mutants of ULK kinase block autophagy initiation, indicating that small-molecule inhibitors targeting ULK kinase activity can effectively regulate autophagy.

FAK (Focal Adhesion Kinase 1) is a non-receptor tyrosine kinase closely associated with the development and progression of various solid tumors, including ovarian, pancreatic, and breast cancers. FAK undergoes a conformational change upon integrin-mediated interaction with the extracellular matrix, resulting in autophosphorylation at tyrosine 397, which activates its kinase activity. Aberrant activation of FAK contributes to tumor cell survival, drug resistance, immunosuppression, and angiogenesis. Recent studies have also suggested that FAK inhibitors may be effective in treating pulmonary fibrosis.

ALK (Anaplastic Lymphoma Kinase) was originally identified in anaplastic large-cell lymphoma. Normal ALK is a classical receptor tyrosine kinase consisting of an extracellular ligand-binding domain, a transmembrane domain, and an intracellular tyrosine kinase domain. Its activation is typically ligand-dependent. Various genetic alterations of ALK, including point mutations, deletions, and especially gene fusions, have been identified in multiple cancers. A prominent example is the EML4-ALK fusion commonly observed in non-small cell lung cancer (NSCLC), resulting in constitutive activation of the ALK kinase domain and subsequent tumorigenesis. Thus, ALK represents a promising therapeutic target for cancer treatment.

CDK7 (Cyclin-Dependent Kinase 7) is a unique member of the cyclin-dependent kinase family. CDK7 forms the CDK-activating kinase (CAK) complex with Cyclin H and MAT1, which phosphorylates and activates other CDKs involved in cell cycle regulation, such as CDK2, CDK4, and CDK6. On the other hand, CDK7 phosphorylates serine residues at positions 5 and 7 within the C-terminal domain (CTD) of RNA polymerase II, promoting transcription initiation. CDK7 is frequently overexpressed in various cancers, and its inhibition has been shown to impair tumor cell proliferation.

HPK1 (Hematopoietic Progenitor Kinase 1), also known as MAP4K1 (Mitogen-Activated Protein Kinase Kinase Kinase Kinase 1), is a negative regulator of immune responses in T cells and other immune cells. It is predominantly expressed in hematopoietic cells, such as T cells, B cells, macrophages, dendritic cells, and mast cells. It belongs to the Ste20 family of serine/threonine kinases, which includes MAP4K1/HPK1, MAP4K2/GCK, MAP4K3/GLK, MAP4K4/HGK, MAP4K5/KHS, and MAP4K6/MINK. HPK1 functions as a tissue-specific upstream activator of the MEKK/JNK/SAPK signaling pathway.

HPK1 contains an N-terminal kinase domain, a central proline-rich region, and a citron homology domain. Upon T cell receptor (TCR) activation, cytoplasmic HPK1 iSR^{e}cruited to the plasma membrane and phosphorylated at Y381, S171, and T165, leading to full activation. Activated HPK1 phosphorylates downstream adaptor proteins, such as SLP76 at S376 and Gads at T254, promoting the recruitment of the negative regulator 14-3-3 proteins, which disrupt the TCR signal complex (Lat-Gads-SLP76) and inhibit downstream MAPK signaling required for T cell activation and proliferation. Besides TCR signaling pathway, HPK1 can also inhibit T cell signaling pathway by negatively regulating PKA-dependent PGE2 (Prostaglandin E2) receptors. HPK1 kinase activity can also be activated by B cell receptors (BCRs) and transforming growth factor receptors, thereby suppressing multiple immune cell functions.

HPK1 knockout mice exhibit enhanced T cell responses, reduced activation thresholds, and improved dendritic cell antigen presentation. Due to its immunosuppressive effects on immune cells like T cells, B cells, and dendritic cells, inhibition of HPK1 can potentiate immune responses, making HPK1 an attractive target for anti-tumor and antiviral therapies.

AXL (from the Greek word *anexelekto*) is a receptor tyrosine kinase belonging to the TAM family, which includes AXL, Tyro3 and MER. Initially identified as a transforming gene in chronic myelogenous leukemia (CML), AXL's ligand is the vitamin K-dependent growth factor GAS6. Downstream signaling of AXL includes the AXL-PI3K-AKT, RAS-RAF-MEK, and FAK-SRC pathways. Activated AXL pathways play important roles in cell proliferation, survival, migration, and the formation of an immunosuppressive tumor microenvironment. AXL signaling is hyperactivated in various solid and hematologic malignancies, particularly in acute myeloid leukemia (AML).

FLT3 (Fms-like Tyrosine Kinase-3) is a class III receptor tyrosine kinase expressed in hematopoietic progenitor cells. Upon ligand binding, FLT3 undergoes dimerization or autophosphorylation, activating downstream JAK-STAT, PI3K, and MAPK pathways that promote tumor cell proliferation or inhibit tumor cell apoptosis. FLT3 is highly expressed in most AML cases, with mutations observed in approximately 30% of patients, including internal tandem duplications (ITDs) and point mutations in the kinase domain. Both types of mutations lead to constitutive activation of FLT3 signaling, contributing to tumorigenesis and progression.

TNK1 (Thirty-eight Negative Kinase 1) is a non-receptor tyrosine kinase widely expressed in fetal tissues and restricted in adult tissues such as prostate, testis, ovary, colon, and small intestine. MARK-mediated phosphorylation of TNK1 at serine 502 promotes interaction between TNK1 and 14-3-3 proteins, resulting in sequestration and inhibition of TNK1 kinase activity. Conversely, release from 14-3-3 binding promotes TNK1 aggregation and activation. Genome-wide CRISPR screens have identified TNK1 as a top sensitizing factor in chemotherapy for pancreatic cancer and myeloma. In the Hodgkin lymphoma cell line L540, a constitutively active C-terminal truncated form of TNK1 is essential for cell proliferation and survival. TNK1 also plays a key role in promoting in vitro proliferation of hematologic tumor samples from primary patient specimens.

In summary, the development of novel kinase inhibitors targeting ULK, FAK, ALK, CDK7, HPK1, AXL, FLT3, or TNK1 holds significant therapeutic potential.

### Summary of the Invention

The object of the present invention is to provide a novel class of kinase inhibitors.

In a first aspect, the present invention provides a compound having a structure represented by Formula (I), or an optical isomer, pharmaceutically acceptable salt, prodrug, deuterated derivative, hydrate, or solvate thereof: 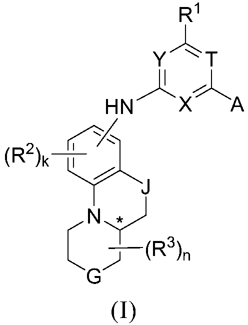
wherein the A is selected from Formula (IIa), Formula (IIb), Formula (IIc), Formula (IId) and Formula (IIe): 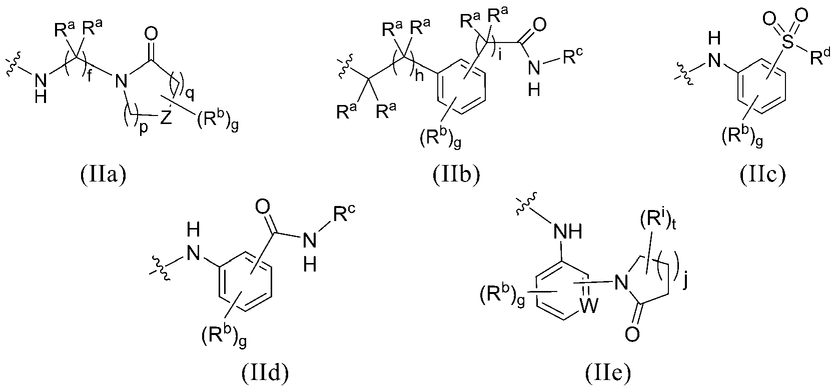
wherein in Formula (Ia), Formula (Ib), and Formula (Ic), " 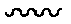 " represents the point which attach to other site of Formula (I);
"*" represents the chiral center;
X, Y and T are each independently N or CR¹;
each R¹ is independently selected from hydrogen, halogen, C₁₋₄ alkyl, C₁₋₄ haloalkyl, C₂₋₄ alkenyl, C₂₋₄ alkynyl, OR^{e} and CN;
each R² is independently selected from hydrogen, halogen, C₁₋₄ alkyl, C₁₋₄ haloalkyl, C₂₋₄ alkenyl, C₂₋₄ alkynyl, C₃₋₆ cycloalkyl, 3- to 6-membered heterocyclyl, OR^{e}, SR^{e}, NR^{e}R^{e}, CN and C(O)R^{h};
each R³ is independently selected from hydrogen and C₁₋₄ alkyl; or when two R³ are attached to the same carbon atom, said two R³ together with the carbon atom to which they are attached optionally form a carbonyl group (C=O);
J and G are each independently selected from NR^{f}, O, S, S(O), S(O)₂ and CR^{g}R^{g};
Z is selected from O, NR^{e} and CH₂;
W is N or CR^{b};
each R^{a} is hydrogen, halogen, or C₁₋₄ alkyl;
each R^{b} is independently selected from hydrogen, halogen, C₁₋₄ alkyl, C₁₋₄ haloalkyl, OR^{e}, SR^{e}, NR^{e}R^{e} and CN;
each R^{c} is independently selected from hydrogen, C₁₋₄ alkyl and C₃₋₆ cycloalkyl;
R^{d} is selected from C₁₋₄ alkyl, C₂₋₄ alkenyl, C₂₋₄ alkynyl, C₃₋₆ cycloalkyl, or 3- to 6-membered heterocyclyl;
each Rⁱ is independently selected from hydrogen, halogen, C₁₋₄ alkyl, C₁₋₄ haloalkyl, OR^{e}, SR^{e} and NR^{e}R^{e};
R^{f} is hydrogen, C₁₋₄ alkyl, C₁₋₄ haloalkyl, C₂₋₄ alkenyl, C₂₋₄ alkynyl, C₃₋₈ cycloalkyl, 3- to 12-membered heterocyclyl, aryl, heteroaryl, C(O)R^{h}, C(O)OR^{e}, C(O)NR^{e}R^{e}, S(O)₂R^{h} and S(O)₂NR^{e}R^{e}; wherein said alkyl, cycloalkyl, heterocyclyl, aryl, heteroaryl is optionally substituted by one or more substituents selected from the group consisting of halogen, C₁₋₄ alkyl, C₁₋₄ haloalkyl, C₃₋₆ cycloalkyl, 3- to 6-membered heterocyclyl, aryl, heteroaryl, OR^{e}, SR^{e}, NR^{e}R^{e}, CN, C(O)R^{h}, C(O)OR^{e}, C(O)NR^{e}R^{e}, NR^{e}C(O)R^{h}, S(O)₂R^{h}, S(O)₂NR^{e}R^{e} and NR^{e}S(O)₂R^{h};
each R^{g} is independently selected from the group consisting of hydrogen, halogen, C₁₋₄ alkyl, C₁₋₄ haloalkyl, OR^{e}, SR^{e}, NR^{e}R^{e}, CN, C(O)R^{h}, C(O)OR^{e}, C(O)NR^{e}R^{e}, NR^{e}C(O)R^{h}, or NR^{e}S(O)₂R^{h}; or two R^{g} attached to the same carbon atom together form a carbonyl group (C=O); or two R^{g} attached to the same carbon atom together form a 3- to 8-membered cyclic structure, said cyclic structure optionally containing 0, 1, or 2 heteroatoms selected from N, O and S;
each R^{e} is independently selected from hydrogen, C₁₋₄ alkyl, C₁₋₄ haloalkyl, C₃₋₆ cycloalkyl, or 3- to 6-membered heterocyclyl; or two R^{e} together with the nitrogen atom to which they are attached form a 3- to 8-membered heterocyclyl containing 1 or 2 N atoms and 0 or 1 heteroatom selected from O and S;
each R^{h} is independently selected from the group consisting of hydrogen, C₁₋₄ alkyl, C₁₋₄ haloalkyl, C₂₋₄ alkenyl, C₂₋₄ alkynyl, C₃₋₆ cycloalkyl, 3- to 6-membered heterocyclyl, aryl and heteroaryl;
k is 0, 1, 2, or 3;
n is 0, 1, 2, 3, or 4;
p and q are each independently 0, 1, 2, 3, 4, or 5;
f is 2, 3, 4, or 5;
g is 0, 1, 2, 3, or 4;
h is 0, 1, 2, or 3;
i is 0, 1, 2, or 3;
j is 0, 1, 2, 3, or 4;
t is 0, 1, 2, 3, or 4;
provided that when X is selected from CH, Y is selected from N, T is selected from CR¹, and
   A is selected from formula (IId), the structural fragment 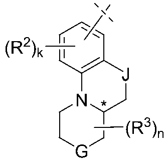 in formula (I) is selected from formula (IIf): 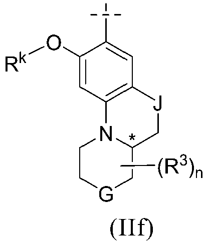
" - - -" means the point in formula (IIf) which attach to the remaining part of formula (I);
R^{k} is selected from the group consisting of hydrogen, C₁₋₄ alkyl, C₁₋₄ haloalkyl, C₃₋₆ cycloalkyl and 3- to 6-membered heterocyclyl; the remaining groups in formula (IIf) are as defined above;
wherein, each of the aforementioned alkyl, alkenyl, alkynyl, cycloalkyl, heterocyclyl, cyclic structure, aryl, and heteroaryl groups is optionally and independently substituted with 1 to 3 substituents, each independently selected from the group consisting of: halogen, C₁₋₄ alkyl, C₁₋₄ haloalkyl, C₂₋₄ alkenyl, C₂₋₄ alkynyl, C₃₋₈ cycloalkyl, 3- to 12-membered heterocyclyl, aryl, heteroaryl, CN, NO₂, OR^{e}, SR^{e}, NR^{e}R^{e}, C(O)R^{h}, C(O)OR^{e}, C(O)NR^{e}R^{e}, NR^{e}C(O)R^{h}, S(O)₂R^{h}, and NR^{e}S(O)₂R^{h}, provided that the resulting chemical structure is stable and meaningful; wherein R^{e} and R^{h} are as defined above.
unless otherwise specified, the aforementioned aryl is an aromatic group containing 6-12 carbon atoms; heteroaryl is a 5- to 15-membered heteroaromatic group; cyclic structure is a saturated or unsaturated cyclic group, optionally containing heteroatoms.

In another preferred embodiment, formula (I) is formula (IIIa) or formula (IIIb): 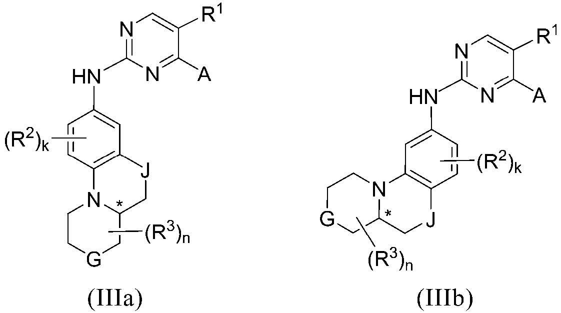 wherein the definitions of the groups in Formula (IIIa) and Formula (IIIb) are as defined in above.

In another preferred embodiment, Formula (I) is Formula (IVa), Formula (IVb), Formula (IVc) or Formula (IVd): 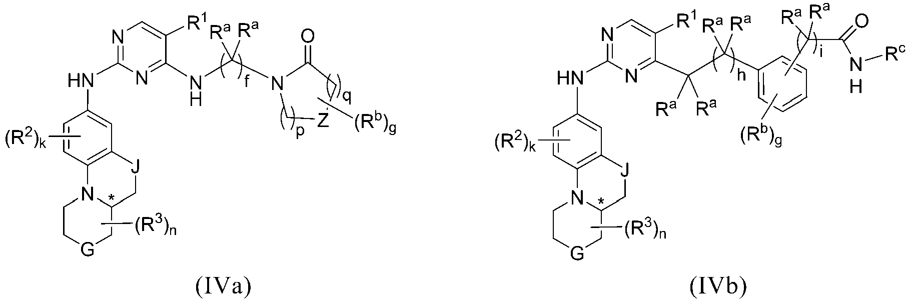 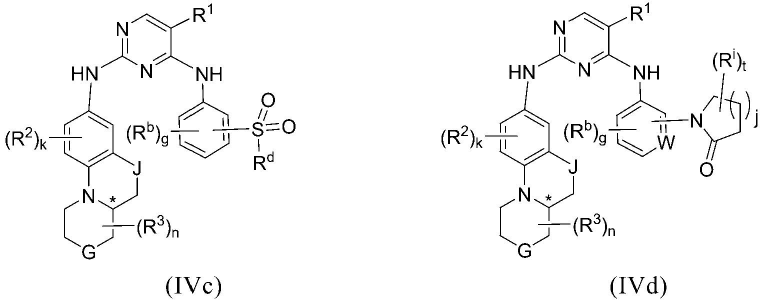 wherein the groups in Formula (IVa), Formula (IVb), Formula (IVc) and Formula (IVd) are as defined above.

In another preferred embodiment, Formula (I) is Formula (V): 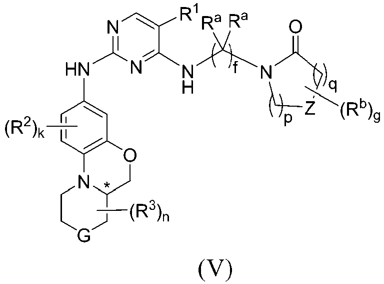 wherein the groups in Formula (V) are as defined as above.

In another preferred embodiment, Formula (I) is Formula (VI): 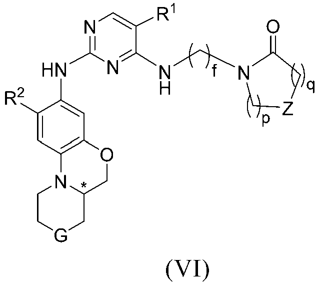
R¹ is selected from hydrogen, halogen, C₁₋₄ alkyl and C₁₋₄ haloalkyl;
R² is selected from hydrogen, halogen, C₁₋₄ alkyl, C₁₋₄ haloalkyl, C₃₋₆ cycloalkyl and O R^{e};
G is selected from NR^{f}, O and CR^{g}R^{g};
Z is selected from O, NR^{e} and CH₂;
R^{f} is hydrogen, C₁₋₄ alkyl, C₃₋₈ cycloalkyl, 3- to 12-membered heterocyclyl (preferably 3- to 8-membered heterocyclyl), aryl, heteroaryl, C(O)R^{h}, C(O)OR^{e}, C(O)NR^{e}R^{e}, S(O)₂R^{h}, or S(O)₂NR^{e}R^{e}; wherein said alkyl, cycloalkyl, heterocyclyl, aryl, heteroaryl is optionally substituted by one or more substituents selected from the group consisting of halogen, C₁₋₄ alkyl, C₁₋₄ haloalkyl, C₃₋₆ cycloalkyl, 3- to 6-membered heterocyclyl, aryl, heteroaryl, OR^{e}, SR^{e}, NR^{e}R^{e}, CN, C(O)R^{h}, C(O)OR^{e}, C(O)NR^{e}R^{e}, NR^{e}C(O)R^{h}, S(O)₂R^{h}, S(O)₂NR^{e}R^{e} and NR^{e}S(O)₂R^{h};
each R^{g} is independently selected from the group consisting of hydrogen, halogen, C₁₋₄ alkyl, C₁₋₄ haloalkyl, OR^{e}, SR^{e}, NR^{e}R^{e}, CN, NR^{e}C(O)R^{h}, or NR^{e}S(O)₂R^{h};
each R^{e} is independently selected from hydrogen, C₁₋₄ alkyl, C₁₋₄ haloalkyl, C₃₋₆ cycloalkyl, or 3- to 6-membered heterocyclyl;
each R^{h} is independently selected from hydrogen, C₁₋₄ alkyl, C₁₋₄ haloalkyl, C₂₋₄ alkenyl, C₂₋₄ alkynyl, C₃₋₆ cycloalkyl, 3- to 6-membered heterocyclyl, aryl, heteroaryl;
p and q are each independently 0, 1, 2, 3 or 4;
f is 2, 3 or 4.

In another preferred embodiment, formula (I) is formula (VII): 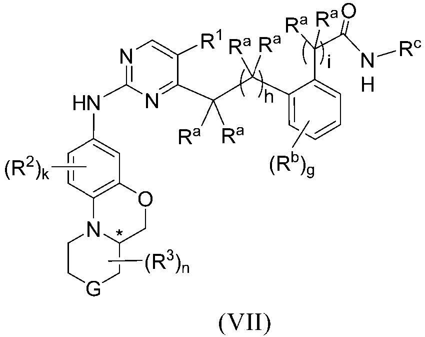 the groups in Formula (VII) are as defined above.

In another preferred embodiment, Formula (I) is Formula (VIII): 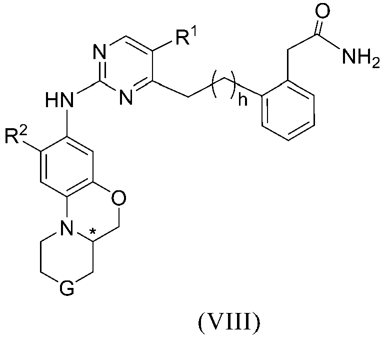
h is 0, 1, 2 or 3;
R¹, R² and G are defined as above.

In another preferred embodiment, Formula (I) is Formula (IX): 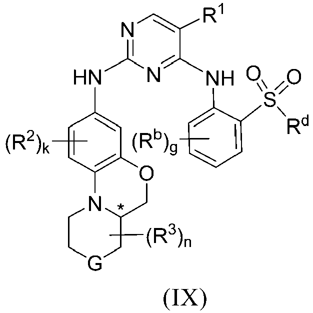 the groups in Formula (IX) are as defined above.

In another preferred embodiment, Formula (I) is Formula (X): 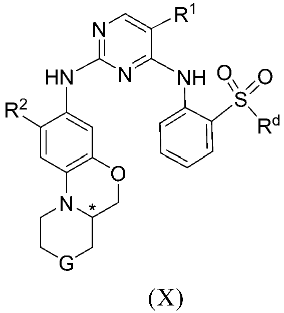
R^{d} is independently selected from C₂₋₄ alkenyl, C₂₋₄ alkynyl, C₃₋₆ cycloalkyl, and 3- to 6-membered heterocyclyl;
R¹, R² and G are as defined above.

In another preferred embodiment, Formula (I) is Formula (XI): 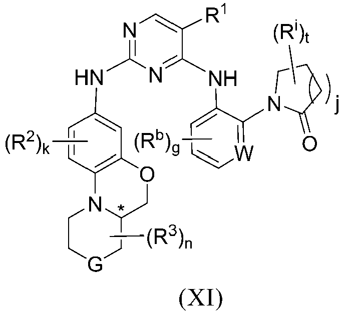 the groups in Formula (XI) are as defined above.

In another preferred embodiment, Formula (I) is Formula (XII): 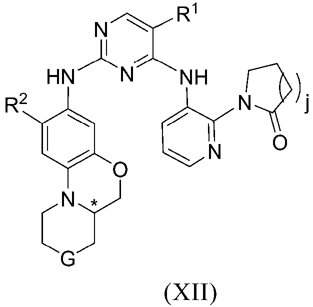
j is 0, 1, 2, 3 or 4;
R¹, R² and G are as defined above.

In another preferred embodiment, Formula (I) is Formula (XIII): 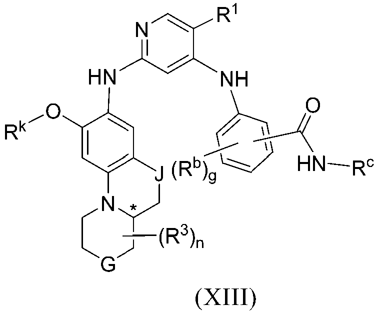 the groups in Formula (XIII) are as defined above.

In another preferred embodiment, Formula (I) is Formula (XIV): 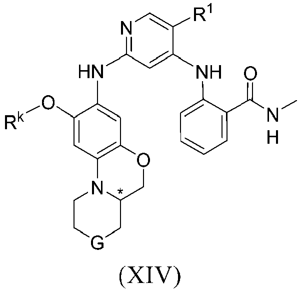
R^{k} is selected from hydrogen, C₁₋₄ alkyl, C₁₋₄ haloalkyl, C₃₋₆ cycloalkyl, or 3- to 6-membered heterocyclyl;
the R¹ and G are as defined above.

In another preferred embodiment, the compound is selected from one of the following:

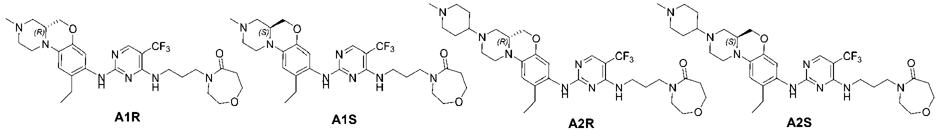

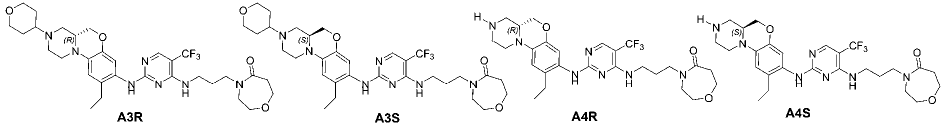

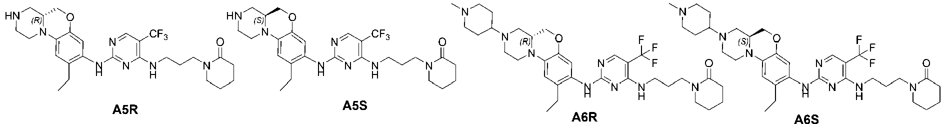

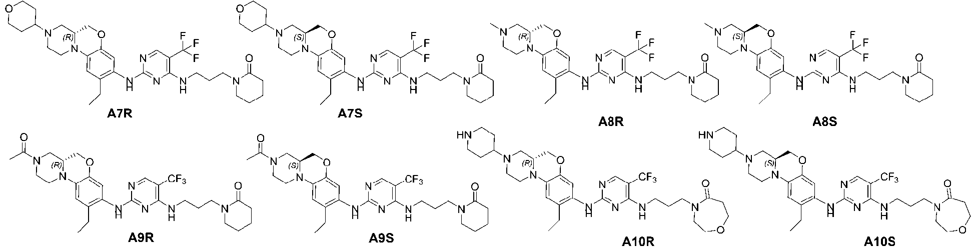

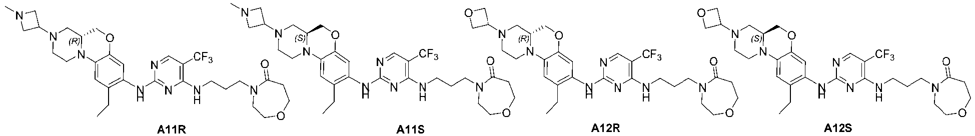

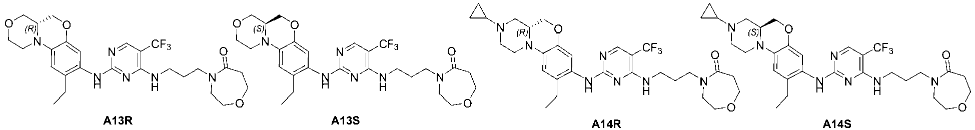

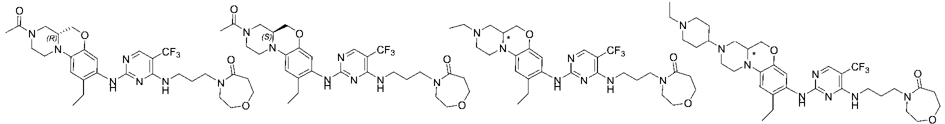

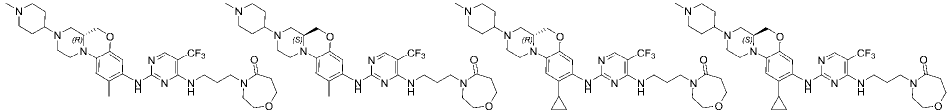

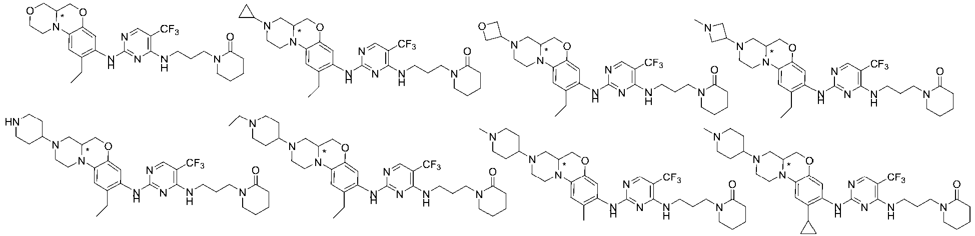

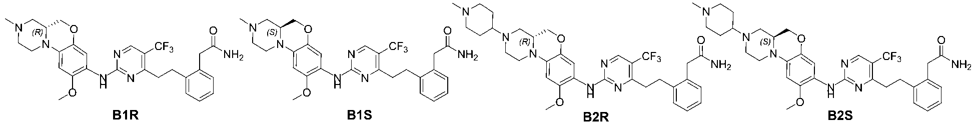

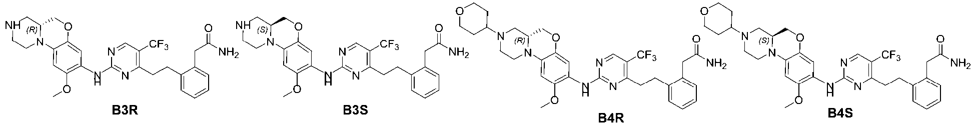

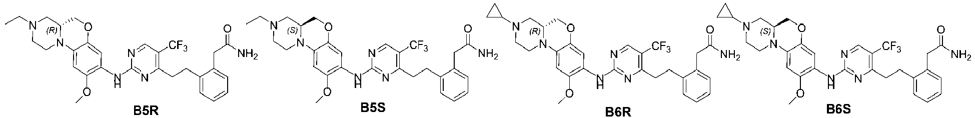

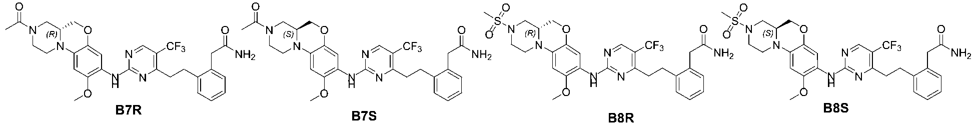

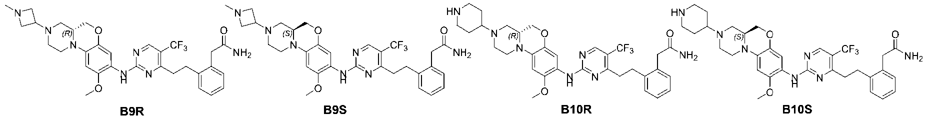

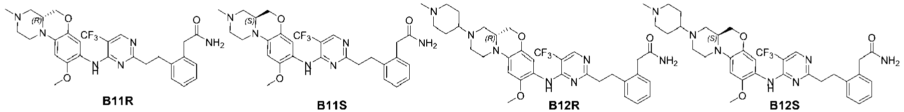

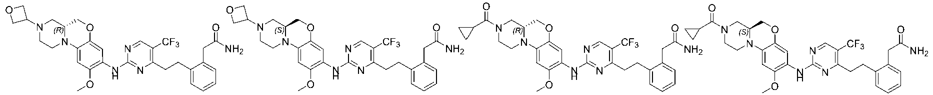

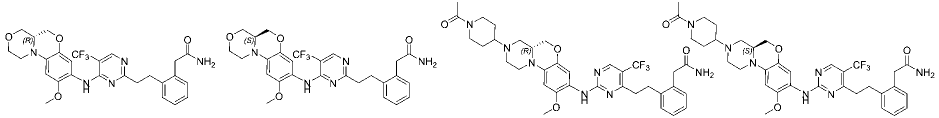

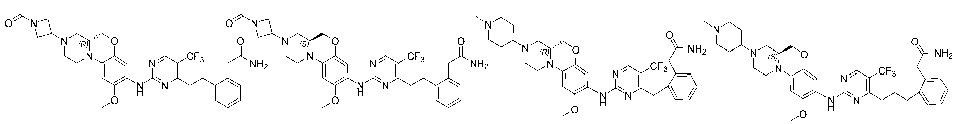

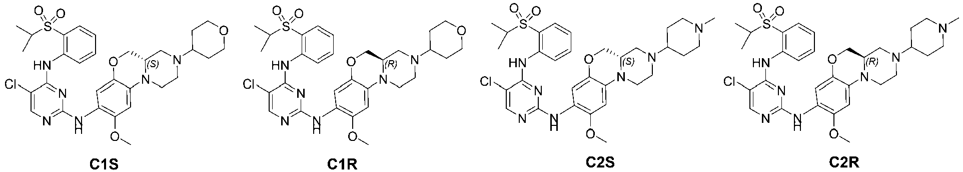

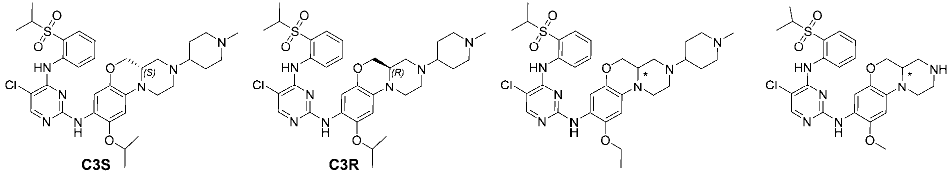

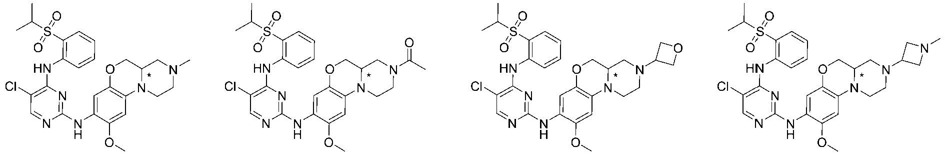

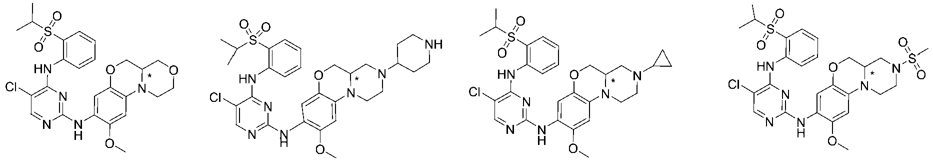

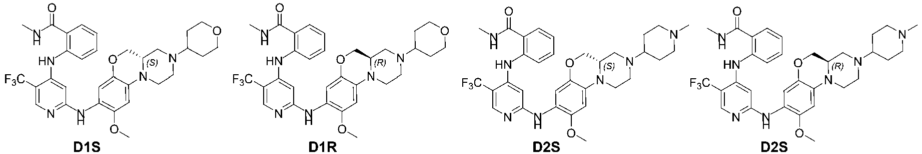

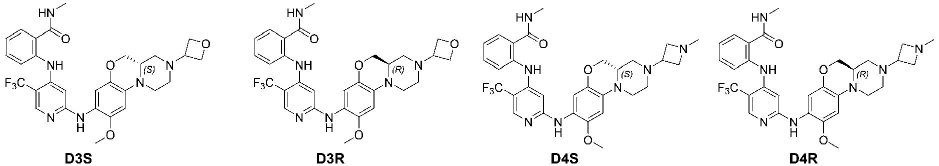

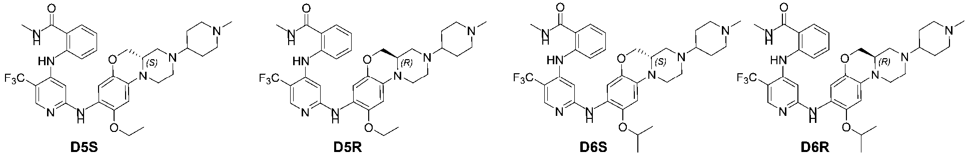

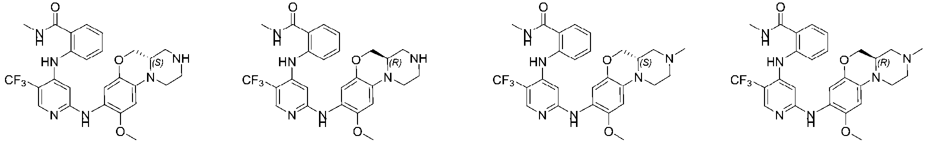

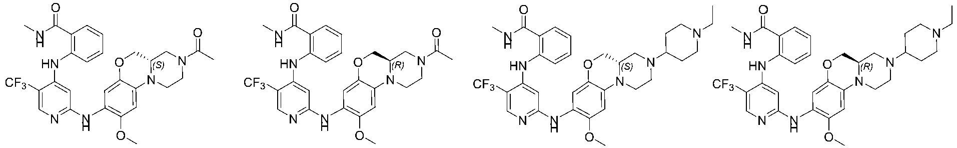

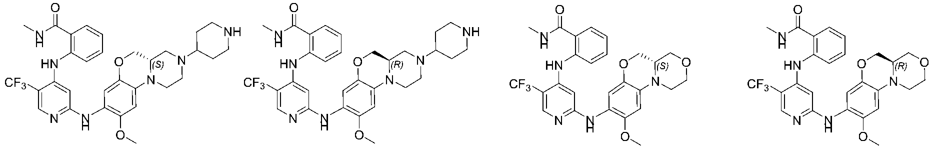

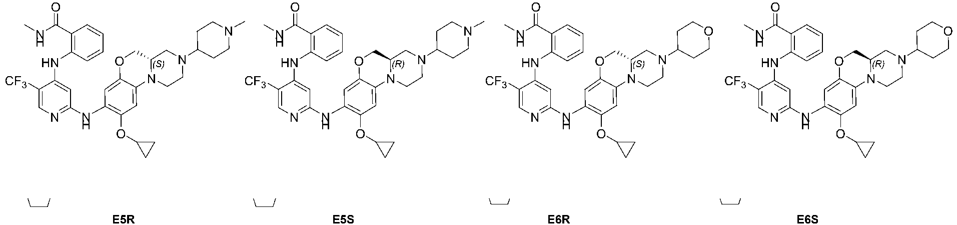

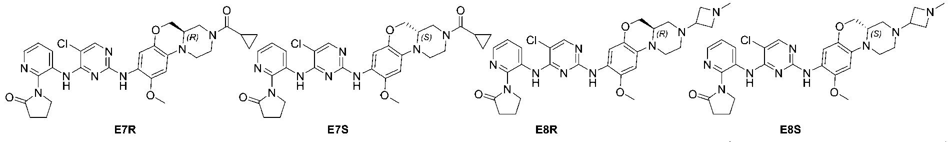

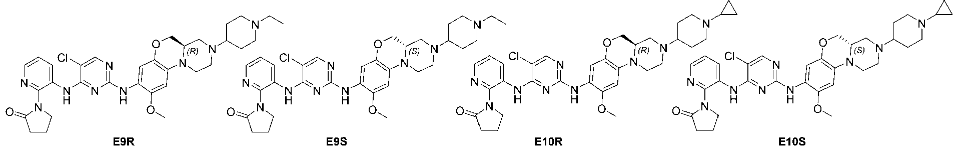

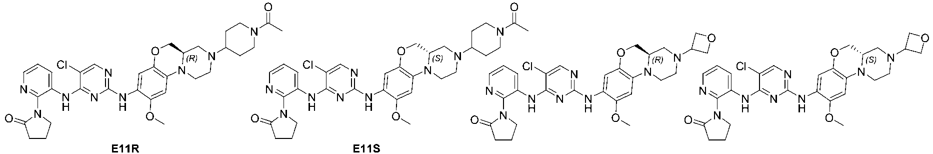

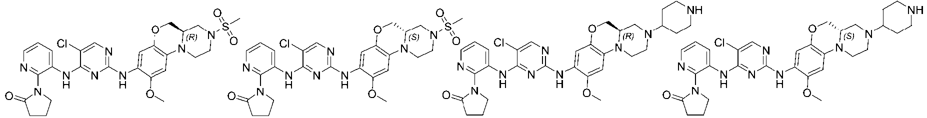

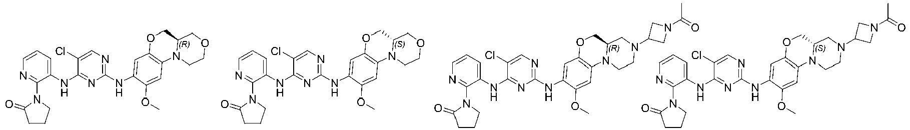

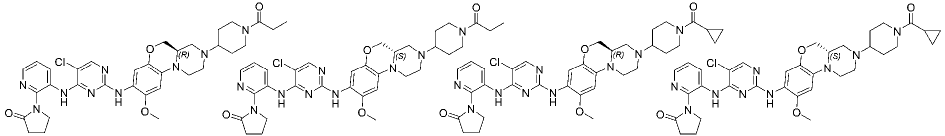

"*" denotes a chiral center in chemical structures.

In the second aspect of the present invention, a pharmaceutical composition comprising a compound according to the first aspect of the invention, or an optical isomer, pharmaceutically acceptable salt, prodrug, deuterated derivative, hydrate, or solvate thereof, and a pharmaceutically acceptable carrier is provided.

In the third aspect of the present invention, a use of a compound according to the first aspect of the invention, or an optical isomer, pharmaceutically acceptable salt, prodrug, deuterated derivative, hydrate, or solvate thereof for use in the preparation of a pharmaceutical composition for treating a disease, disorder, or condition associated with kinase activity or expression level, including ULK, FAK, ALK, CDK7, HPK1, AXL, FLT3, TNK1, and the like.

In another preferred embodiment, said disease, disorder, or condition is selected from the group consisting of various other solid tumors and hematological malignancies, including breast cancer, non-small cell lung cancer (NSCLC), small cell lung cancer (SCLC), lung adenocarcinoma, lung squamous cell carcinoma, colon cancer, colorectal cancer, thyroid cancer, embryonal rhabdomyosarcoma, granular cell tumor of the skin, melanoma, liver cancer, rectal cancer, bladder cancer, laryngeal cancer, pancreatic cancer, prostate cancer, glioma, ovarian cancer, head and neck squamous cell carcinoma (HNSCC), cervical cancer, esophageal cancer, renal cancer, skin cancer, lymphoma, gastric cancer, mesothelioma, osteosarcoma, acute myeloid leukemia (AML), myelofibrosis, B-cell lymphoma, monocytic leukemia, splenomegalic polycythemia, hypereosinophilic syndrome, multiple myeloma; pulmonary fibrosis; DNA and RNA viral infections such as AIDS, herpesvirus infections, influenza virus infections, and the like.

### Detailed Description

After long-term and intensive research, the inventors of the present invention have unexpectedly discovered a class of novel structural CTPS1 inhibitors, as well as their preparation methods and applications. The compounds of the present invention can be applied to treat various diseases associated with the activity of said CTPS1. Based on this discovery, the inventors have completed the present invention.

### Definitions

As used herein, the word "or" has the meaning of both "or" and "and" and is equivalent to "and/or" - unless otherwise specifically limited to just "or".

As used herein, unless otherwise stated, a chiral carbon atom (or chiral center) of the compound(s) in the invention is optionally R-type, S-type, or a combination thereof.

As used herein, unless otherwise stated, the term "alkyl" by itself or as part of another substituent (which may include the short form of "alk,"e.g., alkoxy), refers to a straight (i.e. unbranched), branched chain, or cyclic hydrocarbon radical, or combination thereof, which may be fully saturated, mono- or polyunsaturated and can include di-and multivalent radicals. When an alkyl is preceded by a carbon-number modifier, e.g., C₁₋₁₀, its means the alkyl group contains 1 to 10 carbon atoms. For instance, examples of C₁₋₈ alkyl may include a linear or branched alkyl having 1-8 carbon atoms, such as methyl, ethyl, propyl, isopropyl, butyl, isobutyl, sec-butyl, and t-butyl.

As used herein, the term "alkenyl," by itself or as part of another substituent, refers to a straight chain, or branched hydrocarbon chains having at least one carbon-carbon double bond. An alkenyl group with one double bond can be denoted as -CₙH₂ₙ₋₁ or -CₙH₂ₙ₋₃ with two double bonds. When an alkenyl is preceded by a carbon-number modifier, e.g., C₂₋₈, it means the alkenyl group contains 2 to 8 carbon atoms. For instance, examples of C₂₋₈ alkenyl may include vinyl, allyl, 1,2-butenyl, 2,3-butenyl, and butadienyl etc.

As used herein, the term "alkynyl," by itself or as part of another substituent, refers to an aliphatic hydrocarbon group with at least one carbon-carbon triple bond. An alkynyl group may be linear or branched or combinations thereof. In some embodiments, it can contain 2 to 12 (e.g., 2 to 8, 2 to 6, or 2 to 4) carbon atoms. When an alkynyl is preceded by a carbon-number modifier, e.g., C₂₋₈, it means the alkynyl group contains 2 to 8 carbon atoms. Examples of an alkynyl group (e.g., C₂₋₈ alkynyl) may include acetenyl, propynyl, isopropynyl, 1-butynyl, isobutynl, and sec-butynyl etc.

As used herein, the term "cycloalkyl" by itself or as part of another substituent, refers to a saturated or partially saturated carbocyclic mono-, bi-, or tri-cyclic (fused or bridged or spiral) ring system. It can contain 3 to 12 (e.g., 3 to 10, or 5 to 10) carbon atoms. When a cycloalkyl group is preceded by a carbon-number modifier, e.g., C₃₋₁₀, it means the cycloalkyl group contains 3 to 10 carbon atoms. In some embodiments, the term "C₃₋₁₀ cycloalkyl" may refer to a saturated or partially saturated mono- or bicyclic alkyl ring system containing 3 to 10 carbon atoms, such as cyclopropyl, cyclobutyl, cyclopentyl, cyclohexyl, and cycloheptyl. Below are some examples of cycloalkyl group.

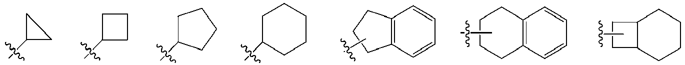

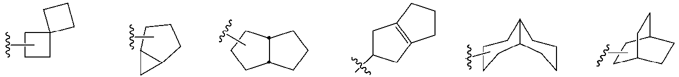

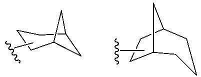

Unless otherwise stated, the following terms used in the specification and claims have the following meanings. "Aryl" means an all-carbon monocyclic or fused polycyclic (ie, a ring that shares a pair of adjacent carbon atoms) groups having a conjugated π-electron system, such as phenyl and naphthyl. The aryl ring may be fused to other cyclic groups (including saturated and unsaturated rings), but may not contain heteroatoms such as nitrogen, oxygen, or sulfur, while the point of attachment to the parent must be on the carbon atoms of the ring in a conjugated π-electron system. The aryl group can be substituted or unsubstituted. The following are some examples of aryl groups, and the present invention is not limited to the aryl groups described below.

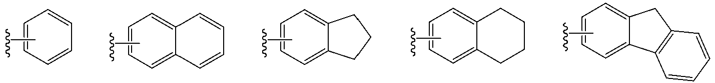

"Heteroaryl" refers to an aromatic monocyclic or polycyclic group containing one to more heteroatoms (optionally from nitrogen, oxygen, and sulfur), or a polycyclic group formed by condensing a heterocyclic group (containing one to more heteroatoms, optionally selected from nitrogen, oxygen, and sulfur) with an aryl group, and the attachment site is located on the aryl group. The heteroaryl group can be optionally substituted or unsubstituted. The following are some examples of heteroaryl groups, and the present invention is not limited to the following heteroaryl groups.

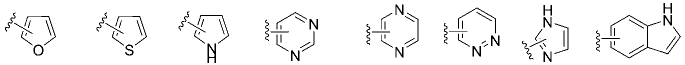

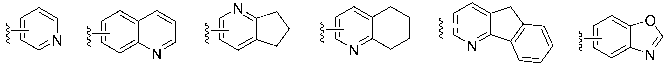

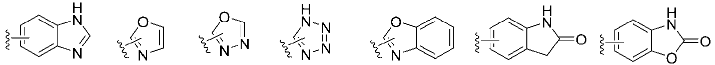

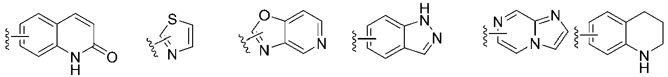

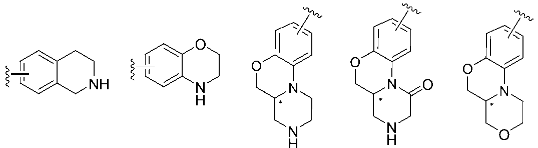

"Heterocyclyl" means a saturated or partially unsaturated monocyclic or polycyclic cyclic hydrocarbon substituent wherein one or more of the ring atoms are selected from nitrogen, oxygen or sulfur and the remaining ring atoms are carbon. Non-limiting examples of monocyclic heterocyclic groups include pyrrolidinyl, piperidinyl, piperazinyl, morpholinyl, thiomorpholinyl, homopiperazinyl. Polycyclic heterocyclic group refers to a heterocyclic group including a spiro ring, a fused ring, and a bridged ring. "Spirocyclic heterocyclyl" refers to a polycyclic heterocyclic group in which each ring of the system shares an atom (referred to as a spiro atom) with other rings in the system, wherein one or more of the ring atoms is selected from the group consisting of nitrogen and oxygen. Or sulfur, the remaining ring atoms are carbon. "Fused ring heterocyclyl" refers to a polycyclic heterocyclic group in which each ring of the system shares an adjacent pair of atoms with other rings in the system, and one or more rings may contain one or more double bonds, but none One ring has a fully conjugated pi-electron system, and wherein one or more ring atoms are selected from nitrogen, oxygen or sulfur, and the remaining ring atoms are carbon. "Bridged heterocyclyl" refers to a polycyclic heterocyclic group in which any two rings share two atoms which are not directly bonded, these may contain one or more double bonds, but none of the rings have a fully conjugated pi-electron system, and wherein one or more of the ring atoms are selected from nitrogen, oxygen or sulfur, and the remaining ring atoms are carbon. If a heterocyclic group has both a saturated ring and an aromatic ring (for example, the saturated ring and the aromatic ring are fused together), the point attached to the parent must be on the saturated ring. Note: When the point attached to the parent is on the aromatic ring, it is called a heteroaryl group and is not called a heterocyclic group. Some examples of the heterocyclic group are as follows, and the present invention is not limited to the following heterocyclic group.

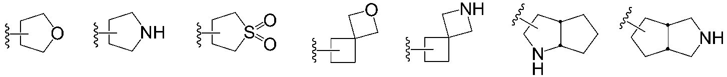

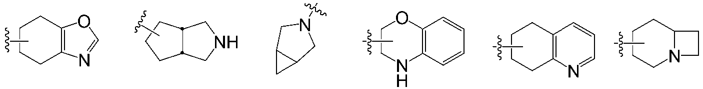

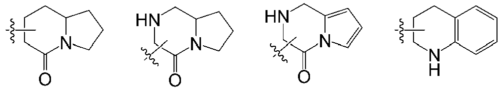

As used herein, the term "halogen", when used alone or as part of another substituent, refers to F, Cl, Br, and I.

As used herein, the term "substituted" (when with or without "optionally") means that one or more hydrogen atoms on a particular group are replaced by a particular substituent. Particular substituents are the substituents described above in the corresponding paragraphs, or the substituents which appear in the examples. Unless otherwise stated, an optionally substituted group may have a substituent selected from a particular group at any substitutable position of the group, and the substituents may be the same or different at each position. A cyclic substituent, such as a heterocyclic group, may be attached to another ring, such as a cycloalkyl group, to form a spirobicyclic ring system, i.e., the two rings have a common carbon atom. Those skilled in the art will appreciate that the combinations of substituents contemplated by the present invention are those that are stable or chemically achievable. The substituents are, for example but not limited to, C₁₋₈ alkyl, C₂₋₈ alkenyl, C₂₋₈ alkynyl, C₃₋₈ cycloalkyl, 3- to 12-membered heterocyclyl, aryl, heteroaryl, halogen, hydroxy, carboxy (-COOH), C₁₋₈ aldehyde, C₂₋₁₀ acyl, C₂₋₁₀ ester, amino group.

For convenience and in accordance with conventional understanding, the term "optionally substituted" or "optionally substituted" applies only to sites which are capable of being substituted by a substituent, and does not include those which are not chemically achievable.

As used herein, unless otherwise specified, the term "pharmaceutically acceptable salt" refers to a salt that is suitable for contact with the tissue of a subject (eg, a human) without causing unpleasant side effects. In some embodiments, a pharmaceutically acceptable salt of a compound of the invention includes a salt (eg, a potassium salt, a sodium salt, a magnesium salt, a calcium salt) of a compound of the invention having an acidic group or is a salt of a compound of a compound of the invention having a basic group the invention (e.g., a sulfate, a hydrochloride, a phosphate, a nitrate, a carbonate).

### Application:

The present invention provides a class of compounds of formula (I), or their deuterated derivatives, their pharmaceutically acceptable salts, optical isomers (enantiomers or diastereomers, if any case), hydrates, solvates, or pharmaceutical combinations comprising the compound represented by formula (I), its optical isomers, pharmaceutically acceptable salts, prodrugs, deuterated forms, hydrates, and solvates for inhibiting kinase activity.

The compound of the present invention can be used as kinase inhibitor.

The present invention provides a monospecific inhibitor of kinase, which achieves the purpose of preventing, alleviating, or curing diseases by modulating the activity of kinase. The diseaseSR^{e}ferred to include, but are not limited to: breast cancer, non-small cell lung cancer, small cell lung cancer, lung adenocarcinoma, lung squamous cell carcinoma, colon cancer, colorectal cancer, thyroid cancer, embryonal rhabdomyosarcoma, skin granular cell tumor, melanoma, liver cancer, rectal cancer, bladder cancer, laryngeal cancer, pancreatic cancer, prostate cancer, glioma, ovarian cancer, head and neck squamous cell carcinoma, cervical cancer, esophageal cancer, renal cancer, skin cancer, lymphoma, gastric cancer, mesothelioma, osteosarcoma, acute myeloid leukemia, myelofibrosis, B-cell lymphoma, monocyte leukemia, splenomegaly with polycythemia, eosinophilia syndrome, multiple myeloma, and various other solid tumors and hematologic malignancies; pulmonary fibrosis; HIV, herpes viruses, and influenza viruses, among other DNA and RNA viral infections.

The compounds of the present invention and their deuterated forms, as well as pharmaceutically acceptable salts or isomers (if present) or hydrates and/or compositions thereof can be combined with pharmaceutically acceptable excipients or carriers formulated together, the resulting composition can be administered to humans or animals for the treatment of disorders, symptoms and diseases. The composition can be: tablets, pills, suspensions, solutions, emulsions, capsules, aerosols, sterile injections, sterile powders and the like. In a preferred embodiment, the pharmaceutical composition is a dosage form suitable for oral administration, including but not limited to tablets, solutions, suspensions, capsules, granules, and powders. The amount of the compound or pharmaceutical composition administered to the patient is not fixed, and is usually administered in a pharmaceutically effective amount. At the same time, the amount of the compound actually administered can be determined by the physician according to the actual situation, including the disease to be treated, the route of administration selected, the actual compound administered, the individual condition of the patient, and so on. The dosage of the compound of the present invention depends on the specific use of the treatment, the mode of administration, the state of the patient, and the judgment of the physician. The ratio or concentration of the compound of the present invention in the pharmaceutical composition depends on a variety of factors, including dosage, physical and chemical properties, route of administration, and the like.

It is to be understood that within the scope of the present invention, the various technical features of the present invention and the various technical features specifically described hereinafter (as in the embodiments) may be combined with each other to form a new or preferred technical solution.

### Pharmaceutical composition and method of administration

Since the compound of the present invention has excellent inhibitory activity against CTPS1, the compound of the present invention and its various crystal forms, optical isomers, pharmaceutically acceptable inorganic or organic salts, prodrugs, deuterated forms, hydrates or solvates, and pharmaceutical compositions containing the compounds of the present invention as the main active ingredients can be used to treat, prevent and alleviate diseases associate with the activity or expression of CTPS1.

The pharmaceutical compositions of the present invention comprise a safe or effective amount of a compound of the present invention, or a pharmaceutically acceptable salt thereof, and a pharmaceutically acceptable excipient or carrier. By "safe and effective amount" it is meant that the amount of the compound is sufficient to significantly improve the condition without causing serious side effects. In general, the pharmaceutical compositions contain from 1 to 2000 mg of the compound of the invention per agent, more preferably from 5 to 200 mg of the compound of the invention per agent. Preferably, the "one dose" is a capsule or tablet.

"Pharmaceutically acceptable carrier" means: one or more compatible solid or liquid fillers or gel materials which are suitable for human use and which must be of sufficient purity and of sufficiently low toxicity. By "compatibility" it is meant herein that the components of the composition are capable of intermingling with the compounds of the invention and with each other without significantly reducing the efficacy of the compound. Examples of pharmaceutically acceptable carriers include, but are not limited to, filler (or diluent), disintegrant, lubricant, binder, matrix, emulsifiers, run wet agents, colorants, flavoring agents, stabilizers, antioxidants, preservatives, pyrogen-free water, and the like.

The mode of administration of the compound or pharmaceutical composition of the present invention is not particularly limited, and representative modes of administration include, but are not limited to, oral, intratumoral, rectal, parenteral (intravenous, intramuscular or subcutaneous), and topical administration.

Solid dosage forms for oral administration include capsules, tablets, pills, powders, and granules. In these solid dosage forms, the active compound is mixed with at least one conventional inert excipient (or carrier). In capsules, tablets and pills, the dosage form may also contain a buffer.

Solid dosage forms such as tablets, dragees, capsules, pills, and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric and waxy materials. If necessary, the active compound may also be in microencapsulated form with one or more of the above-mentioned excipients.

In addition to these inert diluents, the compositions may contain adjuvants such as wetting agents, emulsifying and suspending agents, sweetening agents, flavoring agents and perfumes.

In addition to the active compound, the suspension may contain suspending agents.

Compositions for parenteral injection may comprise a physiologically acceptable sterile aqueous or nonaqueous solution, dispersion, suspension or emulsion, and a sterile powder for reconstitution into a sterile injectable solution or dispersion. Suitable aqueous and nonaqueous vehicles, diluents, solvents or vehicles include water, ethanol, polyols, and suitable mixtures thereof.

Dosage forms for the compounds of the invention for topical administration include ointments, powders, patches, propellants and inhalants. The active ingredient is admixed under sterile conditions with a physiologically acceptable carrier and any preservatives, buffers, or, if necessary, propellants.

The compounds of the invention may be administered alone or in combination with other pharmaceutically acceptable compounds.

When a pharmaceutical composition is used, a safe and effective amount of a compound of the invention is administered to a mammal (e.g., a human) in need of treatment wherein the dosage is a pharmaceutically effective effective dosage, for a 60 kg body weight, The dose to be administered is usually from 1 to 2000 mg, preferably from 5 to 500 mg. Of course, specific doses should also consider factors such as the route of administration, the health of the patient, etc., which are within the skill of the skilled physician.

### The main advantages of the present invention include:

1. Provided a compound as shown in Formula I.
2. Provided a structurally novel kinase inhibitor, and the preparation and application thereof, said inhibitor being able to inhibit the activity of kinase at a very low concentration.
3. Provided a kinase inhibitor of good oral absorption.
4. Provided a class of pharmaceutical compositions for the treatment of diseases associated with kinase activity.
5. Said kinase includes: ULK, FAK, ALK, CDK7, HPK1, AXL, FLT3, TNK1, etc.

The invention is further illustrated below in conjunction with specific embodiments. It is to be understood that the examples are not intended to limit the scope of the invention. The experimental methods in the following examples which do not specify the specific conditions are usually in accordance with conventional conditions or according to the conditionSR^{e}commended by the manufacturer. Percentages and parts are by weight unless otherwise stated.

Some of the representative compounds of the present invention can be prepared by the following synthetic methods, and in each of the following reaction formulas, the reagents and conditions for each step can be selected from those conventional in the field for carrying out such preparation methods, and the above selections can be made by a person skilled in the art according to the knowledge in the field after the structures of the compounds of the present invention are disclosed.

### Examples

### Abbreviations:

Boc = tert-Butoxycarbonyl
DCM = Dichloromethane
DIPEA or DIEA = N,N-Diisopropylethylamine
DMF = N,N-Dimethylformamide
DMSO = Dimethyl sulfoxide
EtOAc or EA = Ethyl acetate
Et = Ethyl
HATU = N,N,N',N'-Tetramethyl-O-(7-azabenzotriazol-1-yl)uronium hexafluorophosphate
Me = Methyl
MeOH = Methanol
NMP = N-Methylpyrrolidone
Ph = Phenyl
Pd₂(dba)₃ = Tris(dibenzylideneacetone)dipalladium
TEA = Triethylamine
TFA = Trifluoroacetic acid
THF = Tetrahydrofuran
TsCl = p-Toluenesulfonyl chloride
TBDPSCl = tert-Butyldiphenylsilyl chloride
XantPhos = 4,5-Bis(diphenylphosphino)-9,9-dimethylxanthene
XPhos = 2-Dicyclohexylphosphino-2',4',6'-triisopropylbiphenyl

### Example 1: Preparation of Compounds A1R and A4R

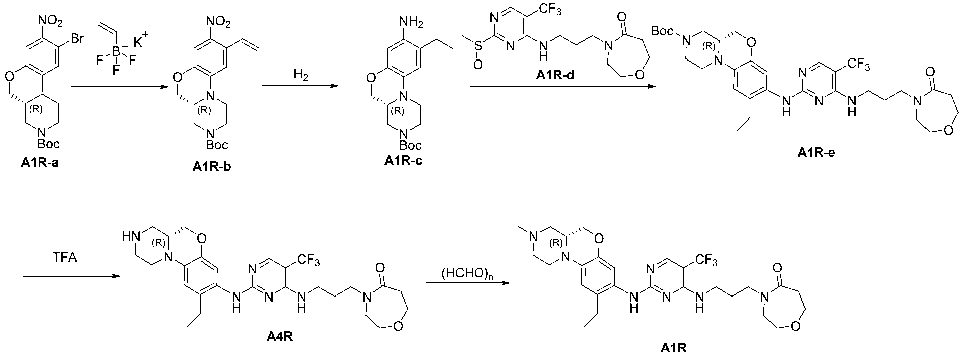

Compound **A1R-a** (850 mg, 2.06 mmol) (Compound **A1R-a** was synthesized with reference to the method for **D50R**), potassium vinyltrifluoroborate (414 mg, 3.09 mmol), [1,1'-bis(diphenylphosphino)ferrocene]dichloropalladium (75 mg, 0.103 mmol) and potassium carbonate (854 mg, 6.18 mmol) were dissolved in dimethyl sulfoxide (10 mL). The mixture was heated to 100 °C under a nitrogen atmosphere and stirred for 2 hours. After the reaction was completed, the mixture was diluted with water. The mixture was extracted with ethyl acetate (3×30 mL). The combined organic phases were washed with saturated brine, dried over anhydrous sodium sulfate, filtered and concentrated under reduced pressure. The obtained crude product was separated and purified by silica gel column chromatography (petroleum ether:ethyl acetate = 5:1) to give compound **A1R-b** as a yellow solid (600 mg, yield 81%). MS m/z 362.1 [M+H]⁺.

Compound **A1R-b** (600 mg, 1.66 mmol) and palladium-carbon catalyst (10%, 200 mg) were dissolved in methanol (10 mL). The reaction mixture was stirred at room temperature under a hydrogen atmosphere for 60 minutes. The completion of the reaction was monitored by TLC. The reaction mixture was filtered through celite, and the filtrate was concentrated under reduced pressure to obtain a crude product. The obtained crude product was separated and purified by silica gel column chromatography (petroleum ether:ethyl acetate = 2:1) to give compound **A1R-c** as a pale yellow solid (500 mg, yield 90%). MS m/z 334.1 [M+H]⁺.

Compound **A1R-d** was synthesized according to the method in the patent (WO2020/231808, 2020, A1). Compound **A1R-c** (75 mg, 0.22 mmol) and compound **A1R-d** (91 mg, 0.24 mmol) were dissolved in N,N-dimethylformamide (3 mL), and then trifluoroacetic acid (25 mg, 0.22 mmol) was added. The reaction mixture was stirred at 100 °C overnight. After the reaction was completed, the mixture was diluted with water. The mixture was extracted with ethyl acetate (3×20 mL). The combined organic phases were washed with saturated brine, dried over anhydrous sodium sulfate, filtered and concentrated under reduced pressure. The obtained crude product was separated and purified by silica gel column chromatography (dichloromethane:methanol = 20:1) to give compound **A1R-e** as a yellow solid (90 mg, yield 62%). MS m/z 650.2 [M+H]⁺.

Compound **A1R-e** (90 mg, 0.14 mmol) was dissolved in dichloromethane (3 mL), and then trifluoroacetic acid (1 mL) was added. The reaction mixture was stirred at room temperature for 1 hour. The completion of the reaction was monitored by TLC. The reaction mixture was concentrated under reduced pressure, and then methanol (5 mL) and ammonia water (1 mL) were added, followed by stirring at room temperature for 15 minutes. The mixture was concentrated under reduced pressure, and the obtained crude product was separated and purified by silica gel column chromatography (dichloromethane:methanol = 15:1, 2% ammonia water) to give compound **A4R** as a white solid (45 mg, yield 59%). ¹H NMR (500 MHz, DMSO-d6) *δ* 8.53 (s, 1H), 8.03 (s, 1H), 6.94 (s, 1H), 6.65 (d, J = 10.3 Hz, 2H), 4.14 (dd, J = 10.6, 2.4 Hz, 1H), 3.89 - 3.75 (m, 1H), 3.62 (dd, J = 8.8, 5.9 Hz, 4H), 3.38 (s, 2H), 3.24 (s, 4H), 2.99 (d, J = 12.2 Hz, 1H), 2.93 - 2.79 (m, 2H), 2.71 (td, J = 12.1, 3.2 Hz, 1H), 2.67 - 2.54 (m, 2H), 2.50 - 2.40 (m, 5H), 2.27 (t, J = 11.4 Hz, 1H), 1.58 (s, 2H), 1.03 (t, J = 7.5 Hz, 3H). MS m/z 550.2 [M+H]+.

Compound **A4R** (20 mg, 0.04 mmol) and paraformaldehyde (20 mg) were dissolved in 1,2-dichloroethane (2 mL), and then glacial acetic acid (0.1 mL) was added. The reaction mixture was stirred at 60 °C for 30 minutes and then cooled to room temperature. Sodium triacetoxyborohydride (25 mg, 0.12 mmol) was added to the reaction mixture at room temperature, and then the mixture was stirred at room temperature for 60 minutes. After the reaction was completed, the mixture was diluted with water. The mixture was extracted with dichloromethane (3×10 mL). The combined organic phases were washed with saturated brine, dried over anhydrous sodium sulfate, filtered and concentrated under reduced pressure. The obtained crude product was separated and purified by silica gel column chromatography (dichloromethane:methanol = 15:1, 2% ammonia water) to give compound **A1R** as a white solid (10 mg, yield 49%). ¹H NMR (500 MHz, DMSO-d6) *δ* 8.54 (s, 1H), 8.03 (s, 1H), 7.00 - 6.88 (m, 1H), 6.78 - 6.58 (m, 2H), 4.18 (dd, J = 10.6, 2.6 Hz, 1H), 3.85 (dd, J = 10.4, 9.2 Hz, 1H), 3.72 (d, J = 11.1 Hz, 1H), 3.66 - 3.51 (m, 3H), 3.38 (s, 2H), 3.30 - 3.09 (m, 4H), 3.06 - 2.96 (m, 1H), 2.84 (dd, J = 39.5, 8.7 Hz, 2H), 2.68 - 2.56 (m, 2H), 2.49 - 2.41 (m, 2H), 2.23 (s, 3H), 2.15 - 1.95 (m, 2H), 1.84 - 1.62 (m, 2H), 1.62 - 1.45 (m, 2H), 1.03 (t, J = 7.5 Hz, 3H). MS m/z 564.2 [M+H]⁺.

### Example 2: Preparation of Compound A2R

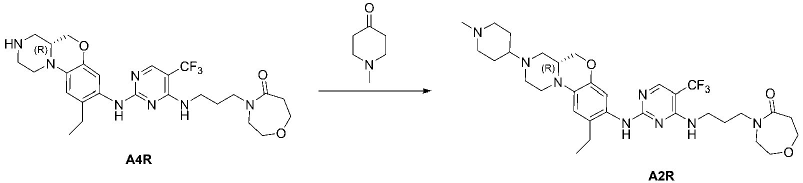

Compound **A4R** (20 mg, 0.04 mmol) and N-methyl-4-piperidone (14 mg, 0.12 mmol) were dissolved in dimethylacetamide (2 mL), followed by the addition of glacial acetic acid (0.1 mL). The reaction mixture was stirred at 60 °C for 30 minutes, then cooled to room temperature. Sodium triacetoxyborohydride (25 mg, 0.12 mmol) was added to the reaction mixture at room temperature. The reaction mixture was then heated to 75 °C and stirred at 75 °C for 60 minutes. After the reaction was completed, the mixture was diluted with water. The mixture was extracted with ethyl acetate (3×10 mL). The combined organic phases were washed with saturated brine, dried over anhydrous sodium sulfate, filtered, and concentrated under reduced pressure. The obtained crude product was separated and purified by silica gel column chromatography (dichloromethane:methanol = 15:1, 2% ammonia water) to give compound **A2R** as a white solid (5 mg, yield 21%). ¹H NMR (500 MHz, DMSO-d6) *δ* 8.54 (s, 1H), 8.03 (s, 1H), 7.03 - 6.82 (m, 1H), 6.78 - 6.54 (m, 2H), 4.19 (dd, J = 10.5, 2.4 Hz, 1H), 3.84 (dd, J = 10.3, 9.4 Hz, 1H), 3.78 - 3.68 (m, 1H), 3.67 - 3.51 (m, 3H), 3.49 - 3.34 (m, 2H), 3.30 - 3.05 (m, 4H), 3.04 - 2.72 (m, 5H), 2.68 - 2.51 (m, 4H), 2.49 - 2.39 (m, 2H), 2.33 - 2.24 (m, 1H), 2.17 (s, 3H), 2.08 - 1.78 (m, 4H), 1.78 - 1.66 (m, 2H), 1.66 - 1.50 (m, 2H), 1.50 - 1.34 (m, 2H), 1.03 (t, J = 7.5 Hz, 3H). MS m/z 647.3 [M+H]⁺.

### Example 3: Preparation of Compound A3R

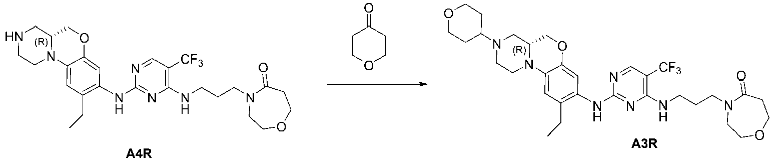

Compound **A4R** (15 mg, 0.04 mmol), tetrahydropyranone (12 mg, 0.12 mmol), glacial acetic acid (0.1 mL) and sodium cyanoborohydride (13 mg, 0.20 mmol) were dissolved in methanol (2 mL) and stirred at room temperature overnight. After the reaction was completed, the mixture was diluted with water. The mixture was extracted with ethyl acetate (3×10 mL). The combined organic phases were washed with saturated brine, dried over anhydrous sodium sulfate, filtered and concentrated under reduced pressure. The obtained crude product was separated and purified by silica gel column chromatography (dichloromethane:methanol = 15:1, 2% ammonia water) to give compound **A3R** as a white solid (3 mg, yield 17%). ¹H NMR (500 MHz, DMSO-d6) *δ* 8.54 (s, 1H), 8.03 (s, 1H), 7.00 - 6.88 (m, 1H), 6.77 - 6.56 (m, 2H), 4.20 (d, J = 9.5 Hz, 1H), 3.99 - 3.78 (m, 3H), 3.78 - 3.70 (m, 1H), 3.69 - 3.53 (m, 3H), 3.46 - 3.33 (m, 2H), 3.31 - 3.10 (m, 6H), 3.06 - 2.76 (m, 3H), 2.66 - 2.53 (m, 2H), 2.49 - 2.37 (m, 4H), 2.34 - 2.23 (m, 1H), 2.02 - 1.81 (m, 2H), 1.79 - 1.67 (m, 2H), 1.65 - 1.51 (m, 2H), 1.50 - 1.32 (m, 2H), 1.03 (t, J = 7.5 Hz, 3H). MS m/z 634.2 [M+H]⁺.

### Example 4: Preparation of Compounds A5R and A8R

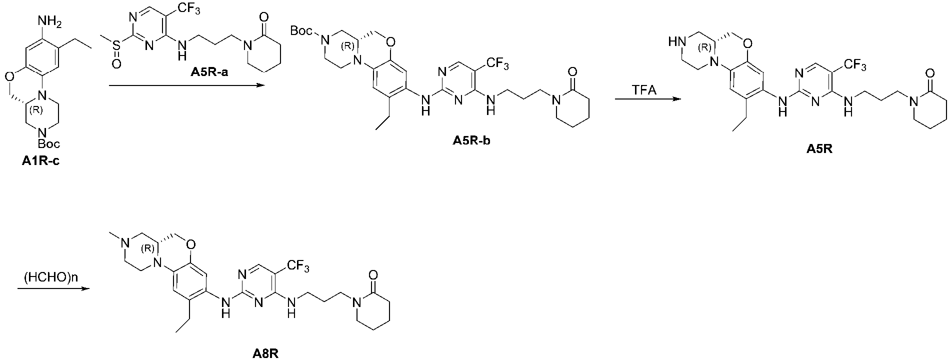

Compound **A5R-a** was synthesized according to the method described in the patent (WO2020/231808, 2020, A1). Compound **A1R-c** (160 mg, 0.48 mmol) and compound **A5R-a** (193 mg, 0.53 mmol) were dissolved in N,N-dimethylformamide (3 mL), and trifluoroacetic acid (58 mg, 0.48 mmol) was added. The reaction mixture was stirred at 100 °C for 4 hours. After the reaction was completed, the mixture was diluted with water. The mixture was extracted with ethyl acetate (3×20 mL). The combined organic phases were washed with saturated brine, dried over anhydrous sodium sulfate, filtered, and concentrated under reduced pressure. The obtained crude product was separated and purified by silica gel column chromatography (dichloromethane:methanol = 20:1) to give compound **A5R-b** as a yellow solid (250 mg, yield 82%). MS m/z 634.5 [M+H]⁻.

Compound **A5R-b** (250 mg, 0.39 mmol) was dissolved in dichloromethane (3 mL), and trifluoroacetic acid (1 mL) was added. The reaction mixture was stirred at room temperature for 1 hour. The completion of the reaction was monitored by TLC. The reaction mixture was concentrated under reduced pressure, then methanol (5 mL) and ammonia water (1 mL) were added, followed by stirring at room temperature for 15 minutes. The mixture was concentrated under reduced pressure, and the obtained crude product was separated and purified by silica gel column chromatography (dichloromethane:methanol = 15:1, 2% ammonia water) to give compound **A5R** as a white solid (125 mg, yield 59%). ¹H NMR (500 MHz, DMSO-d6) *δ* 8.54 (s, 1H), 8.02 (s, 1H), 7.05 - 6.91 (m, 1H), 6.75 - 6.55 (m, 2H), 4.15 (dd, J = 10.6, 2.4 Hz, 1H), 3.87 - 3.75 (m, 1H), 3.67 - 3.56 (m, 1H), 3.31 - 3.05 (m, 6H), 3.05 - 2.94 (m, 1H), 2.94 - 2.80 (m, 2H), 2.76 - 2.66 (m, 2H), 2.49 - 2.40 (m, 3H), 2.33 - 2.15 (m, 3H), 1.80 - 1.52 (m, 6H), 1.03 (t, J = 7.5 Hz, 3H). MS m/z 534.3 [M+H]⁺.

Compound **A5R** (25 mg, 0.05 mmol) and paraformaldehyde (25 mg) were dissolved in 1,2-dichloroethane (2 mL), and glacial acetic acid (0.1 mL) was added. The reaction mixture was stirred at room temperature for 2 hours. Sodium triacetoxyborohydride (50 mg, 0.24 mmol) was added to the reaction mixture at room temperature, and then the mixture was stirred at room temperature for 60 minutes. After the reaction was completed, the mixture was diluted with water. The mixture was extracted with dichloromethane (3×10 mL). The combined organic phases were washed with saturated brine, dried over anhydrous sodium sulfate, filtered, and concentrated under reduced pressure. The obtained crude product was separated and purified by silica gel column chromatography (dichloromethane:methanol = 15:1, 2% ammonia water) to give compound **A8R** as a white solid (17 mg, yield 66%). ¹H NMR (500 MHz, DMSO-d6) *δ* 8.55 (s, 1H), 8.02 (s, 1H), 6.99 (t, J = 5.7 Hz, 1H), 6.67 (s, 2H), 4.18 (dd, J = 10.6, 2.6 Hz, 1H), 3.84 (dd, J = 10.5, 9.1 Hz, 1H), 3.77 - 3.62 (m, 1H), 3.31 - 3.06 (m, 6H), 3.04 - 2.95 (m, 1H), 2.86 (d, J = 11.1 Hz, 1H), 2.82 - 2.74 (m, 1H), 2.66 - 2.56 (m, 1H), 2.49 - 2.39 (m, 3H), 2.34 - 2.11 (m, 5H), 2.11 - 2.03 (m, 1H), 1.79 - 1.51 (m, 6H), 1.03 (t, J = 7.5 Hz, 3H). MS m/z 548.3 [M+H]⁻.

### Example 5: Preparation of Compound A6R

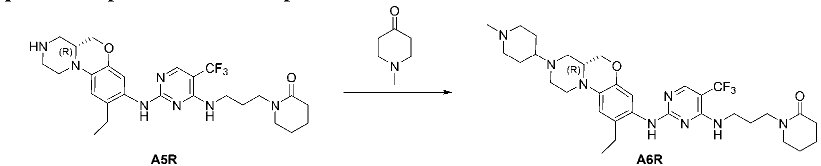

Compound **A5R** (25 mg, 0.05 mmol) and N-methyl-4-piperidone (23 mg, 0.20 mmol) were dissolved in dimethylacetamide (2 mL), followed by the addition of glacial acetic acid (0.1 mL). The reaction mixture was stirred at 60 °C for 30 minutes, then cooled to room temperature. Sodium triacetoxyborohydride (42 mg, 0.20 mmol) was added to the reaction mixture. The reaction mixture was then heated to 75 °C and stirred at 75 °C for 60 minutes. After the reaction was completed, the mixture was diluted with water. The mixture was extracted with ethyl acetate (3×10 mL). The combined organic phases were washed with saturated brine, dried over anhydrous sodium sulfate, filtered, and concentrated under reduced pressure. The obtained crude product was separated and purified by silica gel column chromatography (dichloromethane:methanol = 15:1, 2% ammonia water) to give compound **A6R** as a white solid (25 mg, yield 85%). ¹H NMR (500 MHz, DMSO-d6) *δ* 8.54 (s, 1H), 8.02 (s, 1H), 6.98 (t, J = 5.7 Hz, 1H), 6.79 - 6.54 (m, 2H), 4.19 (dd, J = 10.5, 2.4 Hz, 1H), 3.84 (dd, J = 10.4, 9.2 Hz, 1H), 3.76 - 3.63 (m, 1H), 3.30 - 3.04 (m, 6H), 3.03 - 2.86 (m, 3H), 2.79 (d, J = 11.2 Hz, 2H), 2.60 - 2.52 (m, 1H), 2.49 - 2.39 (m, 3H), 2.34 - 2.24 (m, 1H), 2.23 - 2.01 (m, 6H), 1.93 - 1.78 (m, 3H), 1.77 - 1.51 (m, 7H), 1.49 - 1.34 (m, 2H), 1.03 (t, J = 7.5 Hz, 3H). MS m/z 631.4 [M+H]⁻.

### Example 6: Preparation of Compound A7R

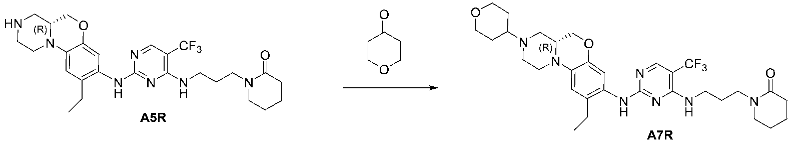

Compound **A5R** (22 mg, 0.04 mmol), tetrahydropyranone (12 mg, 0.12 mmol), glacial acetic acid (0.1 mL) and sodium cyanoborohydride (13 mg, 0.20 mmol) were dissolved in methanol (2 mL) and stirred at room temperature overnight. After the reaction was completed, the mixture was diluted with water. The mixture was extracted with ethyl acetate (3×10 mL). The combined organic phases were washed with saturated brine, dried over anhydrous sodium sulfate, filtered and concentrated under reduced pressure. The obtained crude product was separated and purified by silica gel column chromatography (dichloromethane:methanol = 15:1, 2% ammonia water) to give compound **A7R** as a white solid (9 mg, yield 35%). ¹H NMR (500 MHz, DMSO-*d*₆) *δ* 8.54 (s, 1H), 8.02 (s, 1H), 6.98 (t, *J =* 5.8 Hz, 1H), 6.76 - 6.54 (m, 2H), 4.20 (dd, *J =* 10.5, 2.5 Hz, 1H), 3.94 - 3.80 (m, 3H), 3.78 - 3.68 (m, 1H), 3.30 - 3.05 (m, 8H), 3.05 - 2.87 (m, 3H), 2.61 - 2.52 (m, 1H), 2.49 - 2.38 (m, 3H), 2.33 - 2.24 (m, 1H), 2.20 (t, *J* = 5.9 Hz, 2H), 1.95 - 1.80 (m, 2H), 1.77 - 1.53 (m, 7H), 1.50 - 1.33 (m, 2H), 1.03 (t, *J =* 7.5 Hz, 3H). MS *m*/*z* 618.3 [M+H]⁺.

### Example 7: Preparation of Compound A9R

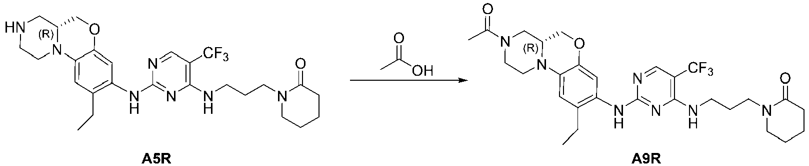

Compound **A5R** (18 mg, 0.03 mmol) and glacial acetic acid (18 mg, 0.30 mmol) were dissolved in pyridine (2 mL), and 1-(3-dimethylaminopropyl)-3-ethylcarbodiimide hydrochloride (19 mg, 0.10 mmol) was added. The mixture was stirred at room temperature for one hour. After the reaction was completed, the mixture was diluted with water. The mixture was extracted with ethyl acetate (3×10 mL). The combined organic phases were washed with saturated brine, dried over anhydrous sodium sulfate, filtered, and concentrated under reduced pressure. The obtained crude product was separated and purified by silica gel column chromatography (dichloromethane:methanol = 15:1, 2% ammonia water) to give compound **A9R** as a white solid (9 mg, yield 46%). MS m/z 576.3 [M+H]⁺.

### Example 8: Preparation of Compounds B1R and B3R

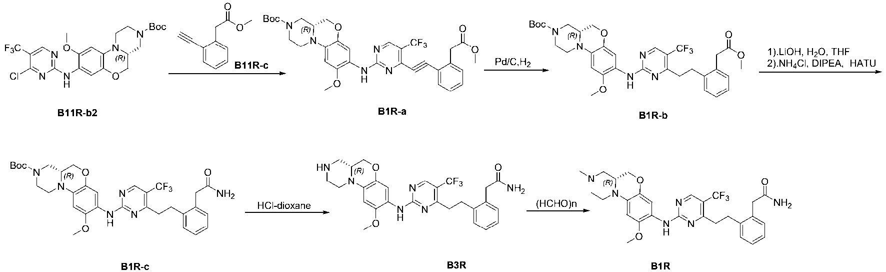

Compound **B11R-b2** (420 mg, 0.81 mmol), triphenylphosphine (21 mg, 0.08 mmol), bis(triphenylphosphine)palladium dichloride (56 mg, 0.08 mmol), and cuprous iodide (15 mg, 0.08 mmol) were dissolved in N,N-dimethylformamide (10 mL). Under nitrogen protection, compound **B11R-c** (170 mg, 0.98 mmol) and triethylamine (327 mg, 3.24 mmol) were added successively. The mixed system waSR^{e}acted at 120°C for one hour. After the reaction was completed as monitored by LCMS, the reaction solution was evaporated to dryness under reduced pressure to obtain a crude product. The crude product was purified by silica gel column chromatography (petroleum ether:ethyl acetate = 4:1) to obtain compound B1R-a as a yellow solid (500 mg, yield 94%). MS m/z 654.3 [M+H]⁺.

At room temperature, compound **B1R-a** (500 mg, 0.76 mmol) and palladium on carbon (10%, 100 mg) were added to N,N-dimethylformamide (15 mL). The reaction mixture was stirred at room temperature under a hydrogen atmosphere for 18 hours. After the reaction was completed, the mixture was filtered through Celite, and the filtrate was concentrated under reduced pressure to obtain a crude product. The crude product was purified by silica gel column chromatography (dichloromethane:methanol = 20:1) to obtain compound **B1R-b** as a gray solid (360 mg, yield 71%). MS m/z 658.3 [M+H]⁺.

Compound **B1R-b** (337 mg, 0.51 mmol) was dissolved in tetrahydrofuran and water (1:1, 15 mL), then lithium hydroxide (61 mg, 2.56 mmol) was added, and the mixture was stirred at room temperature overnight. After the reaction was completed, the reaction solution was evaporated to dryness under reduced pressure, then washed with saturated sodium bicarbonate, extracted with ethyl acetate. The organic phase was washed with saturated brine, dried over anhydrous sodium sulfate, filtered, and the filtrate was dried under reduced pressure to obtain a crude product. The crude product was dissolved in a mixed solution of N,N-dimethylformamide and acetonitrile, and ammonium chloride (54 mg, 1.02 mmol), N,N-diisopropylethylamine (199 mg, 1.53 mmol), and HATU (387 mg, 1.02 mmol) were added successively. The reaction was carried out at room temperature for 1 hour. After the reaction was completed, the reaction solution was evaporated to dryness under reduced pressure to obtain a crude product. The crude product was purified by silica gel column chromatography (dichloromethane:methanol = 20:1) to obtain compound **B1R-c** as a gray solid (300 mg, yield 91%). MS m/z 643.3 [M+H] .

Compound **B1R-c** (200 mg, 0.31 mmol) was dissolved in methanol (10 mL), and then a solution of HCl in dioxane (4.0 M, 2 mL) was added. The reaction mixture was stirred at 40°C for 2 hours. The completion of the reaction was monitored by TLC. After the mixture was cooled to room temperature, it was concentrated under reduced pressure. The obtained mixture was dissolved in a small amount of methanol, neutralized with ammonia water, and then concentrated under reduced pressure. The resulting crude product was purified by silica gel column chromatography (dichloromethane:methanol = 15:1, 2% ammonia water) to obtain compound B3R as a yellow solid (150 mg, yield 89%). ¹H NMR (500 MHz, DMSO-*d*₆) *δ* 8.77 (s, 1H), 8.53 (s, 1H), 7.39 (s, 1H), 7.24 - 7.19 (m, 1H), 7.19 - 7.04 (m, 4H), 6.89 (s, 1H), 6.57 (s, 1H), 4.15 (dd, J = 10.6, 2.5 Hz, 1H), 3.84 - 3.78 (m, 1H), 3.74 (s, 3H), 3.68 (d, J = 11.0 Hz, 1H), 3.47 (s, 2H), 3.07 - 2.85 (m, 7H), 2.72 (td, J = 12.0, 3.0 Hz, 1H), 2.54 (dd, J = 11.7, 3.3 Hz, 1H), 2.28 (t, J = 11.3 Hz, 1H), 2.00 (dd, J = 15.9, 8.2 Hz, 1H). MS m/z 543.2 [M+H]⁻.

Compound **B3R** (45 mg, 0.08 mmol), paraformaldehyde (4 mg, 0.12 mmol) and zinc chloride (22 mg, 0.16 mmol) were successively dissolved in methanol (1 mL), and then sodium cyanoborohydride (15 mg, 0.24 mmol) was slowly added. The reaction mixture was stirred at 75°C for 1 hour. The completion of the reaction was monitored by TLC. The mixture was concentrated under reduced pressure, and the obtained crude product was purified by silica gel column chromatography (dichloromethane:methanol = 20:1, 2% ammonia water) to obtain compound **B1R** as a yellow solid (20 mg, yield 43%). ¹H NMR (500 MHz, DMSO-*d*₆) *δ* 8.77 (s, 1H), 8.54 (s, 1H), 7.39 (s, 1H), 7.24 - 7.19 (m, 1H), 7.19 - 7.05 (m, 4H), 6.89 (s, 1H), 6.60 (s, 1H), 4.18 (dd, *J =* 10.6, 2.6 Hz, 1H), 3.85 (dd, *J =* 10.5, 8.9 Hz, 1H), 3.79 (d, *J =* 11.5 Hz, 1H), 3.75 (s, 3H), 3.47 (s, 2H), 3.08 - 3.00 (m, 3H), 2.95 (dd, *J =* 10.2, 5.5 Hz, 2H), 2.88 (d, *J =* 10.9 Hz, 1H), 2.79 (d, *J =* 10.3 Hz, 1H), 2.68 (td, *J =* 11.7, 2.9 Hz, 1H), 2.23 (s, 3H), 2.13 - 2.05 (m, 1H), 1.70 (t, J = 10.5 Hz, 1H). MS m/z 557.3 [M+H]⁺.

### Example 9: Preparation of Compound B2R

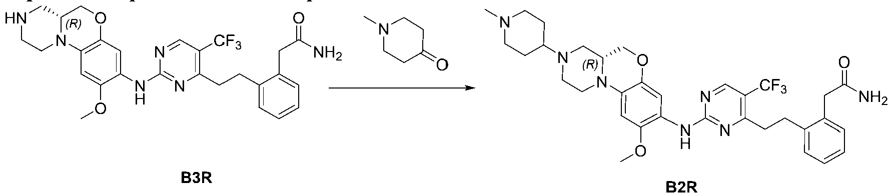

Compound **B3R** (45 mg, 0.08 mmol), N-methyl-4-piperidone (14 mg, 0.12 mmol) and zinc chloride (22 mg, 0.16 mmol) were sequentially dissolved in methanol (1 mL), followed by the addition of sodium cyanoborohydride (15 mg, 0.24 mmol). The reaction mixture was stirred at 75 °C for 1 hour. After the reaction was completed as monitored by TLC, the mixture was concentrated under reduced pressure. The obtained crude product was purified by silica gel column chromatography (dichloromethane:methanol = 20:1, 2% ammonia water) to afford compound **B2R** as a yellow solid (26 mg, yield 49%). ¹H NMR (500 MHz, DMSO-*d*₆) *δ* 8.77 (s, 1H), 8.53 (s, 1H), 7.39 (s, 1H), 7.24 - 7.19 (m, 1H), 7.19 - 7.03 (m, 4H), 6.89 (s, 1H), 6.59 (s, 1H), 4.19 (dd, *J =* 10.6, 2.6 Hz, 1H), 3.87 - 3.77 (m, 2H), 3.74 (s, 3H), 3.47 (s, 2H), 3.07 - 2.88 (m, 7H), 2.80 (d, *J =* 11.2 Hz, 2H), 2.66 - 2.57 (m, 1H), 2.31 (td, *J =* 11.2, 2.7 Hz, 1H), 2.17 (d, *J =* 3.6 Hz, 1H), 2.15 (s, 3H), 1.91 - 1.81 (m, 3H), 1.74 (d, *J =* 10.9 Hz, 2H), 1.49 - 1.37 (m, 2H). MS m/z 640.3 [M+H]⁺.

### Example 10: Preparation of Compound B4R

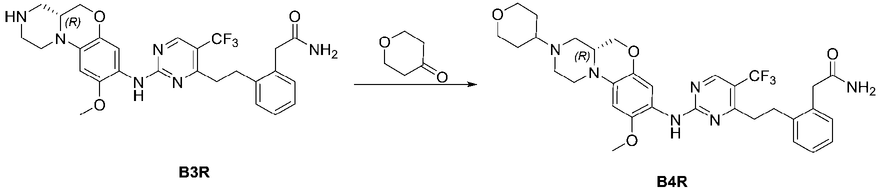

Compound **B3R** (40 mg, 0.07 mmol), tetrahydropyranone (11 mg, 0.11 mmol) and zinc chloride (19 mg, 0.14 mmol) were sequentially dissolved in methanol (1 mL), followed by the addition of sodium cyanoborohydride (25 mg, 0.21 mmol). The reaction mixture was stirred at 75 °C for 1 hour. After the reaction was completed as monitored by TLC, the mixture was concentrated under reduced pressure. The obtained crude product was purified by silica gel column chromatography (dichloromethane:methanol = 20:1, 2% ammonia water) to afford compound **B4R** as a yellow solid (26 mg, yield 57%). ¹H NMR (500 MHz, DMSO-*d*₆) *δ* 8.77 (s, 1H), 8.53 (s, 1H), 7.39 (s, 1H), 7.26 - 7.20 (m, 1H), 7.19 - 7.02 (m, 4H), 6.89 (s, 1H), 6.59 (s, 1H), 4.20 (dd, *J =* 10.5, 2.5 Hz, 1H), 3.93 - 3.87 (m, 2H), 3.86 - 3.78 (m, 2H), 3.75 (s, 3H), 3.47 (s, 2H), 3.30 - 3.25 (m, 2H), 3.08 - 2.98 (m, 4H), 2.98 - 2.91 (m, 3H), 2.67 - 2.59 (m, 1H), 2.46 - 2.40 (m, 1H), 2.35 - 2.27 (m, 1H), 1.88 (t, *J =* 10.4 Hz, 1H), 1.73 (d, *J=* 12.3 Hz, 2H), 1.48-1.39 (m, 2H). MS m/z 627.3[M+H]⁺.

### Example 11: Preparation of Compound B5R

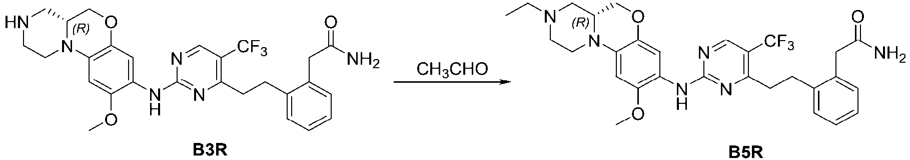

Compound **B3R** (30 mg, 0.05 mmol), acetaldehyde (4 mg, 0.08 mmol) and zinc chloride (14 mg, 0.10 mmol) were sequentially dissolved in methanol (1 mL), followed by the addition of sodium cyanoborohydride (9 mg, 0.15 mmol). The reaction mixture was stirred at 75 °C for 1 hour. After the reaction was completed as monitored by TLC, the mixture was concentrated under reduced pressure. The obtained crude product was purified by silica gel column chromatography (dichloromethane:methanol = 20:1, 2% ammonia water) to afford compound **B5R** as a yellow solid (14 mg, yield 44%). ¹H NMR (500 MHz, DMSO-*d*₆) *δ* 8.77 (s, 1H), 8.53 (s, 1H), 7.39 (br, 1H), 7.22 (d, *J =* 5.8 Hz, 1H), 7.16 (s, 4H), 6.89 (s, 1H), 6.60 (s, 1H), 4.20 (dd, *J =* 10.6, 2.5 Hz, 1H), 3.88 - 3.77 (m, 2H), 3.75 (s, 3H), 3.47 (s, 2H), 3.07 - 2.86 (m, 7H), 2.70 - 2.61 (m, 1H), 2.43 - 2.31 (m, 2H), 2.1 - 2.05 (m, 1H), 1.67 (t, *J* = 10.6 Hz, 1H), 1.04 (t, *J =* 7.2 Hz, 3H). MS *m*/*z* 571.3 [M+H]⁺.

### Example 12: Preparation of Compound B6R

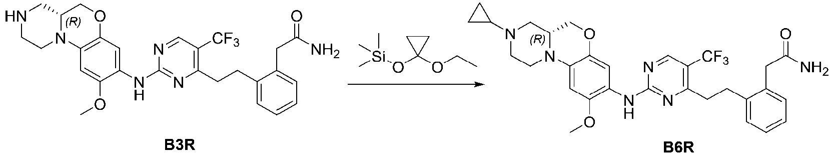

Compound **B3R** (30 mg, 0.05 mmol) and 1-ethoxy-1-trimethylsilyloxycyclopropane (96 mg, 0.55 mmol) were dissolved in methanol (1 mL). Then, one drop of acetic acid was added. After stirring at room temperature for one hour, sodium cyanoborohydride (34 mg, 0.55 mmol) was added. The reaction mixture was continuously stirred at room temperature for 12 hours. After the reaction was completed as monitored by TLC, the mixture was concentrated under reduced pressure. The obtained crude product was purified by silica gel column chromatography (dichloromethane:methanol = 20:1, 2% ammonia water) to afford compound B6R as a yellow solid (5 mg, yield 24%). ¹H NMR (500 MHz, DMSO-*d*₆) *δ* 8.77 (s, 1H), 8.53 (s, 1H), 7.39 (br, 1H), 7.23 - 7.20 (m, 1H), 7.19 - 7.08 (m, 4H), 6.89 (s, 1H), 6.60 (s, 1H), 4.21 (dd, *J =* 10.7, 2.5 Hz, 1H), 3.88 - 3.78 (m, 2H), 3.74 (s, 3H), 3.47 (s, 2H), 3.06 - 2.99 (m, 3H), 2.97 - 2.91 (m, 4H), 2.62 - 2.55 (m, 1H), 2.42 - 2.35 (m, 1H), 1.99 (t, *J =* 11 Hz, 1H), 1.70 - 1.64 (m, 1H), 0.48 - 0.42(m, 2H), 0.40 - 0.33(m, 2H). MS *m*/*z* 583.3 [M+H]⁺.

### Example 13: Preparation of Compound B7R

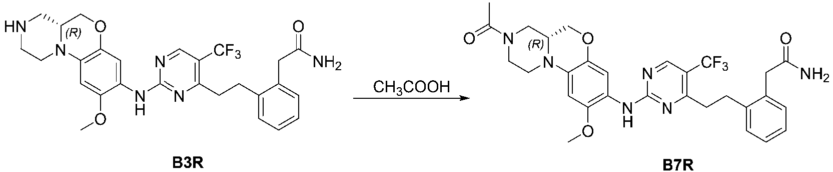

Compound **B3R** (25 mg, 0.05 mmol), acetic acid (4 mg, 0.07 mmol) and N,N-diisopropylethylamine (19 mg, 0.15 mmol) were dissolved in acetonitrile (1 mL), followed by the addition of HATU (19 mg, 0.05 mmol). The reaction was carried out at room temperature for 1 hour. After the reaction was completed, the reaction solution was evaporated to dryness under reduced pressure to obtain a crude product. The crude product was purified by silica gel column chromatography (dichloromethane:methanol = 20:1) to afford compound **B7R** as a gray solid (15.20 mg, yield 56%). ¹H NMR (500 MHz, DMSO-*d*₆) *δ* 8.79 (s, 1H), 8.55 (s, 1H), 7.39 (br, 1H), 7.24 - 7.20 (m, 1H), 7.19 - 7.07 (m, 4H), 6.90 (s, 1H), 6.66 (s, 1H), 4.43 (dd, *J =* 22.5, 12.4 Hz, 1H), 4.28 (dd, *J =* 10.7, 2.3 Hz, 1H), 3.94 - 3.87 (m, 2H), 3.76 (s, 3H), 3.47 (s, 2H), 3.30 - 3.22 (m, 1H), 3.08 - 3.00 (m, 2H), 2.98 - 2.92 (m, 2H), 2.91 - 2.83 (m, 1H), 2.77 - 2.65 (m, 1H), 2.58 - 2.51 (m, 1H), 2.42 - 2.33 (m, 1H), 2.07 (d, *J =* 5.6 Hz, 3H). MS *m*/*z* 585.2 [M+H]⁺.

### Example 14: Preparation of Compound B8R

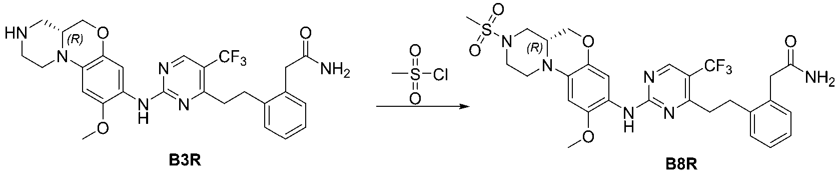

Compound **B3R** (25 mg, 0.05 mmol) and N,N-diisopropylethylamine (19 mg, 0.15 mmol) were dissolved in dichloromethane (1 mL). Methanesulfonyl chloride (6 mg, 0.05 mmol) was added under ice bath condition, and the reaction was carried out at room temperature for 1 hour. After the reaction was completed, the reaction solution was evaporated to dryness under reduced pressure to obtain a crude product. The crude product was purified by silica gel column chromatography (dichloromethane:methanol = 20:1) to afford compound **B8R** as a gray solid (6.34 mg, yield 22%). ¹H NMR (500 MHz, DMSO-*d*₆) *δ* 8.80 (s, 1H), 8.55 (s, 1H), 7.39 (br., 1H), 7.24 - 7.21 (m, 1H), 7.20 - 7.07 (m, 4H), 6.89 (s, 1H), 6.69 (s, 1H), 4.30 (dd, *J =* 10.8, 2.6 Hz, 1H), 4.05 (d, *J =* 12.3, 1H), 3.90 (dd, *J =* 10.8, 8.1, 1H), 3.76 (s, 3H), 3.60 (d, *J =* 11.5, 2H), 3.47 (s, 2H), 3.17 (br., 1H), 3.04 (dd, *J =* 9.9, 5.3 Hz, 2H), 2.97-2.89 (m, 5H), 2.78 (dd, *J =* 12.0, 9.3 Hz, 1H) 2.60 (t, *J =* 11.2 Hz ,1H), 2.03 - 1.96 (m, 1H). MS *m*/*z* 621.2 [M+H]⁺.

### Example 15: Preparation of Compound B9R

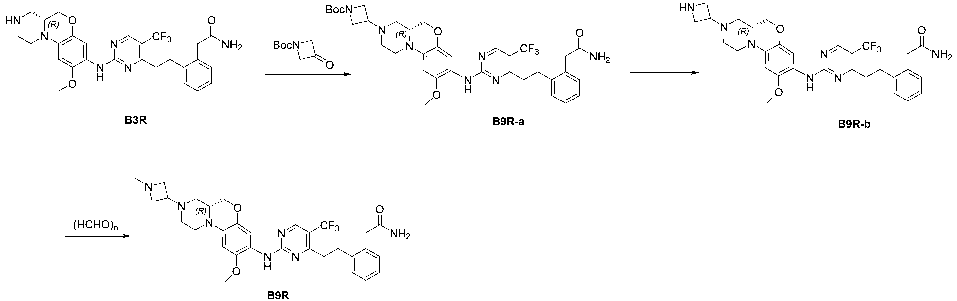

Compound **B3R** (60 mg, 0.11 mmol), 1-Boc-3-azetidinone (28 mg, 0.16 mmol) and zinc chloride (27 mg, 0.22 mmol) were sequentially dissolved in methanol (1 mL), followed by the addition of sodium cyanoborohydride (14 mg, 0.22 mmol). The reaction mixture was stirred at 75 °C for 1 hour. After the reaction was completed as monitored by TLC, the mixture was concentrated under reduced pressure. The obtained crude product was purified by silica gel column chromatography (dichloromethane:methanol = 20:1, 2% ammonia water) to afford compound **B9R-a** as a yellow solid (41 mg, yield 53%). MS m/z 698.3 [M+H]⁻.

Compound **B9R-a** (41 mg, 0.06 mmol) was dissolved in methanol (1 mL), and then a solution of hydrochloric acid in dioxane (4.0 M, 0.5 mL) was added. The reaction mixture was stirred at 40 °C for 2 hours. After the reaction was completed as monitored by TLC, the mixture was cooled to room temperature and concentrated under reduced pressure. The obtained mixture was dissolved in a small amount of methanol, neutralized with ammonia water, and then concentrated under reduced pressure. The resulting crude product was purified by silica gel column chromatography (dichloromethane:methanol = 15:1, 2% ammonia water) to obtain compound **B9R-b** as a yellow solid (35 mg, yield 99%).

Compound **B9R-b** (35 mg, 0.06 mmol), paraformaldehyde (9 mg, 0.30 mmol) and zinc chloride (16 mg, 0.12 mmol) were sequentially dissolved in methanol (1 mL), followed by the addition of sodium cyanoborohydride (7 mg, 0.12 mmol). The reaction mixture was stirred at 75 °C for 1 hour. After the reaction was completed as monitored by TLC, the mixture was concentrated under reduced pressure. The obtained crude product was purified by silica gel column chromatography (dichloromethane:methanol = 20:1, 2% ammonia water) to afford compound **B9R** as a yellow solid (22 mg, yield 63%). ¹H NMR (500 MHz, DMSO-*d*₆) *δ* 8.78 (s, 1H), 8.53 (s, 1H), 7.39 (br., 1H), 7.25 - 7.20 (m, 1H), 7.14 (dd, *J* = 9.7, 5.6 Hz, 4H), 6.89 (s, 1H), 6.59 (s, 1H), 4.19 (dd, *J* = 10.6, 2.5 Hz, 1H), 3.87 - 3.77 (m, 2H), 3.74 (s, 3H), 3.47 (s, 2H), 3.41 - 3.35 (m, 2H), 3.08 - 2.99 (m, 3H), 2.98 - 2.91 (m, 2H), 2.89 - 2.71 (m, 5H), 2.69 - 2.60 (m, 1H), 2.23 (s, 3H), 2.04 - 1.95 (m, 1H), 1.60 (t, *J =* 10.6 Hz, 1H). MS *m*/*z* 612.4 [M+H]⁺.

### Example 16: Preparation of Compound B10R

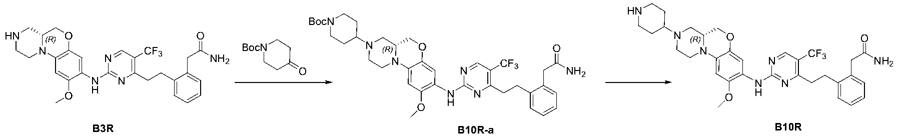

Compound **B3R** (40 mg, 0.07 mmol), N-tert-butoxycarbonyl-4-piperidone (22 mg, 0.11 mmol) and zinc chloride (49 mg, 0.15 mmol) were sequentially dissolved in methanol (1 mL), followed by the addition of sodium cyanoborohydride (14 mg, 0.22 mmol). The reaction mixture was stirred at 75 °C for 1 hour. After the reaction was completed as monitored by TLC, the mixture was concentrated under reduced pressure. The obtained crude product was purified by silica gel column chromatography (dichloromethane:methanol = 20:1, 2% ammonia water) to afford compound **B10R-a** as a yellow solid (25 mg, yield 47%). MS m/z 726.5 [M+H]⁺.

Compound **B10R-a** (25 mg, 0.03 mmol) was dissolved in methanol (1 mL), and then a solution of hydrochloric acid in dioxane (4.0 M, 0.5 mL) was added. The reaction mixture was stirred at 40 °C for 2 hours. The completion of the reaction was monitored by TLC. After the reaction mixture was cooled to room temperature, it was concentrated under reduced pressure. The obtained residue was dissolved in a small amount of methanol, neutralized with ammonia water, and then concentrated under reduced pressure. The resulting crude product was purified by silica gel column chromatography (dichloromethane:methanol = 15:1, 2% ammonia water) to obtain compound **B10R** as a yellow solid (9.84 mg, yield 47%). ¹H NMR (500 MHz, CD₃OD) *δ* 8.49 (s, 1H), 7.68 (s, 1H), 7.28 - 7.13 (m, 4H), 6.57 (s, 1H), 4.22 (dd, *J* = 10.5, 2.4 Hz, 1H), 3.94 (dd, *J =* 10.4, 9.0 Hz, 1H), 3.84 (s, 3H), 3.79 (d, *J =* 11.7 Hz, 1H), 3.68 (s, 2H), 3.17 - 2.95 (m, 9H), 2.80 - 2.70 (m, 1H), 2.66 - 2.56 (m, 2H), 2.50 - 2.41 (m, 2H), 2.05 (t, *J=* 10.8 Hz, 1H), 1.96 - 1.88 (m, 2H), 1.5 - 1.41 (m, 2H). MS *m*/*z* 626.3 [M+H]⁺.

### Example 17: Preparation of Compound B11R

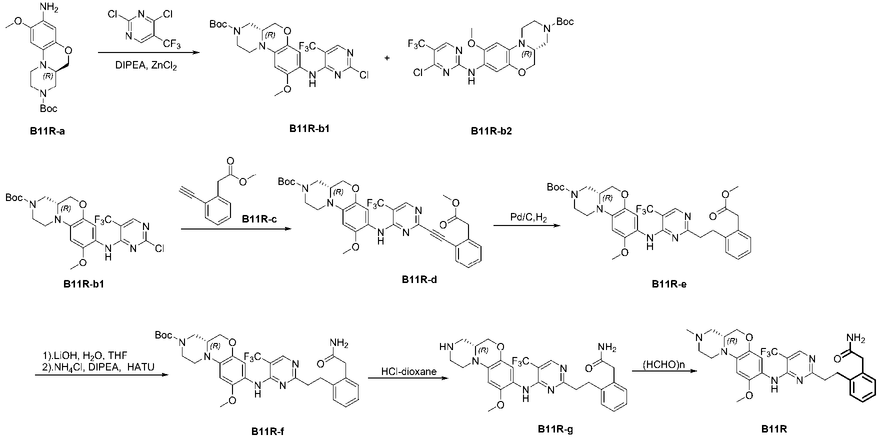

2,4-Dichloro-5-trifluoromethylpyrimidine (1.1 g, 3.28 mmol) was dissolved in dichloromethane (20 mL). Under a nitrogen atmosphere, a tetrahydrofuran solution of zinc chloride (6.56 mL, 1.0 M) was added at -10 °C. After the addition was complete, stirring was continued for one hour. Then, a dichloromethane solution of compound **B11R-a** (1.1 g, 3.28 mmol) and N,N-diisopropylethylamine (996 mg, 9.84 mmol) was slowly added dropwise under an ice bath. Stirring was continued at room temperature for 3 hours. After the reaction was completed as monitored by LCMS, the reaction solution was concentrated under reduced pressure. The obtained crude product was purified by silica gel column chromatography (petroleum ether:ethyl acetate = 5:1) to afford compound **B11R-b1** as a yellow solid (420 mg, yield 25%), MS m/z 516.0, 518.0 [M+H]⁺, and compound **B11R-b2** (760 mg, yield 45%), MS m/z 516.0, 518.0 [M+H]⁺, respectively.

Compound **B11R-b1** (700 mg, 1.36 mmol), triphenylphosphine (36 mg, 0.14 mmol), bis(triphenylphosphine)palladium dichloride (48 mg, 0.07 mmol), and cuprous iodide (26 mg, 0.14 mmol) were dissolved in N,N-dimethylformamide (20 mL). Under nitrogen protection, compound B11R-c (284 mg, 1.63 mmol) and triethylamine (549 mg, 5.44 mmol) were added successively. The mixed system waSR^{e}acted at 120°C for one hour. After the reaction was completed, the reaction solution was evaporated to dryness under reduced pressure to obtain a crude product. The crude product was purified by silica gel column chromatography (petroleum ether:ethyl acetate = 4:1) to obtain compound **B11R-d** as a yellow solid (800 mg, yield 90%). MS m/z 654.2 [M+H]⁺.

At room temperature, compound **B11R-d** (800 mg, 1.22 mmol), palladium on carbon (10%, 100 mg), and triethylamine (99 mg, 0.98 mmol) were added to N,N-dimethylformamide (20 mL). The reaction mixture was stirred at room temperature under a hydrogen atmosphere for 18 hours. After the reaction was completed, the mixture was filtered through Celite, and the filtrate was concentrated under reduced pressure to obtain a crude product. The crude product was purified by silica gel column chromatography (dichloromethane:methanol = 20:1) to obtain compound **B11R-e** as a gray solid (700 mg, yield 87%). MS m/z 658.2 [M+H]⁻.

Compound **B11R-e** (700 mg, 1.06 mmol) was dissolved in tetrahydrofuran and water (1:1, 50 mL), then lithium hydroxide (601 mg, 5.32 mmol) was added, and the mixture was stirred at room temperature overnight. After the reaction was completed, the reaction solution was concentrated under reduced pressure, then washed with saturated sodium bicarbonate, extracted with ethyl acetate. The organic phase was washed with saturated brine, dried over anhydrous sodium sulfate, filtered, and the filtrate was concentrated under reduced pressure to obtain a crude product. The crude product was dissolved in a mixed solution of N,N-dimethylformamide and acetonitrile, and ammonium chloride (84 mg, 1.59 mmol), N,N-diisopropylethylamine (413 mg, 3.18 mmol), and HATU (806 mg, 2.12 mmol) were added successively. The mixed system waSR^{e}acted at room temperature for 1 hour. After the reaction was completed, the reaction solution was evaporated to dryness under reduced pressure to obtain a crude product. The crude product was purified by silica gel column chromatography (dichloromethane:methanol = 20:1) to obtain compound **B11R-f** as a gray solid (700 mg, yield 87%). MS m/z 643.3 [M+H]⁺.

Compound **B11R-f** (300 mg, 0.47 mmol) was dissolved in methanol (10 mL), and then a solution of hydrochloric acid in dioxane (4.0 M, 2 mL) was added. The reaction mixture was stirred at 40°C for 2 hours. The completion of the reaction was monitored by TLC. After the system was cooled to room temperature, it was concentrated under reduced pressure. The obtained mixture was dissolved in a small amount of methanol, neutralized with ammonia water, and then concentrated under reduced pressure. The resulting crude product was purified by silica gel column chromatography (dichloromethane:methanol = 15:1, 2% ammonia water) to obtain compound **B11R-g** as a yellow solid (250 mg, yield 98%).

Compound **B11R-g** (50 mg, 0.09 mmol), paraformaldehyde (6 mg, 0.18 mmol) and zinc chloride (6 mg, 0.18 mmol) were sequentially dissolved in methanol (1 mL), followed by the addition of sodium cyanoborohydride (12 mg, 0.18 mmol). The reaction mixture was stirred at 75°C for 1 hour. After the reaction was completed as monitored by TLC, the reaction mixture was concentrated under reduced pressure. The obtained crude product was purified by silica gel column chromatography (dichloromethane:methanol = 20:1, 2% ammonia water) to afford compound **B11R** as a yellow solid (17 mg, yield 33%). ¹H NMR (500 MHz, DMSO-*d*₆) *δ* 8.54 (s, 1H), 8.04 (s, 1H), 7.35 (br, 1H), 7.33 (s, 1H), 7.21 - 7.18 (m, 1H), 7.14 - 7.09 (m, 3H), 6.86 (br, 1H), 6.64 (s, 1H), 4.20 (dd, *J =* 10.6, 2.7 Hz, 1H), 3.89 - 3.77 (m, 2H), 3.75 (s, 3H), 3.45 (s, 2H), 3.09 - 2.99 (m, 3H), 2.99 - 2.92 (m, 2H), 2.83 (dd, *J =* 40.6, 10.7 Hz, 2H), 2.71 - 2.63 (m, 1H), 2.22 (s, 3H), 2.12-2.05 (m, 1H), 1.69 (t, *J =* 10.6 Hz, 1H). MS *m*/*z* 557.3 [M+H]⁺.

### Example 18: Preparation of Compound B12R

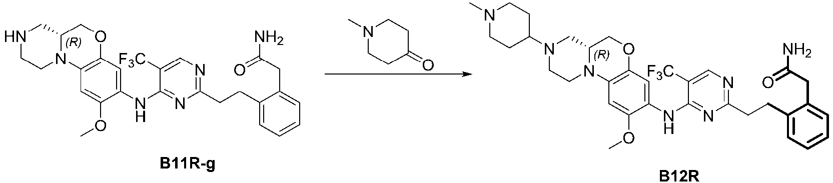

Compound **B11R-g** (50 mg, 0.09 mmol), N-methyl-4-piperidone (12 mg, 0.10 mmol) and zinc chloride (25 mg, 0.18 mmol) were sequentially dissolved in methanol (1 mL), followed by the addition of sodium cyanoborohydride (12 mg, 0.18 mmol). The reaction mixture was stirred at 75 °C for 1 hour. After the reaction was completed as monitored by TLC, the reaction mixture was concentrated under reduced pressure. The obtained crude product was purified by silica gel column chromatography (dichloromethane:methanol = 20:1, 2% ammonia water) to afford compound **B12R** as a yellow solid (22 mg, yield 37%). ¹H NMR (500 MHz, DMSO-*d*₆) *δ* 8.54 (s, 1H), 8.03 (s, 1H), 7.34 (br, 1H), 7.33 (s, 1H), 7.21-7.17 (m, 1H), 7.11 (dd, *J =* 5.6, 3.9 Hz, 3H), 6.86 (br, 1H), 6.62 (s, 1H), 4.21 (dd, *J =* 10.6, 2.6 Hz, 1H), 3.88 - 3.78 (m, 2H), 3.75 (s, 3H), 3.45 (s, 2H), 3.06-2.89 (m, 7H), 2.79 (d, *J =* 11.1 Hz, 2H), 2.65 - 2.59 (m, 1H), 2.33 - 2.26 (m, 1H), 2.19 - 2.15 (m, 1H), 2.14 (s, 3H), 1.91 - 1.79 (m, 3H), 1.73 (d, *J=* 11.4 Hz, 2H), 1.47 - 1.37 (m, 2H). MS *m*/*z* 640.3 [M+H]⁺.

### Example 19: Preparation of Compound C1S

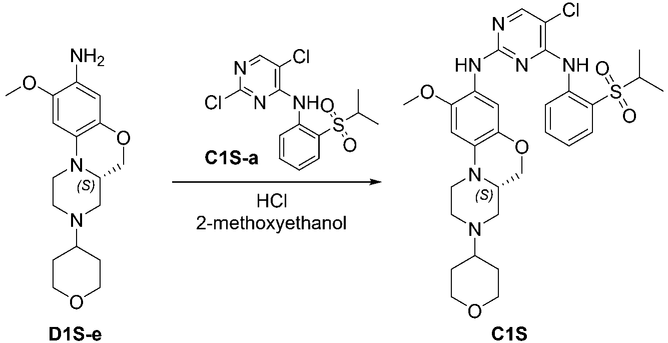

Compound **D1S-e** (50 mg, 0.16 mmol, for preparation, see Example 22) and compound **C1S-a** (54 mg, 0.16 mmol) were dissolved in 2-methoxyethanol (3 mL), followed by the addition of a 2.5 M solution of hydrochloric acid in methanol (0.16 mL). The reaction mixture was placed in a sealed tube, heated to 120 °C and stirred overnight. The completion of the reaction was monitored by TLC. After the reaction mixture was cooled to room temperature, an appropriate amount of ammonia water was added to adjust the pH to approximately 7. The mixture was then concentrated under reduced pressure. The obtained crude product was purified by silica gel column chromatography (dichloromethane:methanol = 20:1, 2% ammonia water) to afford compound C1S as a yellow solid (54 mg, yield 55%). ¹H NMR (500 MHz, MeOD-*d*₄) *δ* 8.45 (d, *J =* 8.1 Hz, 1H), 7.99 (s, 1H), 7.77 (dd, *J =* 8.0, 1.6 Hz, 1H), 7.63 - 7.54 (m, 1H), 7.26 - 7.18 (m, 2H), 6.46 (s, 1H), 4.11 (dd, *J =* 10.5, 2.5 Hz, 1H), 3.92 (dd, *J =* 11.2, 4.2 Hz, 2H), 3.8 - 3.80 (m, 1H), 3.71 - 3.69 (m, 4H), 3.38 - 3.29 (m, 3H), 3.10 - 3.02 (m, 1H), 3.01 - 2.91 (m, 2H), 2.71 - 2.60 (m, 1H), 2.48 - 2.38 (m, 1H), 2.38 - 2.30 (m, 1H), 1.92 (t, *J =* 10.5 Hz, 1H), 1.79 (d, *J =* 11.5 Hz, 2H), 1.54 - 1.43 (m, 2H), 1.16 (d, *J =* 11.2 Hz, 6H). MS *m*/*z* 629.6 [M+H]⁺.

### Example 20: Preparation of Compound C2S

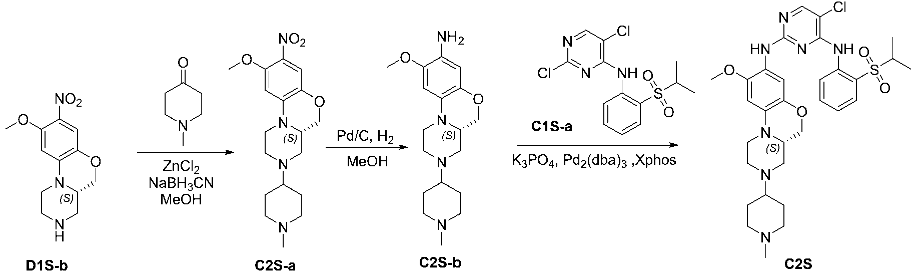

Compound **D1S-b** (150 mg, 0.57 mmol), N-methyl-4-piperidone (96 mg, 0.85 mmol) and zinc chloride (154 mg, 1.13 mmol) were sequentially dissolved in methanol (10 mL), followed by the addition of sodium cyanoborohydride (106 mg, 1.70 mmol). The reaction mixture was stirred at 75 °C for 1 hour. The completion of the reaction was monitored by TLC. The reaction mixture was concentrated under reduced pressure. The obtained crude product was purified by silica gel column chromatography (dichloromethane:methanol = 20:1, 2% ammonia water) to afford compound **C2S-a** as a yellow solid (150 mg, yield 73%). MS m/z 363.3 [M+H]⁺.

At room temperature, compound **C2S-a** (150 mg, 0.41 mmol) and palladium on carbon (10%, 15 mg) were added to methanol (10 mL). The reaction mixture was stirred at room temperature under a hydrogen atmosphere for 1 hour. After the reaction was completed as monitored by TLC, the reaction mixture was filtered through Celite, and the filtrate was concentrated under reduced pressure to obtain a crude product. The crude product was purified by silica gel column chromatography (dichloromethane:methanol = 20:1) to obtain compound **C2S-b** as a gray solid (137 mg, yield 100%). MS m/z 333.3 [M+H]⁺.

Compound **C2S-b** (15 mg, 0.045 mmol), compound **C1S-a** (17 mg, 0.05 mmol), tris(dibenzylideneacetone)dipalladium (4 mg, 0.004 mmol), 2-dicyclohexylphosphino-2',4',6'-triisopropylbiphenyl (4 mg, 0.009 mmol), and potassium phosphate (29 mg, 0.13 mmol) were dissolved in tert-butanol (1 mL). The reaction mixture was placed in a sealed tube, heated to 110 °C under a nitrogen atmosphere and stirred for 3 hours. The completion of the reaction was monitored by TLC. The reaction mixture was cooled to room temperature, filtered, and the filtrate was concentrated under reduced pressure. The obtained crude product was purified by silica gel column chromatography (dichloromethane:methanol = 20:1, 2% ammonia water) to afford compound **C2S** as a yellow solid (4 mg, yield 14%). ¹H NMR (500 MHz, CD₃OD) *δ* 8.54 (d, *J =* 8.4 Hz, 1H), 8.09 (s, 1H), 7.87 (dd, *J =* 8.0, 1.6 Hz, 1H), 7.70 - 7.63 (m, 1H), 7.33 (s, 1H), 7.31 - 7.29 (m, 1H), 6.55 (s, 1H), 4.20 (dd, *J =* 10.5, 2.6 Hz, 1H), 3.93 (dd, *J* = 10.5, 8.9 Hz, 1H), 3.81 - 3.78 (m, 4H), 3.21 - 3.14 (m, 2H), 3.13 - 3.03 (m, 2H), 3.01 (d, *J =* 10.9 Hz, 1H), 2.78 - 2.70 (m, 1H), 2.51 (s, 3H), 2.49 - 2.40 (m, 4H), 2.05 - 1.99 (m, 2H), 1.74 - 1.65 (m, 2H), 1.31 - 1.27 (m, 2H), 1.25 (d, *J* = 6.8 Hz, 6H). MS m/z 642.7 [M+H]⁺.

### Example 21: Preparation of Compound C3S

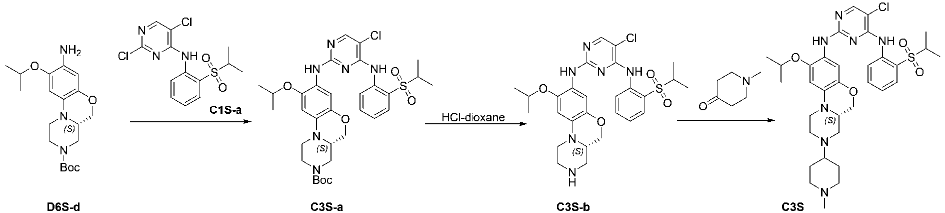

Compound **D6S-d** (100 mg, 0.28 mmol), compound C1S-a (114 mg, 0.33 mmol), tris(dibenzylideneacetone)dipalladium (13 mg, 0.01 mmol), 2-dicyclohexylphosphino-2',4',6'-triisopropylbiphenyl (13 mg, 0.03 mmol), and potassium phosphate (175 mg, 0.83 mmol) were dissolved in tert-butanol (2 mL). The reaction mixture was placed in a sealed tube, heated to 110 °C under a nitrogen atmosphere, and stirred for 3 hours. The completion of the reaction was monitored by TLC. After the reaction mixture was cooled to room temperature, it was filtered, and the filtrate was concentrated under reduced pressure. The obtained crude product was purified by silica gel column chromatography (dichloromethane:methanol = 20:1, 2% ammonia water) to afford compound **C3S-a** as a yellow solid (100 mg, yield 54%). MS m/z 673.2, 675.1 [M+H]⁺.

Compound **C3S-a** (50 mg, 0.07 mmol) was dissolved in methanol (1 mL), and then a 1,4-dioxane solution of hydrochloric acid (4.0 M, 1 mL) was added. The reaction solution was stirred at 40 °C for 2 hours. After the completion of the reaction was monitored by TLC, the mixture was cooled to room temperature and concentrated under reduced pressure. The resulting mixture was dissolved in a small amount of methanol, neutralized with ammonia water, and then concentrated under reduced pressure. The obtained crude product was purified by silica gel column chromatography (dichloromethane:methanol = 15:1, 2% ammonia water) to give compound **C3S-b** as a yellow solid (30 mg, yield 70%).

Compound **C3S-b** (30 mg, 0.05 mmol), N-methyl-4-piperidone (9 mg, 0.08 mmol), and zinc chloride (14 mg, 0.10 mmol) were sequentially dissolved in methanol (2 mL). After stirring for 30 minutes, sodium cyanoborohydride (10 mg, 0.16 mmol) was added. The reaction mixture was stirred at 75 °C for 1 hour. The completion of the reaction was monitored by TLC, and the mixture was concentrated under reduced pressure. The obtained crude product was purified by silica gel column chromatography (dichloromethane:methanol = 20:1, 2% ammonia water) to obtain compound **C3S** as a yellow solid (20 mg, yield 57%). ¹H NMR (500 MHz, MeOD-*d*₄) *δ* 8.53 (d, *J =* 8.2 Hz, 1H), 8.10 (s, 1H), 7.87 (dd, *J =* 8.0, 1.5 Hz, 1H), 7.71 - 7.65 (m, 1H), 7.37 (s, 1H), 7.34 - 7.29 (m, 1H), 6.55 (s, 1H), 4.48 - 4.40 (m, 1H), 4.20 (dd, *J =* 10.5, 2.6 Hz, 1H), 3.92 (dd, *J =* 10.4, 9.0 Hz, 1H), 3.74 - 3.67 (m, 1H), 3.29 - 3.23 (m, 1H), 3.12 - 3.03 (m, 2H), 3.02 - 2.92 (m, 3H), 2.72 - 2.67 (m, 1H), 2.48 - 2.40 (m, 1H), 2.36 - 2.30 (m, 1H), 2.29 (s, 3H), 2.12 - 2.00 (m, 3H), 1.96 - 1.89 (m, 2H), 1.65 - 1.55 (m, 2H), 1.27 (d, *J =* 6.1 Hz, 6H), 1.24 (d, *J =* 6.8 Hz, 6H). MS *m*/*z* 670.3, 672.2 [M+H]⁺.

### Example 22: Preparation of Compound D1S

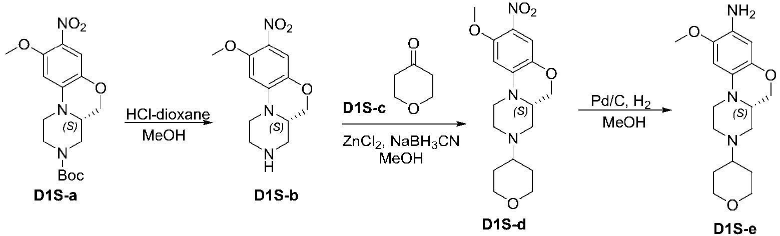

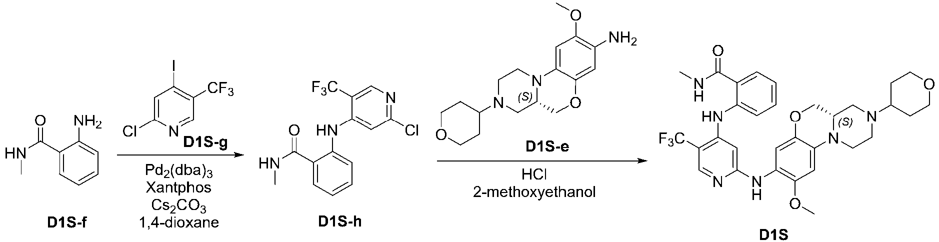

Compound **D1S-a** (800 mg, 2.19 mmol) was dissolved in methanol (5 mL), followed by the addition of a 1,4-dioxane solution of hydrochloric acid (4.0 M, 3 mL). The reaction mixture was stirred at 40 °C for 2 hours. The completion of the reaction was monitored by TLC. After the reaction mixture was cooled to room temperature, it was concentrated under reduced pressure. The resulting mixture was dissolved in a small amount of methanol, neutralized by adding ammonia water, and then concentrated under reduced pressure. The obtained crude product was purified by silica gel column chromatography (dichloromethane:methanol = 15:1, 2% ammonia water) to afford compound **D1S-b** as a yellow solid (580 mg, yield 100%).

Compound **D1S-b** (280 mg, 1.06 mmol), tetrahydropyranone D1S-c (126 mg, 1.27 mmol), and zinc chloride (288 mg, 2.11 mmol) were sequentially dissolved in methanol (10 mL), and then sodium cyanoborohydride (199 mg, 3.17 mmol) was added. The reaction mixture was stirred at 75 °C for 1 hour. The completion of the reaction was monitored by TLC. The reaction mixture was concentrated under reduced pressure. The obtained crude product was purified by silica gel column chromatography (dichloromethane:methanol = 20:1, 2% ammonia water) to give compound **D1S-d** as a yellow solid (300 mg, yield 81%). MS m/z 350.3 [M+H]⁻.

At room temperature, 10% palladium on carbon (Pd/C) catalyst (80 mg) was added to a solution of compound **D1S-d** (300 mg, 0.86 mmol) in methanol (10 mL). The reaction mixture was stirred at room temperature under a hydrogen atmosphere for 1 hour. The completion of the reaction was monitored by TLC. The reaction mixture was filtered through Celite, and the filtrate was concentrated under reduced pressure to obtain a crude product. The crude product was purified by silica gel column chromatography (dichloromethane:methanol = 20:1) to afford compound **D1S-e** as a gray solid (240 mg, yield 88%). MS m/z 320.3 [M+H]⁺.

Compound **D1S-f** (2.0 g, 13.32 mmol), **D1S-g** (4.5 g, 14.65 mmol), Pd₂(dba)₃ (1.22 g, 1.33 mmol), 4,5-bis(diphenylphosphino)-9,9-dimethylxanthene (1.54 g, 2.66 mmol), and cesium carbonate (8.68 g, 26.63 mmol) were dissolved in 1,4-dioxane (35 mL). The mixture was stirred at 90 °C for 2 hours under a nitrogen atmosphere. The completion of the reaction was monitored by TLC. After cooling to room temperature, the reaction mixture was filtered and washed. The filtrate was concentrated under reduced pressure to obtain a crude product. The crude product was purified by silica gel column chromatography (petroleum ether:ethyl acetate = 3:1) to give compound **D1S-h** as a pale yellow solid (4.13 g, yield 90%). ¹H NMR (500 MHz, CDCl₃) *δ* 10.13 (s, 1H), 8.41 (s, 1H), 7.56-7.45 (m, 3H), 7.30 (s, 1H), 7.15 (td, J = 7.6, 1.3 Hz, 1H), 6.20 (s, 1H). MS m/z 330.4 [M+H]⁺.

Compound **D1S-h** (34 mg, 0.10 mmol) and compound **D1S-e** (30 mg, 0.09 mmol) were dissolved in 2-methoxyethanol (1.5 mL), and then a methanol solution of hydrochloric acid (2.5 M, 0.10 mL) was added. The reaction mixture was placed in a sealed tube, heated to 120 °C, and stirred overnight. The completion of the reaction was monitored by TLC. After the reaction mixture was cooled to room temperature, an appropriate amount of ammonia water was added to adjust the pH to around 7. The mixture was then concentrated under reduced pressure. The obtained crude product was purified by silica gel column chromatography (dichloromethane:methanol = 20:1) to obtain compound **D1S** as an off-white solid (8 mg, yield 14%). ¹H NMR (500 MHz, DMSO-*d*₆) *δ* 10.16 (s, 1H), 8.67 - 8.61 (m, 1H), 8.30 (s, 1H), 8.16 (s, 1H), 7.68 (dd, *J =* 7.9, 1.2 Hz, 1H), 7.48 (t, *J =* 9.2 Hz, 1H), 7.45 (t, *J =* 7.1 Hz, 1H), 7.10 (s, 1H), 7.09 - 7.04 (m, 1H), 6.63 (s, 1H), 6.54 (s, 1H), 4.18 (dd, *J =* 10.5, 2.5 Hz, 1H), 3.90 (dd, *J =* 10.9, 3.6 Hz, 2H), 3.84 - 3.75 (m, 2H), 3.73 (s, 3H), 3.31 - 3.24 (m, 2H), 2.99 (d, *J =* 11.0 Hz, 1H), 2.96 - 2.90 (m, 2H), 2.75 (d, *J =* 4.5 Hz, 3H), 2.62 - 2.55 (m, 1H), 2.46 - 2.38 (m, 1H), 2.33 - 2.24 (m, 1H), 1.86 (t, *J =* 11.1 Hz, 1H), 1.74 - 1.71 (m, 2H) 1.47-1.38 (m, 2H). MS *m*/*z* 613.7 [M+H]⁺.

### Example 23: Preparation of Compound D1R

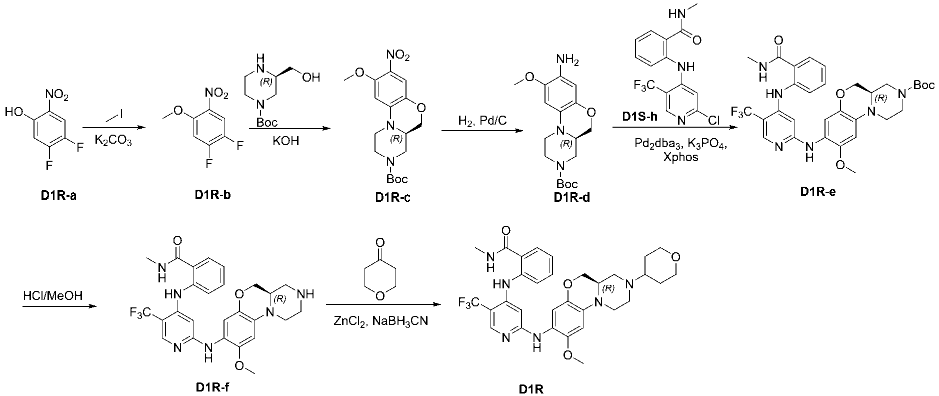

Compound **D1R-a** (900 mg, 5.14 mmol), methyl iodide (2.19 g, 15.42 mmol), and potassium carbonate (2.13 g, 15.42 mmol) were dissolved in N,N-dimethylformamide (10 mL). The reaction mixture was stirred at room temperature for 12 hours. The completion of the reaction was monitored by LCMS. The reaction solution was quenched with water, and the mixture was extracted with ethyl acetate (3×40 mL). The combined organic phases were washed with saturated brine, dried over anhydrous sodium sulfate, filtered, and concentrated under reduced pressure. The obtained crude product was purified by silica gel column chromatography (petroleum ether:ethyl acetate = 10:1) to afford compound D1R-b as a yellow solid (824 mg, yield 85%).

Compound **D1R-b** (250 mg, 1.32 mmol) and (R)-1-tert-butoxycarbonyl-3-hydroxymethylpiperazine (286 mg, 1.32 mmol) were dissolved in dimethyl sulfoxide (5 mL). Potassium hydroxide (223 mg, 3.97 mmol) was added, and the mixture was stirred at room temperature for 1 hour, then heated to 60 °C for 12 hours. After the reaction was completed, the mixture was extracted with ethyl acetate (3×40 mL). The combined organic phases were washed with saturated brine, dried over anhydrous sodium sulfate, filtered, and concentrated under reduced pressure. The obtained crude product was purified by silica gel column chromatography (dichloromethane:methanol = 50:1) to give compound **D1R-c** as a yellow solid (235 mg, yield 49%).

At room temperature, compound **D1R-c** (217 mg, 0.59 mmol) and 10% palladium on carbon (20 mg) were added to methanol (10 mL). The mixture was stirred at room temperature under a hydrogen atmosphere for 1 hour. The reaction mixture was filtered through Celite, and the filtrate was concentrated under reduced pressure to obtain compound **D1R-d** as a crude gray solid (199 mg), which was used directly in the next step.

Compound **D1R-d** (199 mg, 0.59 mmol), compound **D1S-h** (215 mg, 0.65 mmol), tris(dibenzylideneacetone)dipalladium (54 mg, 0.06 mmol), 2-dicyclohexylphosphino-2',4',6'-triisopropylbiphenyl (62 mg, 0.12 mmol), and potassium phosphate (377 mg, 1.77 mmol) were dissolved in tert-butanol (10 mL). The reaction mixture was heated to 110 °C under a nitrogen atmosphere and stirred for 3 hours. After the reaction was completed, the mixture was cooled to room temperature, filtered, and concentrated under reduced pressure. The obtained crude product was purified by silica gel column chromatography (dichloromethane:methanol = 20:1, 2% ammonia water) to afford compound **D1R-e** as a yellow solid (206 mg, yield 55%).

Compound **D1R-e** (199 mg, 0.59 mmol) was dissolved in methanol (5 mL), and then a methanol solution of hydrogen chloride (4 M, 2 mL) was added. The reaction solution was heated at 50 °C and stirred for 2 hours. The reaction mixture was concentrated under reduced pressure, and the obtained crude product was purified by silica gel column chromatography (dichloromethane:methanol = 15:1, 2% ammonia water) to give compound **D1R-f** as a yellow solid (87 mg, yield 92%).

Compound **D1R-f** (30 mg, 0.06 mmol), 4-oxacyclohexanone (9 mg, 0.09 mmol), and zinc chloride (23 mg, 0.17 mmol) were sequentially dissolved in methanol (2 mL), followed by the addition of sodium cyanoborohydride (11 mg, 0.17 mmol). The reaction mixture was heated at 65 °C and stirred for 1 hour. The reaction mixture was concentrated under reduced pressure, and the obtained crude product was purified by silica gel column chromatography (dichloromethane:methanol = 20:1, 2% ammonia water) to obtain compound **D1R** as a yellow solid (14 mg, yield 40%). ¹H NMR (500 MHz, DMSO-*d*₆) *δ* 10.17 (s, 1H), 8.66 - 8.63 (m, 1H),8.30 (s, 1H), 8.16 (s, 1H), 7.68 (dd, *J* = 7.9, 1.3 Hz, 1H), 7.48 (t, *J* = 9.2 Hz, 1H), 7.46 (t, *J =* 9.0 Hz, 1H), 7.12 (s, 1H), 7.10 - 7.06 (m, 1H), 6.63 (s, 1H), 6.54 (s, 1H), 4.18 (dd, *J =* 10.5, 2.5 Hz, 1H), 3.90 (dd, *J =* 10.6, 3.6 Hz, 2H), 3.85 - 3.75 (m, 2H), 3.73 (s, 3H), 3.31 - 3.25 (m, 2H), 3.02 - 2.90 (m, 3H), 2.76 (d, *J* = 4.5 Hz, 3H), 2.61 - 2.55 (m, 1H), 2.46 - 2.39 (m, 1H), 2.32 - 2.25 (m, 1H), 1.86 (t, *J =* 11.0 Hz, 1H), 1.76 - 1.69 (m, 2H), 1.47 - 1.36 (m, 2H). MS *m*/*z* 613.6 [M+H]⁺.

### Example 24: Preparation of Compound D2S

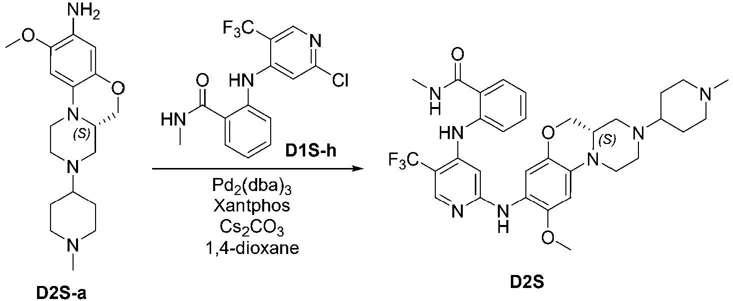

Compound **D2S-a** (40 mg, 0.12 mmol), **D1S-h** (44 mg, 0.13 mmol), Pd₂(dba)₃ (11 mg, 0.01 mmol), 4,5-bis(diphenylphosphino)-9,9-dimethylxanthene (14 mg, 0.02 mmol) and cesium carbonate (78 mg, 0.24 mmol) were dissolved in 1,4-dioxane (2.5 mL). The mixture was stirred at 95 °C for 2 hours under nitrogen protection. The completion of the reaction was monitored by TLC. After cooling to room temperature, the reaction mixture was filtered and washed. The filtrate was concentrated under reduced pressure to obtain a crude product. The crude product was purified by silica gel column chromatography (dichloromethane:methanol = 20:1, 2% ammonia water) to give compound **D2S** as an off-white solid (3 mg, yield 4%). MS m/z 626.7 [M+H]⁺.

### Example 25: Preparation of Compound D2R

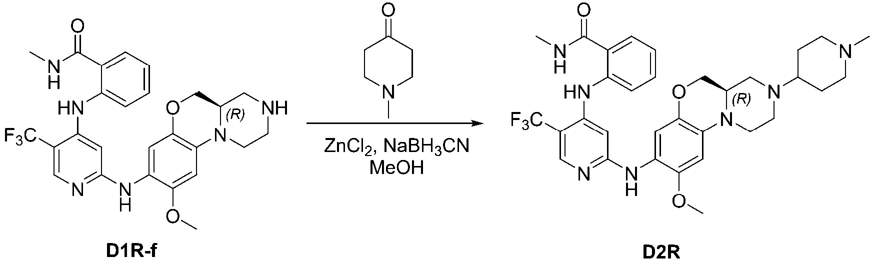

Compound **D1R-f** (30 mg, 0.06 mmol), N-methyl-4-piperidone (10 mg, 0.09 mmol), and zinc chloride (23 mg, 0.17 mmol) were sequentially dissolved in methanol (2 mL), followed by the addition of sodium cyanoborohydride (11 mg, 0.17 mmol). The reaction mixture was heated at 65 °C and stirred for 1 hour. After the reaction was completed, the mixture was concentrated under reduced pressure. The obtained crude product was purified by silica gel column chromatography (dichloromethane:methanol = 20:1, 2% ammonia water) to afford compound **D2R** as a yellow solid (18 mg, yield 51%). ¹H NMR (500 MHz, CD₃OD) *δ* 8.09 (s, 1H), 7.59 (dd, *J =* 7.8, 1.4 Hz, 1H), 7.49 (dd, *J =* 8.2, 0.8 Hz, 1H), 7.45-7.40 (m, 1H), 7.10 - 7.06 (m, 1H), 6.86 (s, 1H), 6.52 (s, 1H), 6.46 (s, 1H), 4.17 (dd, *J =* 10.5, 2.6 Hz, 1H), 3.91 - 3.86 (m, 1H), 3.79 - 3.73 (m, 4H), 3.11 - 2.94 (m, 5H), 2.86 (s, 3H), 2.76 - 2.69 (m, 1H), 2.46 - 2.39 (m, 1H), 2.37 - 2.29 (m, 4H), 2.19 - 2.10 (m, 2H), 2.01 (t, *J =* 10.8 Hz, 1H), 1.96 - 1.90 (m, 2H), 1.66 - 1.56 (m, 2H). MS *m*/*z* 626.7 [M+H]⁺.

### Example 26: Preparation of Compound D3S

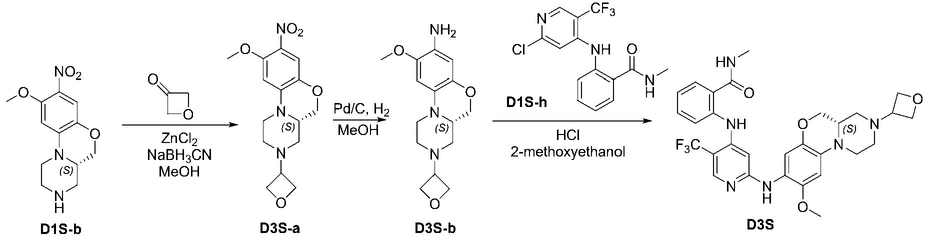

Compound **D1S-b** (60 mg, 0.23 mmol), 3-oxetanone (25 mg, 0.34 mmol), and zinc chloride (62 mg, 0.45 mmol) were sequentially dissolved in methanol (2 mL), followed by the addition of sodium cyanoborohydride (43 mg, 0.68 mmol). The reaction mixture was stirred at 75 °C for 1 hour. The completion of the reaction was monitored by TLC, and the reaction mixture was concentrated under reduced pressure. The obtained crude product was purified by silica gel column chromatography (dichloromethane:methanol = 20:1, 2% ammonia water) to afford compound **D3S-a** as a yellow solid (50 mg, yield 69%). MS m/z 322.3 [M+H]⁺.

At room temperature, compound **D3S-a** (50 mg, 0.16 mmol) and 10% palladium on carbon (5 mg) were added to methanol (10 mL). The reaction mixture was stirred at room temperature under a hydrogen atmosphere for 1 hour. The completion of the reaction was monitored by TLC. The reaction mixture was filtered through Celite, and the filtrate was concentrated under reduced pressure to obtain a crude product. The crude product was purified by silica gel column chromatography (dichloromethane:methanol = 20:1) to give compound **D3S-b** as a gray solid (36 mg, yield 79%). MS m/z 292.3 [M+H]⁺.

Compound **D3S-b** (36 mg, 0.12 mmol), compound **D1S-h** (41 mg, 0.12 mmol), tris(dibenzylideneacetone)dipalladium (11 mg, 0.012 mmol), 2-dicyclohexylphosphino-2',4',6'-triisopropylbiphenyl (11 mg, 0.024 mmol), and potassium phosphate (76 mg, 0.36 mmol) were dissolved in tert-butanol (1 mL). The reaction mixture was placed in a sealed tube, heated to 110 °C under a nitrogen atmosphere, and stirred for 3 hours. The completion of the reaction was monitored by TLC. After the reaction mixture was cooled to room temperature, it was filtered, and the filtrate was concentrated under reduced pressure. The obtained crude product was purified by silica gel column chromatography (dichloromethane:methanol = 20:1, 2% ammonia water) to obtain compound **D3S** as a yellow solid (20 mg, yield 28%). ¹H NMR (500 MHz, DMSO-*d*₆) *δ* 10.17 (s, 1H), 8.67 - 8.61 (m, 1H), 8.31 (s, 1H), 8.17 (s, 1H), 7.68 (dd, *J* = 7.9, 1.3 Hz, 1H), 7.51 - 7.47 (m, 1H), 7.47 - 7.42 (m, 1H), 7.12 (br., 1H), 7.10 - 7.04 (m, 1H), 6.64 (br., 1H), 6.55 (s, 1H), 4.61 - 4.53 (m, 2H), 4.51 - 4.44 (m, 2H), 4.17 (dd, *J =* 10.6, 2.6 Hz, 1H), 3.84 - 3.77 (m, 2H), 3.73 (s, 3H), 3.48 - 3.41 (m, 1H), 3.06 - 2.99 (m, 1H), 2.84 - 2.77 (m, 2H), 2.76 (d, *J =* 4.5 Hz, 3H), 2.69 - 2.61 (m, 1H), 2.06 - 1.98 (m, 1H), 1.62 (t, *J =* 10.6 Hz, 1H). MS *m*/*z* 585.6 [M+H]⁺.

### Example 27: Preparation of Compound D4S

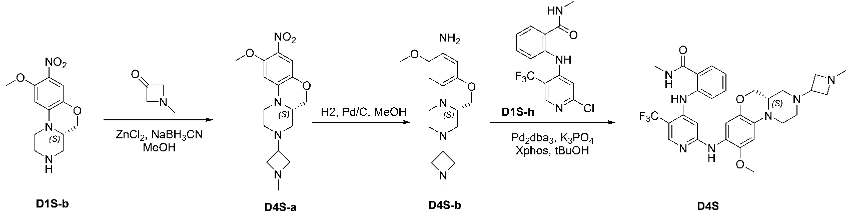

Compound **D1S-b** (50 mg, 0.19 mmol), 1-methyl-3-azetidinone hydrochloride (69 mg, 0.57 mmol), and zinc chloride (77 mg, 0.5 mmol) were sequentially dissolved in methanol (2 mL), followed by the addition of sodium cyanoborohydride (24 mg, 0.38 mmol). The reaction mixture was stirred at 65 °C for 1 hour. After the reaction was completed, the mixture was concentrated under reduced pressure. The obtained crude product was purified by silica gel column chromatography (dichloromethane:methanol = 20:1, 2% ammonia water) to afford compound **D4S-a** as a yellow solid (45 mg, yield 71%).

At room temperature, compound **D4S-a** (45 mg, 0.13 mmol) and 10% palladium on carbon (Pd/C) (5 mg) were added to methanol (5 mL). The reaction mixture was stirred at room temperature under a hydrogen atmosphere for 1 hour. The reaction mixture was filtered through Celite, and the filtrate was concentrated under reduced pressure to obtain compound **D4S-b** as a crude gray solid (39 mg, yield 95%), which was used directly in the next step.

Compound **D4S-b** (14 mg, 0.05 mmol), compound D1S-h (17 mg, 0.05 mmol), tris(dibenzylideneacetone)dipalladium (10 mg, 0.01 mmol), 2-dicyclohexylphosphino-2',4',6'-triisopropylbiphenyl (12 mg, 0.02 mmol), and potassium phosphate (29 mg, 0.15 mmol) were dissolved in tert-butanol (3 mL). The reaction mixture was heated to 110 °C under a nitrogen atmosphere and stirred for 3 hours. The reaction mixture was cooled to room temperature, filtered, and the filtrate was concentrated under reduced pressure. The obtained crude product was purified by silica gel column chromatography (dichloromethane:methanol = 20:1, 2% ammonia water) to obtain compound **D4S** as a yellow solid (12 mg, yield 44%). ¹H NMR (500 MHz, CD₃OD) *δ* 8.09 (s, 1H), 7.61 (dd, *J* = 7.8, 1.4 Hz, 1H), 7.50 (d, *J* = 8.3 Hz, 1H), 7.45 - 7.41 (m, 1H), 7.11 - 7.06 (m, 1H), 6.91 (d, *J =* 2.6 Hz, 1H), 6.54 (s, 1H), 6.49 (s, 1H), 4.20 - 4.12 (m, 3H), 3.99 - 3.87 (m, 3H), 3.84 - 3.78 (m, 1H), 3.76 (s, 3H), 3.29 - 3.25 (m, 1H), 3.13 - 3.06 (m, 1H), 2.97 - 2.92 (m, 1H), 2.88 (s, 3H), 2.87 (s, 3H), 2.86 - 2.83 (m, 1H), 2.80 - 2.73 (m, 1H), 2.19 - 2.13 (m, 1H), 1.77 (t, *J =* 10.6 Hz, 1H). MS m/z 598.6 [M+H]⁺.

### Example 28: Preparation of Compound D5S

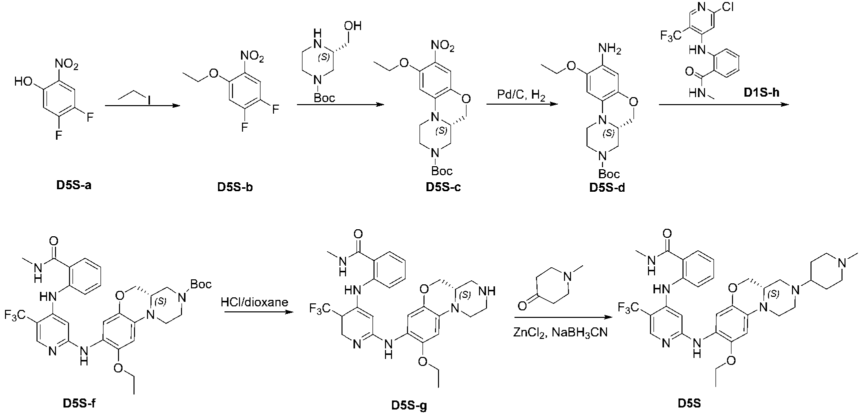

Compound **D5S-a** (500 mg, 2.86 mmol) and potassium carbonate (1.18 g, 8.57 mmol) were dissolved in N,N-dimethylformamide (10 mL), and then ethyl iodide (890 mg, 5.71 mmol) was slowly added. The reaction mixture was stirred at room temperature for 12 hours. After the reaction was completed, it was quenched with water, and the mixture was extracted with ethyl acetate (3 × 20 mL). The combined organic phases were washed with saturated brine, dried over anhydrous sodium sulfate, filtered, and then concentrated under reduced pressure. The obtained crude product was purified by silica gel column chromatography (petroleum ether:ethyl acetate = 15:1) to afford compound **D5S-b** as a yellow solid (530 mg, yield 91%).

Compound **D5S-b** (200 mg, 0.98 mmol) and (S)-1-tert-butoxycarbonyl-3-hydroxymethylpiperazine (213 mg, 0.98 mmol) were dissolved in dimethyl sulfoxide (10 mL), and then potassium hydroxide (165 mg, 2.95 mmol) was added. The mixture was stirred at room temperature for 1 hour and then heated to 60 °C for 12 hours. After the reaction was completed, the mixture was extracted with ethyl acetate (3 × 20 mL). The combined organic phases were washed with saturated brine, dried over anhydrous sodium sulfate, filtered, and then concentrated under reduced pressure. The obtained crude product was purified by silica gel column chromatography (dichloromethane:methanol = 50:1) to give compound **D5S-c** as a yellow solid (160 mg, yield 43%).

At room temperature, compound **D5S-c** (160 mg, 0.42 mmol) and 10% palladium on carbon (20 mg) were added to a methanol solution (10 mL). The reaction mixture was stirred at room temperature under a hydrogen atmosphere for 1 hour. After the reaction was completed, the mixture was filtered through Celite, and the filtrate was concentrated under reduced pressure to obtain a crude product. The crude product was purified by silica gel column chromatography (dichloromethane:methanol = 20:1) to afford compound **D5S-d** as a gray solid (50 mg, yield 34%). MS m/z 350.2 [M+H]⁺.

Compound **D5S-d** (50 mg, 0.14 mmol), compound **D5S-e** (56 mg, 0.17 mmol), tris(dibenzylideneacetone)dipalladium (7 mg, 0.007 mmol), 2-dicyclohexylphosphino-2',4',6'-triisopropylbiphenyl (7 mg, 0.014 mmol), and potassium phosphate (91 mg, 0.43 mmol) were dissolved in tert-butanol (1 mL). The reaction mixture was placed in a sealed tube, heated to 110 °C under a nitrogen atmosphere, and stirred for 3 hours. The completion of the reaction was monitored by TLC. After the reaction system was cooled to room temperature, it was filtered, and the filtrate was concentrated under reduced pressure. The obtained crude product was purified by silica gel column chromatography (dichloromethane:methanol = 20:1, 2% ammonia water) to afford compound **D5S-f** as a yellow solid (60 mg, yield 65%). MS m/z 643.2 [M+H]⁺.

Compound **D5S-f** (50 mg, 0.08 mmol) was dissolved in methanol (1 mL), and then a 1,4-dioxane solution of hydrochloric acid (4.0 M, 1 mL) was added. The reaction mixture was stirred at 40 °C for 2 hours. The completion of the reaction was monitored by TLC. After the reaction was cooled to room temperature, it was concentrated under reduced pressure. The obtained crude product was dissolved in a small amount of methanol, neutralized with ammonia water, and then concentrated under reduced pressure. The obtained crude product was purified by silica gel column chromatography (dichloromethane:methanol = 15:1, 2% ammonia water) to give compound **D5S-g** as a yellow solid (42 mg, yield 100%).

Compound **D5S-g** (42 mg, 0.08 mmol) was dissolved in methanol (2 mL). N-methyl-4-piperidone (13 mg, 0.12 mmol) and zinc chloride (21 mg, 0.15 mmol) were sequentially added to the system. After stirring for 30 minutes, sodium cyanoborohydride (15 mg, 0.23 mmol) was added. The reaction mixture was stirred at 75 °C for 1 hour. The completion of the reaction was monitored by TLC. The reaction mixture was concentrated under reduced pressure. The obtained crude product was purified by silica gel column chromatography (dichloromethane:methanol = 20:1, 2% ammonia water) to obtain compound **D5S** as a yellow solid (32 mg, yield 65%). ¹H NMR (500 MHz, MeOD-*d*₄) *δ* 8.09 (s, 1H), 7.60 (dd, *J* = 7.9, 1.4 Hz, 1H), 7.50 (dd, *J =* 8.2, 0.7 Hz, 1H), 7.44 - 7.39 (m, 1H), 7.11 - 7.05 (m, 1H), 6.80 (s, 1H), 6.49 (d, *J =* 3.0 Hz, 2H), 4.14 (dd, *J =* 10.5, 2.6 Hz, 1H), 4.00 - 3.93 (m, 2H), 3.90 - 3.83 (m, 1H), 3.73 - 3.65 (m, 1H), 3.08 - 2.99 (m, 2H), 2.98 - 2.89 (m, 3H), 2.86 (s, 3H), 2.73 - 2.63 (m, 1H), 2.42 - 2.34 (m, 1H), 2.32 - 2.23 (m, 4H), 2.04 (t, *J* = 11.4 Hz, 2H), 1.96 (t, *J =* 10.8 Hz, 1H), 1.92 - 1.84 (m, 2H), 1.63 - 1.51 (m, 2H), 1.28 (t, *J =* 7.0 Hz, 3H). MS *m*/*z* 640.1 [M+H]⁺.

### Example 29: Preparation of Compound D6S

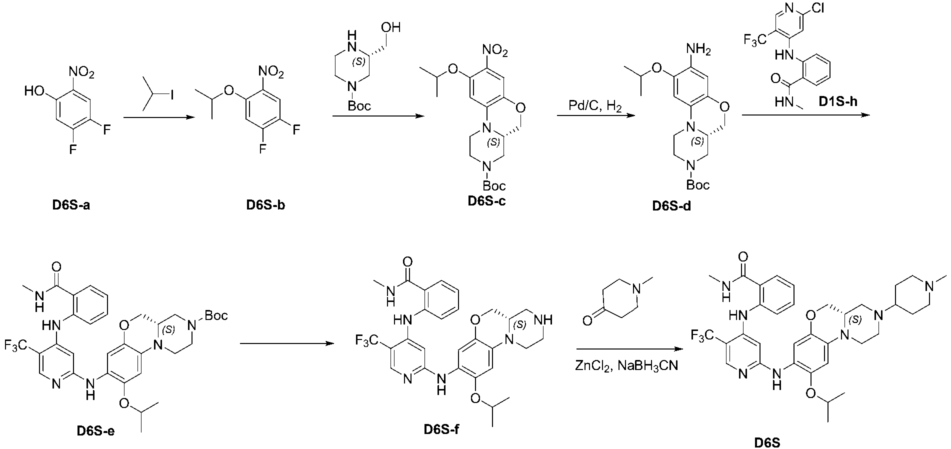

Compound **D6S-a** (500 mg, 2.86 mmol), 2-iodopropane (970 mg, 5.71 mmol), and potassium carbonate (1.18 g, 8.57 mmol) were dissolved in N,N-dimethylformamide (10 mL). The reaction system was stirred at room temperature for 12 hours. After the reaction was completed, it was quenched with water, and the mixture was extracted with ethyl acetate (3 × 20 mL). The combined organic phases were washed with saturated brine, dried over anhydrous sodium sulfate, filtered, and then concentrated under reduced pressure. The obtained crude product was purified by silica gel column chromatography (petroleum ether:ethyl acetate = 15:1) to afford compound **D6S-b** as a yellow solid (300 mg, yield 48%).

Compound **D6S-b** (150 mg, 0.69 mmol) and (S)-1-tert-butoxycarbonyl-3-hydroxymethylpiperazine (149 mg, 0.69 mmol) were dissolved in dimethyl sulfoxide (5 mL), and then potassium hydroxide (116 mg, 2.07 mmol) was added. The mixture was stirred at room temperature for 1 hour and then heated to 60 °C for 12 hours. After the reaction was completed, the mixture was extracted with ethyl acetate (3 × 10 mL). The combined organic phases were washed with saturated brine, dried over anhydrous sodium sulfate, filtered, and then concentrated under reduced pressure. The obtained crude product was purified by silica gel column chromatography (dichloromethane:methanol = 50:1) to give compound **D6S-c** as a yellow solid (170 mg, yield 62%).

At room temperature, compound **D6S-c** (170 mg, 0.43 mmol) and 10% palladium on carbon (10 mg) were added to a methanol solution (5 mL). The reaction system was stirred at room temperature under a hydrogen atmosphere for 1 hour. After the reaction was completed, the mixture was filtered through Celite, and the filtrate was concentrated under reduced pressure to obtain a crude product. The crude product was purified by silica gel column chromatography (dichloromethane:methanol = 20:1) to afford compound **D6S-d** as a gray solid (140 mg, yield 89%). MS m/z 364.5 [M+H]⁺.

Compound **D6S-d** (160 mg, 0.44 mmol), compound **D1S-h** (159 mg, 0.48 mmol), tris(dibenzylideneacetone)dipalladium (20 mg, 0.02 mmol), 2-dicyclohexylphosphino-2',4',6'-triisopropylbiphenyl (21 mg, 0.04 mmol), and potassium phosphate (280 mg, 1.32 mmol) were dissolved in tert-butanol (5 mL). The reaction mixture was placed in a sealed tube, heated to 110 °C under a nitrogen atmosphere, and stirred for 3 hours. After the completion of the reaction was monitored by TLC, the mixture was cooled to room temperature, filtered, and the filtrate was concentrated under reduced pressure. The obtained crude product was purified by silica gel column chromatography (dichloromethane:methanol = 20:1, 2% ammonia water) to afford compound **D6S-e** as a yellow solid (200 mg, yield 69%). MS m/z 657.1 [M+H]⁺.

Compound **D6S-e** (200 mg, 0.30 mmol) was dissolved in methanol (1 mL), and then a 1,4-dioxane solution of hydrochloric acid (4.0 M, 1 mL) was added. The reaction mixture was stirred at 40 °C for 2 hours. The completion of the reaction was monitored by TLC. After the mixture was cooled to room temperature, it was concentrated under reduced pressure. The obtained crude product was dissolved in a small amount of methanol, neutralized with ammonia water, and then concentrated under reduced pressure. The resulting residue was purified by silica gel column chromatography (dichloromethane:methanol = 15:1, 2% ammonia water) to give compound **D6S-f** as a yellow solid (170 mg, yield 100%).

Compound **D6S-f** (50 mg, 0.09 mmol) was dissolved in a methanol solution (2 mL). N-methyl-4-piperidone (15 mg, 0.13 mmol) and zinc chloride (25 mg, 0.18 mmol) were sequentially added. After stirring for 30 minutes, sodium cyanoborohydride (17 mg, 0.27 mmol) was added. The reaction mixture was stirred at 75 °C for 1 hour. The completion of the reaction was monitored by TLC. The mixture was concentrated under reduced pressure, and the obtained crude product was purified by silica gel column chromatography (dichloromethane:methanol = 20:1, 2% ammonia water) to obtain compound **D6S** as a yellow solid (23 mg, yield 39%). ¹H NMR (500 MHz, MeOD-*d*₄) *δ* 8.11 - 8.07 (m, 1H), 7.59 (dd, *J* = 7.9, 1.4 Hz, 1H), 7.55 - 7.51 (m, 1H), 7.44 - 7.38 (m, 1H), 7.10 - 7.05 (m, 1H), 6.78 (s, 1H), 6.53 (s, 1H), 6.49 (s, 1H), 4.38 - 4.30 (m, 1H), 4.18 (dd, *J =* 10.5, 2.6 Hz, 1H), 3.93 - 3.86 (m, 1H), 3.73 - 3.67 (m, 1H), 3.08 - 3.00 (m, 4H), 2.99 - 2.94 (m, 1H), 2.86 (s, 3H), 2.73 - 2.65 (m, 1H), 2.44 - 2.39 (m, 1H), 2.38 - 2.31 (m, 4H), 2.25 - 2.15 (m, 2H), 2.00 (t, *J* = 10.7 Hz, 1H), 1.96 - 1.89 (m, 2H), 1.67 - 1.57 (m, 2H), 1.19 (dd, *J =* 6.1, 2.1 Hz, 6H). MS *m*/*z* 654.2 [M+H]⁺.

### Example 30: Preparation of Compound E1R

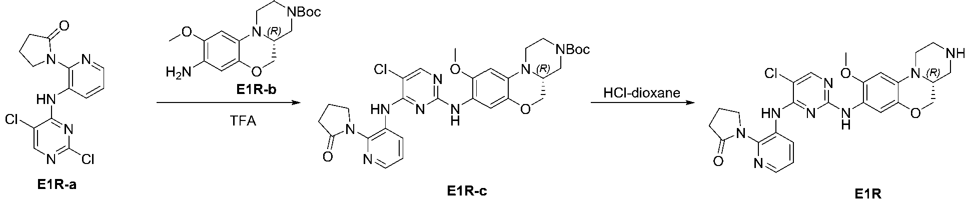

Compound **E1R-a** (200 mg, 0.62 mmol) (the synthetic route of intermediate **E1R-a** refers to the method in patent WO 2021/003417 A1 for preparation) and compound **E1R-b** (207 mg, 0.62 mmol) were dissolved in tert-butanol (5 mL), and then trifluoroacetic acid (126 mg, 1.23 mmol) was added. The reaction system was stirred at 90 °C for 14 hours. After the reaction was completed, the reaction solution was quenched by adding water, and the mixture was extracted with ethyl acetate (3 × 20 mL). The combined organic phases were washed with saturated brine, dried over anhydrous sodium sulfate, filtered and concentrated under reduced pressure. The obtained crude product was separated and purified by silica gel column chromatography (petroleum ether: ethyl acetate = 4:1) to obtain compound **E1R-c** (250 mg, yield 65%).

Compound **E1R-c** (250 mg, 0.40 mmol) was dissolved in dichloromethane (10 mL), and then a dioxane solution of hydrochloric acid (4.0 M, 2 mL) was added. The mixture was stirred at room temperature for 2 hours. TLC monitoring showed that the reaction was completed. The reaction system was cooled to room temperature and then concentrated under reduced pressure. The obtained mixture was dissolved in a small amount of methanol, ammonia water was added for neutralization, and then concentrated under reduced pressure. The obtained crude product was separated and purified by silica gel column chromatography (dichloromethane: methanol = 20:1, with 2% ammonia water) to obtain yellow solid compound **E1R** (160 mg, yield 76%). ¹H NMR (500 MHz, DMSO-*d*₆) *δ* 8.74 (s, 1H), 8.28 - 8.26 (m, 1H), 8.26 (s, 1H), 8.09 (s, 1H), 7.75 (s, 1H), 7.32 (dd, *J =* 7.2, 5.5 Hz, 1H), 7.05 (br., 1H), 6.50 (s, 1H), 4.15 (dd, *J* = 10.5, 2.4 Hz, 1H), 4.02 (t, *J =* 7.0 Hz, 2H), 3.82 - 3.76 (m, 1H), 3.71 (s, 3H), 3.64 (d, *J =* 11.2 Hz, 1H), 2.98 (d, *J =* 12.1 Hz, 1H), 2.91 - 2.81 (m, 2H), 2.71 (d, *J =* 3.1 Hz, 1H), 2.60 (t, *J* = 8.0 Hz, 2H), 2.49 - 2.45 (m, 1H), 2.43 - 2.33 (m, 1H), 2.28 (t, *J =* 10.9 Hz, 1H), 2.16 - 2.08 (m, 2H). MS *m*/*z* 523.2, 525.1 [M+H]⁺.

### Example 31: Preparation of Compound E2R

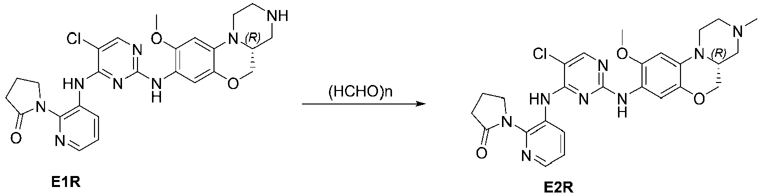

Compound **E1R** (35 mg, 0.07 mmol), paraformaldehyde (3 mg, 0.10 mmol) and zinc chloride (19 mg, 0.14 mmol) were sequentially dissolved in methanol (1 mL), followed by the addition of sodium cyanoborohydride (9 mg, 0.14 mmol). The reaction mixture was stirred at 75 °C for 1 hour. TLC monitoring indicated the completion of the reaction, and the reaction solution was concentrated under reduced pressure. The obtained crude product was separated and purified by silica gel column chromatography (dichloromethane:methanol = 20:1, 2% ammonia water) to afford compound **E2R** as a yellow solid (24 mg, yield 66%). ¹H NMR (500 MHz, DMSO-*d*₆) *δ* 8.75 (s, 1H), 8.26 (d, *J =* 6.1 Hz, 2H), 8.09 (s, 1H), 7.75 (s, 1H), 7.36 - 7.28 (m, 1H), 7.07 (s, 1H), 6.53 (s, 1H), 4.19 (d, *J=* 10.4 Hz, 1H), 4.02 (t, *J =* 6.9 Hz, 2H), 3.86 - 3.80 (m, 1H), 3.75 (d, *J =* 11.6 Hz, 1H), 3.71 (s, 3H), 2.99 (t, *J =* 9.0 Hz, 1H), 2.82 (dd, *J =* 33.5, 10.4 Hz, 2H), 2.68 - 2.57 (m, 3H), 2.22 (s, 3H), 2.16 - 2.03 (m, 3H), 1.69 (t, *J* = 10.5 Hz, 1H). MS *m*/*z* 537.3, 539.2 [M+H]⁺,

### Example 32: Preparation of Compound E3R

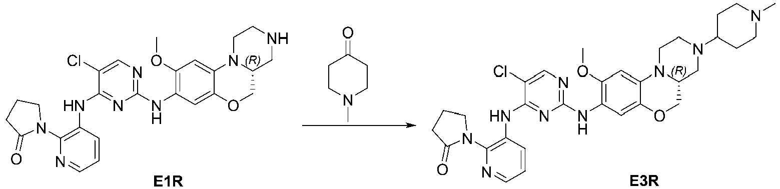

Compound **E1R** (35 mg, 0.07 mmol), N-methyl-4-piperidone (12 mg, 0.10 mmol) and zinc chloride (19 mg, 0.14 mmol) were sequentially dissolved in methanol (1 mL), followed by the addition of sodium cyanoborohydride (9 mg, 0.14 mmol). The reaction solution was stirred at 75 °C for 1 hour. TLC monitoring indicated the completion of the reaction, and the reaction system was concentrated under reduced pressure. The obtained crude product was separated and purified by silica gel column chromatography (dichloromethane:methanol = 20:1, 2% ammonia water) to afford compound **E3R** as a yellow solid (25 mg, yield 61%). ¹H NMR (500 MHz, DMSO-*d*₆) *δ* 8.75 (s, 1H), 8.28 - 8.26 (m, 1H), 8.26 (s, 1H), 8.09 (s, 1H), 7.75 (s, 1H), 7.32 (dd, *J =* 7.4, 5.2 Hz, 1H), 7.06 (s, 1H), 6.52 (s, 1H), 4.20 (dd, *J =* 10.6, 2.4 Hz, 1H), 4.02 (t, *J =* 7.0 Hz, 2H), 3.83 (dd, *J =* 10.5, 8.9 Hz, 1H), 3.76 (d, *J =* 11.6 Hz, 1H), 3.71 (s, 3H), 2.99 - 2.89 (m, 3H), 2.80 (d, *J =* 11.1 Hz, 2H), 2.63 - 2.53 (m, 3H), 2.30 (dd, *J=* 11.0, 8.0 Hz, 1H), 2.20 - 2.08 (m, 6H), 1.91 - 1.81 (m, 3H), 1.74 (d, *J =* 10.4 Hz, 2H), 1.48 - 1.37 (m, 2H). MS *m*/*z* 620.3, 622.2 [M+H]⁺.

### Example 33: Preparation of Compound E4R

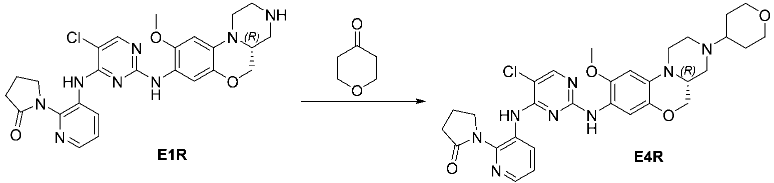

Compound **E1R** (35 mg, 0.07 mmol), tetrahydropyranone (10 mg, 0.10 mmol) and zinc chloride (19 mg, 0.14 mmol) were sequentially dissolved in methanol (1 mL), followed by the addition of sodium cyanoborohydride (9 mg, 0.14 mmol). The reaction mixture was stirred at 75 °C for 1 hour. TLC monitoring indicated the completion of the reaction, and the reaction solution was concentrated under reduced pressure. The obtained crude product was separated and purified by silica gel column chromatography (dichloromethane:methanol = 20:1, 2% ammonia water) to afford compound **E4R** as a yellow solid (23 mg, yield 57%). ¹H NMR (500 MHz, DMSO-*d*₆) *δ* 8.75 (s, 1H), 8.27 (br., 1H), 8.26 (br., 1H), 8.09 (s, 1H), 7.75 (s, 1H), 7.36 - 7.28 (m, 1H), 7.06 (s, 1H), 6.53 (s, 1H), 4.23 - 4.18 (m, 1H), 4.02 (t, *J =* 6.9 Hz, 2H), 3.93 - 3.87 (m, 2H), 3.87 - 3.81 (m, 1H), 3.80 - 3.75 (m, 1H), 3.71 (s, 3H), 3.29 - 3.25 (m, 2H), 3.01 - 2.90 (m, 2H), 2.63 - 2.55 (m, 3H), 2.47 - 2.35 (m, 2H), 2.33 - 2.26 (m, 1H), 2.15 - 2.08 (m, 2H), 1.88 (t, *J =* 11.1 Hz, 1H), 1.76 - 1.70 (m, 2H), 1.48 - 1.38 (m, 2H). MS *m*/*z* 607.3, 609.2 [M+H]⁺.

### Example 34: Preparation of Compound E5R

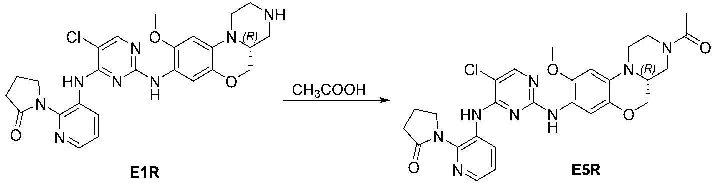

Compound **E1R** (20 mg, 0.04 mmol) was dissolved in acetonitrile (1 mL), followed by the sequential addition of acetic acid (5 mg, 0.08 mmol), N,N-diisopropylethylamine (15 mg, 0.12 mmol), and O-(7-azabenzotriazol-1-yl)-N,N,N',N'-tetramethyluronium hexafluorophosphate (30 mg, 0.08 mmol). The mixture waSR^{e}acted at room temperature for 1 hour. After the reaction was completed, the reaction solution was evaporated to dryness under reduced pressure to obtain a crude product, which was separated and purified by silica gel column chromatography (dichloromethane:methanol = 20:1) to afford compound **E5R** as a yellow solid (16 mg, yield 73%). ¹H NMR (500 MHz, DMSO-*d*₆) *δ* 8.78 - 8.72 (m, 1H), 8.27 (s, 1H), 8.26 (t, *J =* 2.1 Hz, 1H), 8.10 (s, 1H), 7.76 (br., 1H), 7.33 (dd, *J =* 7.3, 4.6 Hz, 1H), 7.11 (d, *J =* 5.0 Hz, 1H), 6.59 (s, 1H), 4.43 (t, *J =* 14.7 Hz, 1H), 4.28 (dd, *J =* 10.7, 2.6 Hz, 1H), 4.02 (t, *J =* 7.0 Hz, 2H), 3.94 - 3.82 (m, 3H), 3.72 (s, 3H), 3.29 - 3.19 (m, 1H), 2.90 - 2.82 (m, 1H), 2.77 - 2.63 (m, 1H), 2.60 (t, *J =* 8.0 Hz, 2H), 2.47 - 2.33 (m, 1H), 2.17 - 2.08 (m, 2H), 2.07 (d, *J =* 1.8 Hz, 3H). MS *m*/*z* 565.2, 567.1 [M+H]⁺.

### Example 35: Preparation of Compound E6R

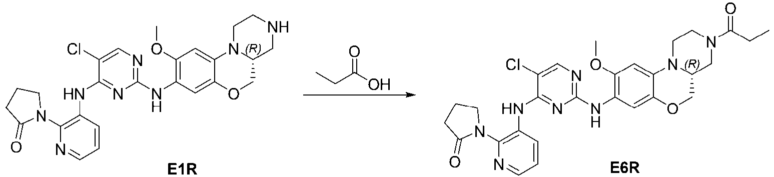

Compound **E1R** (20 mg, 0.04 mmol) was dissolved in acetonitrile (1 mL), followed by the sequential addition of propionic acid (4 mg, 0.06 mmol), N,N-diisopropylethylamine (15 mg, 0.12 mmol), and O-(7-azabenzotriazol-1-yl)-N,N,N',N'-tetramethyluronium hexafluorophosphate (30 mg, 0.08 mmol). The mixture waSR^{e}acted at room temperature for 1 hour. After the reaction was completed, the reaction solution was evaporated to dryness under reduced pressure to obtain a crude product, which was separated and purified by silica gel column chromatography (dichloromethane:methanol = 20:1) to afford compound **E6R** as a solid (10 mg, yield 45%). ¹H NMR (500 MHz, CD₃OD) *δ* 8.32 - 8.29 (m, 1H), 8.27 - 8.23 (m, 1H), 8.00 (s, 1H), 7.40 (dd, *J =* 8.1, 4.7 Hz, 1H), 7.28 (s, 1H), 6.54 (d, *J =* 5.5 Hz, 1H), 4.62 - 4.51 (m, 1H), 4.29 - 4.21 (m, 1H), 4.09 (t, *J* = 7.0 Hz, 2H), 4.06 - 3.96 (m, 1H), 3.95 - 3.90 (m, 1H), 3.83 (br., 1H), 3.80 (s, 3H), 3.04 - 2.82 (m, 3H), 2.66 (t, *J =* 8.0 Hz, 2H), 2.55 - 2.42 (m, 3H), 2.24 - 2.17 (m, 2H), 1.14 (t, *J* = 7.5 Hz, 3H). MS *m*/*z* 579.3, 581.1 [M+H]⁺.

### Example 36: Preparation of Compound E7R

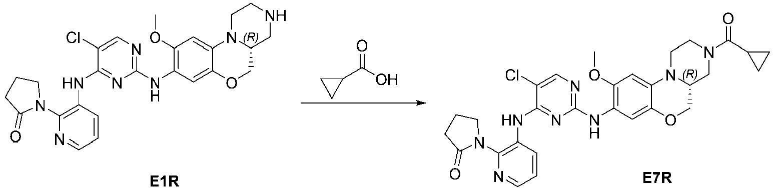

Compound **E1R** (20 mg, 0.04 mmol) was dissolved in acetonitrile (1 mL), followed by the sequential addition of cyclopropanecarboxylic acid (7 mg, 0.08 mmol), N,N-diisopropylethylamine (15 mg, 0.12 mmol), and O-(7-azabenzotriazol-1-yl)-N,N,N',N'-tetramethyluronium hexafluorophosphate (30 mg, 0.08 mmol). The mixture waSR^{e}acted at room temperature for 1 hour. After the reaction was completed, the reaction solution was evaporated to dryness under reduced pressure to obtain a crude product, which was separated and purified by silica gel column chromatography (dichloromethane:methanol = 20:1) to afford compound **E7R** as a solid (10.36 mg, yield 46%). ¹H NMR (500 MHz, CD₃OD) *δ* 8.30 (dd, *J* = 4.7, 1.6 Hz, 1H), 8.25 (d, *J =* 8.0 Hz, 1H), 7.99 (s, 1H), 7.40 (dd, *J =* 8.1, 4.7 Hz, 1H), 7.28 (s, 1H), 6.54 (s, 1H), 4.63 - 4.19 (m, 4H), 4.09 (t, *J =* 7.0 Hz, 2H), 3.98 - 3.90 (m, 1H), 3.84 (dd, *J =* 11.6, 3.1 Hz, 1H), 3.80 (s, 3H), 3.11 - 3.00 (m, 1H), 2.97 - 2.87 (m, 1H), 2.66 (t, *J =* 8.0 Hz, 2H), 2.60 - 2.51 (m, 1H), 2.25 - 2.16 (m, 2H), 2.03 (br., 1H), 0.96 - 0.89 (m, 2H), 0.88 - 0.80 (m, 2H). MS *m*/*z* 591.2, 593.2 [M+H]⁺.

### Example 37: Preparation of Compound E8R

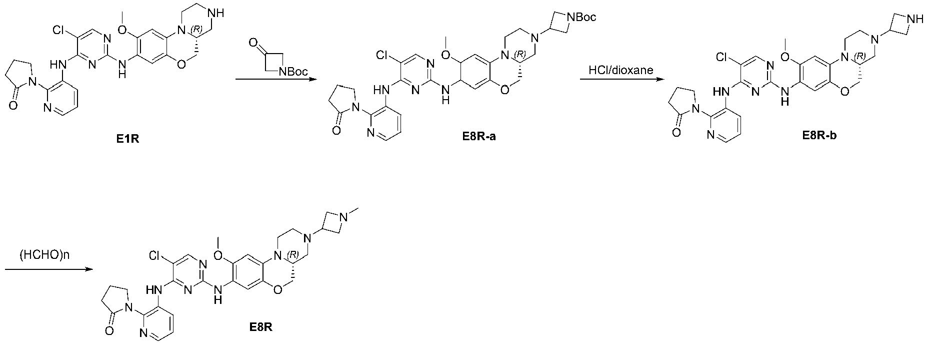

Compound **E1R** (50 mg, 0.09 mmol), 1-Boc-3-azetidinone (25 mg, 0.14 mmol) and zinc chloride (25 mg, 0.18 mmol) were dissolved in methanol (2 mL), followed by the addition of sodium cyanoborohydride (11 mg, 0.18 mmol). The reaction mixture was stirred at 75 °C for 1 hour. TLC monitoring indicated the completion of the reaction, and the reaction mixture was concentrated under reduced pressure. The obtained crude product was separated and purified by silica gel column chromatography (dichloromethane:methanol = 20:1, 2% ammonia water) to afford compound **E8R-a** as a yellow solid (48 mg, yield 74%).

Compound **E8R-a** (48 mg, 0.07 mmol) was dissolved in dichloromethane (2 mL), and then a dioxane solution of hydrochloric acid (4.0 M, 1 mL) was added. The reaction mixture was stirred at room temperature for 2 hours. TLC monitoring showed the reaction was completed, and the reaction solution was concentrated under reduced pressure. The obtained crude product was dissolved in a small amount of methanol, ammonia water was added for neutralization, and then concentrated under reduced pressure. The resulting crude product was separated and purified by silica gel column chromatography (dichloromethane:methanol = 20:1, 2% ammonia water) to obtain compound **E8R-b** as a yellow solid (40 mg, yield 98%).

Compound **E8R-b** (40 mg, 0.07 mmol), paraformaldehyde (3 mg, 0.10 mmol) and zinc chloride (19 mg, 0.14 mmol) were dissolved in methanol (2 mL), followed by the addition of sodium cyanoborohydride (9 mg, 0.14 mmol). The reaction mixture was stirred at 75 °C for 1 hour. TLC monitoring indicated the completion of the reaction. The reaction mixture was filtered and concentrated under reduced pressure. The obtained crude product was separated and purified by silica gel column chromatography (dichloromethane:methanol = 20:1, 2% ammonia water) to afford compound **E8R** as a yellow solid (18 mg, yield 44%). ¹H NMR (500 MHz, DMSO-*d*₆) *δ* 8.75 (s, 1H), 8.27 (br., 1H), 8.26 (s, 1H), 8.09 (s, 1H), 7.76 (br., 1H), 7.35 - 7.29 (m, 1H), 7.07 (br., 1H), 6.53 (s, 1H), 4.19 (dd, *J =* 10.5, 2.6 Hz, 1H), 4.02 (t, *J* = 7.0 Hz, 2H), 3.87 - 3.74 (m, 3H), 3.71 (s, 3H), 3.00 - 2.94 (m, 1H), 2.90 - 2.70 (m, 6H), 2.63 - 2.57 (m, 3H), 2.23 (s, 3H), 2.15 - 2.08 (m, 2H), 2.02 - 1.95 (m, 1H), 1.61 (t, *J =* 10.6 Hz, 1H). MS *m*/*z* 592.2, 594.1 [M+H]⁺.

### Example 38: Preparation of Compound E9R

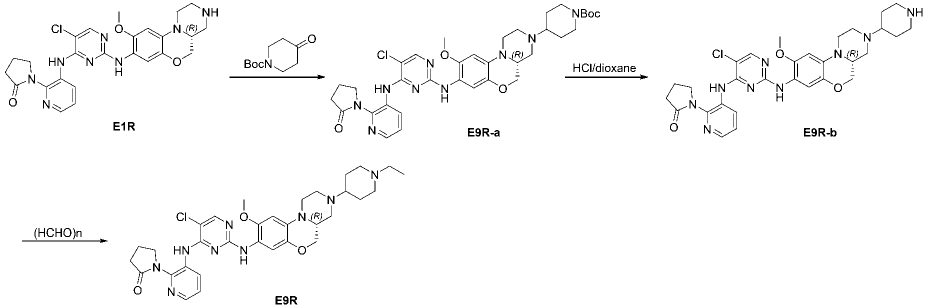

Compound **E1R** (100 mg, 0.19 mmol), N-Boc-4-piperidone (57 mg, 0.29 mmol) and zinc chloride (52 mg, 0.38 mmol) were dissolved in methanol (5 mL), followed by the addition of sodium cyanoborohydride (24 mg, 0.38 mmol). The reaction mixture was stirred at 75 °C for 1 hour. TLC monitoring indicated the completion of the reaction, and the reaction mixture was concentrated under reduced pressure. The obtained crude product was separated and purified by silica gel column chromatography (dichloromethane:methanol *=* 20:1, 2% ammonia water) to afford compound **E9R-a** as a yellow solid (100 mg, yield 74%).

Compound **E9R-a** (100 mg, 0.14 mmol) was dissolved in dichloromethane (2 mL), and then a dioxane solution of hydrochloric acid (4.0 M, 1 mL) was added. The reaction mixture was stirred at room temperature for 2 hours. TLC monitoring showed the reaction was completed. The reaction mixture was cooled to room temperature and then concentrated under reduced pressure. The obtained mixture was dissolved in a small amount of methanol, ammonia water was added for neutralization, and then concentrated under reduced pressure. The resulting crude product was separated and purified by silica gel column chromatography (dichloromethane:methanol *=* 20:1, 2% ammonia water) to obtain compound **E9R-b** as a yellow solid (70 mg, yield 82%).

Compound **E9R-b** (20 mg, 0.03 mmol), acetaldehyde (2 mg, 0.05 mmol) and zinc chloride (8 mg, 0.06 mmol) were dissolved in methanol (2 mL), followed by the addition of sodium cyanoborohydride (4 mg, 0.06 mmol). The reaction mixture was stirred at 75 °C for 1 hour. TLC monitoring indicated the completion of the reaction. The reaction mixture was concentrated under reduced pressure. The obtained crude product was separated and purified by silica gel column chromatography (dichloromethane:methanol = 20:1, 2% ammonia water) to afford compound **E9R** as a yellow solid (11 mg, yield 53%). ¹H NMR (500 MHz, DMSO-*d*₆) *δ* 8.75 (s, 1H), 8.29 - 8.26 (m, 1H), 8.26 (s, 1H), 8.09 (s, 1H), 7.75 (br., 1H), 7.35 - 7.30 (m, 1H), 7.06 (br., 1H), 6.52 (s, 1H), 4.20 (dd, *J =* 10.5, 2.3 Hz, 1H), 4.02 (t, *J* = 7.0 Hz, 2H), 3.83 (dd, *J =* 10.5, 8.9 Hz, 1H ), 3.79 - 3.74 (m, 1H), 3.71 (s, 3H), 2.99 - 2.87 (m, 5H), 2.60 (t, *J* = 7.9 Hz, 2H), 2.57 - 2.54 (m, 1H), 2.34 - 2.26 (m, 3H), 2.23 - 2.17 (m, 1H), 2.15 - 2.08 (m, 2H), 1.92 - 1.73 (m, 5H), 1.46 - 1.37 (m, 2H), 0.98 (t, *J* = 7.2 Hz, 3H). MS *m*/*z* 634.2, 636.2 [M+H]⁺.

### Example 39: Preparation of Compound E10R

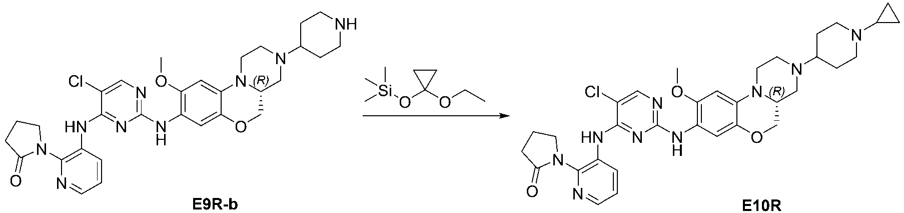

Compound **E9R-b** (20 mg, 0.03 mmol) and 1-ethoxy-1-trimethylsilyloxycyclopropane (11 mg, 0.06 mmol) were dissolved in methanol (1 mL), then 1 drop of acetic acid was added dropwise. The mixture was stirred at room temperature for 1 hour, and then sodium cyanoborohydride (19 mg, 0.3 mmol) was added. The reaction system was stirred continuously at room temperature for 12 hours. TLC monitoring indicated the completion of the reaction, and the reaction mixture was concentrated under reduced pressure. The obtained crude product was separated and purified by silica gel column chromatography (dichloromethane:methanol *=* 20:1, 2% ammonia water) to afford compound **E10R** as a yellow solid (7.68 mg, yield 36%). ¹H NMR (500 MHz, CD₃OD) *δ* 8.29 (dd, *J =* 4.6, 1.5 Hz, 1H), 8.26 (dd, *J =* 8.1, 1.5 Hz, 1H), 7.99 (s, 1H), 7.39 (dd, *J =* 8.1, 4.7 Hz, 1H), 7.24 (s, 1H), 6.49 (s, 1H), 4.18 (dd, *J* = 10.5, 2.5 Hz, 1H), 4.09 (t, *J* = 7.0 Hz, 2H), 3.89 (dd, *J* = 10.3, 9.0 Hz, 1H), 3.79 (s, 3H), 3.73 (d, *J* = 11.8 Hz, 1H), 3.14 - 2.96 (m, 5H), 2.74 - 2.69 (m, 1H), 2.66 (t, *J =* 8.0 Hz, 2H), 2.46 - 2.39 (m, 1H), 2.38 - 2.31 (m, 1H), 2.29 - 2.17 (m, 4H), 2.02 (t, *J* = 10.7 Hz, 1H), 1.91 (d, *J* = 12.4 Hz, 2H), 1.68 - 1.62 (m, 1H), 1.58 - 1.48 (m, 2H), 0.51 - 0.47 (m, 2H), 0.44 - 0.39 (m, 2H). MS *m*/*z* 646.3, 648.2 [M+H]⁺.

### Example 40: Preparation of Compound E11R

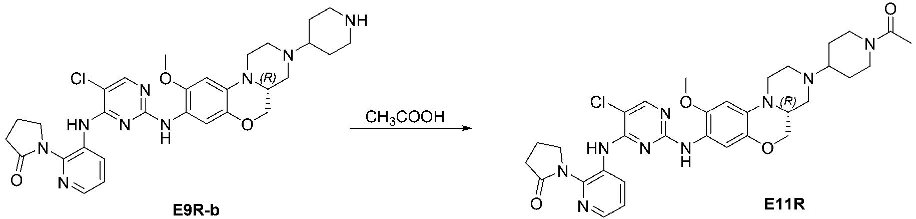

Compound **E9R-b** (20 mg, 0.03 mmol) was dissolved in acetonitrile (1 mL), followed by the sequential addition of acetic acid (4 mg, 0.06 mmol), N,N-diisopropylethylamine (12 mg, 0.09 mmol), and O-(7-azabenzotriazol-1-yl)-N,N,N',N'-tetramethyluronium hexafluorophosphate (23 mg, 0.06 mmol). The mixture waSR^{e}acted at room temperature for 1 hour. After the reaction was completed, the reaction solution was evaporated to dryness under reduced pressure to obtain a crude product, which was separated and purified by silica gel column chromatography (dichloromethane:methanol = 20:1) to afford compound **E11R** as a solid (14.35 mg, yield 67%). ¹H NMR (500 MHz, DMSO-*d*₆) *δ* 8.75 (s, 1H), 8.29 - 8.26 (m, 1H), 8.26 (s, 1H), 8.09 (s, 1H), 7.75 (br., 1H), 7.34 - 7.30 (m, 1H), 7.06 (br., 1H), 6.52 (s, 1H), 4.39 (d, *J* = 12.2 Hz, 1H), 4.20 (d, *J =* 10.8 Hz, 1H), 4.02 (t, *J =* 7.0 Hz, 2H), 3.86 - 3.80 (m, 2H), 3.79 - 3.74 (m, 1H), 3.71 (s, 3H), 3.01 (t, *J* = 12.7 Hz, 1H), 2.96 - 2.87 (m, 3H), 2.65 - 2.53 (m, 5H), 2.39 - 2.32 (m, 1H), 2.15 - 2.08 (m, 2H), 1.99 (s, 3H), 1.94 (t, *J* = 10.5 Hz, 1H), 1.83 - 1.73 (m, 2H), 1.45 - 1.30 (m, 2H). MS *m*/*z* 648.2, 650.2 [M+H]⁺.

### Example 41: Preparation of Compound A10R

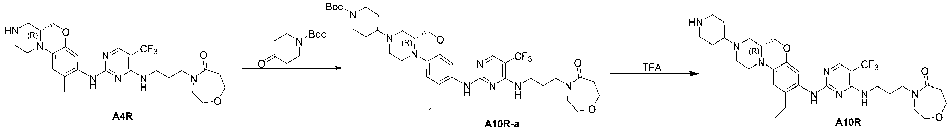

The trifluoroacetate salt of compound **A4R** (80 mg, 0.12 mmol) and N-tert-butoxycarbonyl-4-piperidone (48 mg, 0.24 mmol) were dissolved in tetrahydrofuran (3 mL) and stirred at room temperature for 15 minutes. Then sodium triacetoxyborohydride (102 mg, 0.48 mmol) was added, and the stirring was continued at room temperature for 6 hours. After the reaction was completed, water was added for dilution. The mixture was extracted with ethyl acetate (3×10 mL). The combined organic phases were washed with saturated brine, dried over anhydrous sodium sulfate, filtered and concentrated under reduced pressure. The obtained crude product was separated and purified by silica gel column chromatography (dichloromethane:methanol = 20:1, 2% ammonia water) to afford compound **A10R-a** as a yellow solid (47 mg, yield 52%). MS m/z 733.3 [M+H]⁻.

Compound **A10R-a** (47 mg, 0.06 mmol) was dissolved in dichloromethane (4 mL), and then trifluoroacetic acid (2 mL) was added. The reaction mixture was stirred at room temperature for 60 minutes. After the reaction was completed, the reaction solution was concentrated under reduced pressure. The concentrated crude product was dissolved in methanol (4 mL), then ammonia water (2 mL) was added. The mixture was stirred for 15 minutes and concentrated again under reduced pressure. The obtained crude product was separated and purified by silica gel column chromatography (dichloromethane:methanol = 15:1, 2% ammonia water) to obtain compound **A10R** as a white solid (22.5 mg, yield 55%). ¹H NMR (500 MHz, DMSO-*d*₆) *δ* 8.54 (s, 1H), 8.06 - 7.99 (m, 1H), 6.94 (s, 1H), 6.73 - 6.59 (m, 2H), 4.19 (dd, *J* = 10.5, 2.3 Hz, 1H), 3.90 - 3.80 (m, 1H), 3.76 - 3.68 (m, 1H), 3.66 - 3.52 (m, 4H), 3.43 - 3.35 (m, 2H), 3.29 - 3.16 (m, 4H), 3.02 - 2.84 (m, 5H), 2.65 - 2.51 (m, 3H), 2.47 - 2.25 (m, 6H), 1.90 (t, *J* = 10.3 Hz, 1H), 1.75 - 1.65 (m, 2H), 1.64 - 1.50 (m, 2H), 1.32 - 1.20 (m, 2H), 1.03 (t, *J* = 7.5 Hz, 3H), One of the H atoms is in the solvent peak.MS *m*/*z* 633.1 [M+H]⁺.

### Example 42: Preparation of Compound A11R

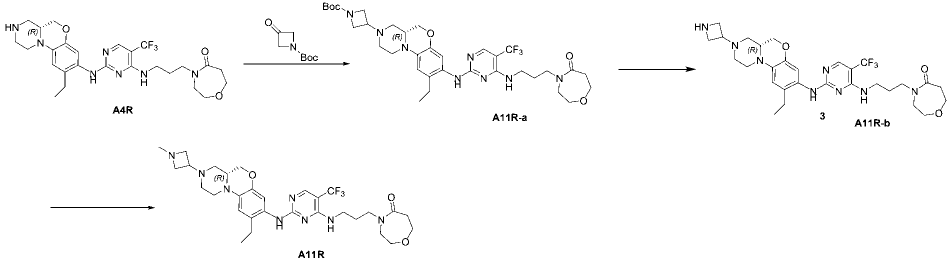

Compound **A4R** (40 mg, 0.06 mmol) and N-tert-butoxycarbonyl-4-cyclobutanone (21 mg, 0.12 mmol) were dissolved in tetrahydrofuran (3 mL) and stirred at room temperature for 15 minutes. Then sodium triacetoxyborohydride (26 mg, 0.12 mmol) was added, and stirring was continued at room temperature for 6 hours. After the reaction was completed, water was added for dilution. The mixture was extracted with ethyl acetate (3×10 mL). The combined organic phases were washed with saturated brine, dried over anhydrous sodium sulfate, filtered, and concentrated under reduced pressure. The obtained crude product was separated and purified by silica gel column chromatography (dichloromethane:methanol = 20:1, 2% ammonia water) to afford compound **A11R-a** as a yellow solid (14 mg, yield 32%). MS m/z 705.5 [M+H]⁺.

Compound **A4R-a** (14 mg, 0.02 mmol) was dissolved in dichloromethane (2 mL), and a 4M hydrochloric acid in dioxane solution (0.2 mL) was slowly added dropwise under ice bath. The reaction solution was stirred at room temperature for 1 hour. Ammonia water was added to the reaction solution to adjust the pH to 8-10, and the mixture was concentrated under reduced pressure. The obtained crude product was separated and purified by preparative thin-layer chromatography to obtain compound **A11R-b** as a white solid (10 mg, yield 83%). MS m/z 605.3 [M+H]⁺.

Compound **A11R-b** (10 mg, 0.016 mmol) was dissolved in methanol (1 mL). Paraformaldehyde (1.5 mg, 0.048 mmol) was added, followed by 1 drop of acetic acid. After stirring at room temperature for 30 minutes, sodium cyanoborohydride (3 mg, 0.05 mmol) was added. The reaction mixture was stirred at room temperature overnight, then the reaction solution was quenched by adding water, and the mixture was extracted with ethyl acetate (3×5 mL). The combined organic phases were washed with saturated brine, dried over anhydrous sodium sulfate, filtered, and concentrated under reduced pressure. The obtained crude product was separated and purified by preparative thin-layer chromatography to obtain compound **A11R** as a white solid (0.43 mg, yield 0.42%). MS m/z 619.0 [M+H]⁺.

### Example 43: Preparation of Compound A12R

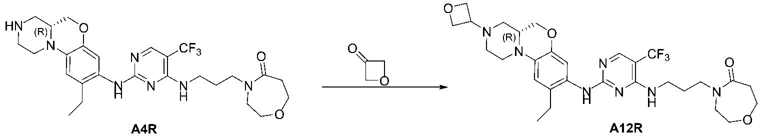

The trifluoroacetate salt of compound **A4R** (35 mg, 0.05 mmol) and 3-oxetanone (7 mg, 0.10 mmol) were dissolved in tetrahydrofuran (2 mL) and stirred at room temperature for 30 minutes. Then sodium triacetoxyborohydride (42 mg, 0.20 mmol) was added, and stirring was continued for 1 hour. After the reaction was completed, water was added for dilution. The mixture was extracted with ethyl acetate (3×10 mL). The combined organic phases were washed with saturated brine, dried over anhydrous sodium sulfate, filtered, and concentrated under reduced pressure. The obtained crude product was separated and purified by silica gel column chromatography (dichloromethane:methanol = 15:1, 2% ammonia water) to afford compound **A12R** as a white solid (7 mg, yield 21%). ¹H NMR (500 MHz, DMSO-*d*₆) δ 8.56 (s, 1H), 8.03 (s, 1H), 7.03 - 6.88 (m, 1H), 6.78 - 6.56 (m, 2H), 4.63 - 4.40 (m, 4H), 4.19 (dd, *J* = 10.6, 2.5 Hz, 1H), 3.89 - 3.81 (m, 1H), 3.76 (d, *J* = 11.2 Hz, 1H), 3.68 - 3.55 (m, 4H), 3.48 - 3.38 (m, 3H), 3.29 - 3.19 (m, 4H), 3.08 - 2.97 (m, 1H), 2.88 - 2.72 (m, 2H), 2.70 - 2.56 (m, 3H), 2.48 - 2.40 (m, 2H), 2.07 - 1.95 (m, 1H), 1.68 - 1.49 (m, 3H), 1.03 (t, *J* = 7.5 Hz, 3H). MS *m*/*z* 606.1 [M+H]⁺.

### Example 44: Preparation of Compound A13R

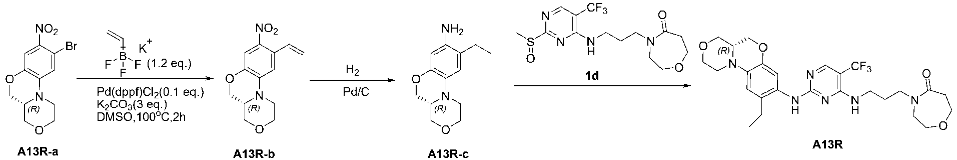

Compound **A13R-a** (1.19 g, 3.78 mmol), potassium vinyltrifluoroborate (607 mg, 4.53 mmol), [1,1'-bis(diphenylphosphino)ferrocene]dichloropalladium(II) (138 mg, 0.189 mmol) and potassium carbonate (1.57 g, 11.33 mmol) were dissolved in dimethyl sulfoxide (15 mL). The mixture was heated to 100°C under a nitrogen atmosphere and stirred continuously for 2 hours. After the reaction was completed, water was added for dilution. The mixture was extracted with ethyl acetate (3×30 mL). The combined organic phases were washed with saturated brine, dried over anhydrous sodium sulfate, filtered, and concentrated under reduced pressure. The obtained crude product was separated and purified by silica gel column chromatography (petroleum ether:ethyl acetate = 5:1) to afford compound **A13R-b** as a yellow solid (360 mg, yield 36%). MS m/z 262.9 [M+H]⁺.

Compound **A13R-b** (600 mg, 1.37 mmol) and palladium on carbon (10%, 60 mg) were dissolved in methanol (10 mL). The reaction mixture was stirred at room temperature under a hydrogen atmosphere for 60 minutes. TLC monitoring indicated the completion of the reaction. The reaction mixture was filtered through celite, and the filtrate was concentrated under reduced pressure to obtain crude compound **A13R-c** (321 mg, yield 100%). MS m/z 235.1 [M+H]⁺.

Compound **A13R-c** (40 mg, 0.17 mmol) and compound **1d** (71 mg, 0.19 mmol) were dissolved in N,N-dimethylacetamide (3 mL), and p-toluenesulfonic acid (44 mg, 0.26 mmol) was added. The mixture was heated to 100°C and reacted for 6 hours. After the reaction was completed, ammonia water was added to the reaction solution to adjust the pH to 7-9, and then the mixture was extracted with ethyl acetate three times. The combined organic phases were washed with saturated brine, dried over anhydrous sodium sulfate, and filtered. The filtrate was evaporated to dryness under reduced pressure to obtain a crude product, which was purified by silica gel column chromatography (petroleum ether:ethyl acetate = 1:1) to obtain compound **A13R** (25 mg, yield 26%). ¹H NMR (500 MHz, DMSO-*d*₆) *δ* 8.57 (s, 1H), 8.03 (s, 1H), 6.95 (br, 1H), 6.70 (br, 1H), 6.67 (s, 1H), 4.17 (dd, *J =* 10.6, 2.6 Hz, 1H), 3.95 (d, *J =* 10.2 Hz, 1H), 3.87-3.78 (m, 2H), 3.68-3.57 (m, 6H), 3.42-3.37 (m, 3H), 3.28-3.19 (m, 3H), 3.15 (t, *J =* 10.7 Hz, 1H), 3.08-3.02 (m, 1H), 2.69-2.59 (m, 3H), 2.48-2.42 (m, 2H), 1.58 (br, 2H), 1.03 (t, *J =* 7.5 Hz, 3H). MS *m*/*z* 647.0 [M+H]⁺.

### Example 45: Preparation of Compound A14R

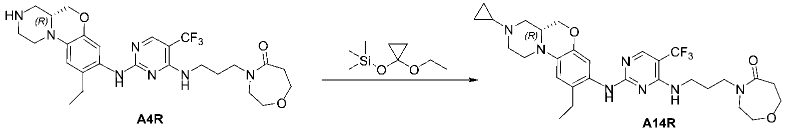

Compound **A4R** (30 mg, 0.05 mmol) and compound 1-ethoxy-1-trimethylsiloxycyclopropane (95 mg, 0.5 mmol) were dissolved in methanol, followed by the addition of one drop of acetic acid. The mixture was stirred at room temperature for 1 hour, and then sodium cyanoborohydride (34 mg, 0.50 mmol) was added. After stirring at room temperature for 14 hours, the reaction was completed, and a saturated sodium bicarbonate solution was added. The resulting mixture was extracted three times with dichloromethane, and the organic phases were combined. The combined organic phase was washed with saturated brine, dried over anhydrous sodium sulfate, and filtered. The filtrate was evaporated to dryness under reduced pressure to obtain the crude product. The crude product was purified by silica gel column chromatography to obtain compound 14R (2.56 mg, yield 8%). ¹H NMR (500 MHz, DMSO-*d*₆) *δ* 8.54 (s, 1H), 8.03 (s, 1H), 6.95 (br, 1H), 6.71-6.60 (m, 2H), 4.20 (dd, *J* = 10.5, 2.4 Hz, 1H), 3.88-3.81 (m, 1H), 3.75-3.67 (m, 1H), 3.66-3.55 (m, 4H), 3.38 (br, 2H), 3.24 (br, 4H), 3.03-2.96 (m, 1H), 2.97-2.87 (m, 2H), 2.65-2.57 (m, 2H), 2.47-2.33 (m, 4H), 1.97 (t, *J* = 10.5 Hz, 1H), 1.70-1.63 (m, 1H), 1.58 (br, 2H), 1.03 (t, *J =* 7.5 Hz, 3H), 0.49-0.40 (m, 2H), 0.40-0.32 (m, 2H). MS *m*/*z* 590.4[M+H]⁺.

### Example 46: Preparation of Compound A2S

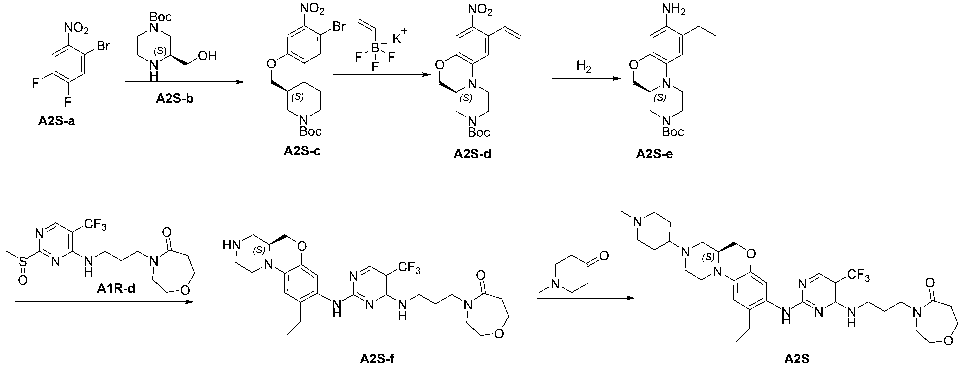

Compound **A2S-a** (1000 mg, 4.20 mmol) and compound 2S-b (908 mg, 4.20 mmol) were dissolved in dimethyl sulfoxide (30 mL), followed by the addition of potassium hydroxide (1178 mg, 21.00 mmol). The mixture was stirred at room temperature for 5 hours and then heated to 60 °C overnight. After the reaction was completed, water was added for dilution. The mixture was extracted with ethyl acetate (3 × 30 mL). The combined organic phases were washed with saturated brine, dried over anhydrous sodium sulfate, filtered, and then concentrated under reduced pressure. The obtained crude product was separated and purified by silica gel column chromatography (petroleum ether: ethyl acetate *=* 5:1) to give compound **A2S-c** as a yellow solid (800 mg, yield 46%). MS m/z 413.1 [M+H]⁻.

Compound **A2S-c** (800 mg, 1.94 mmol), potassium vinyltrifluoroborate (414 mg, 3.09 mmol), [1,1'-bis(diphenylphosphino)ferrocene]dichloropalladium (75 mg, 0.103 mmol) and potassium carbonate (854 mg, 6.18 mmol) were dissolved in dimethyl sulfoxide (10 mL). The mixture was heated to 100 °C under a nitrogen atmosphere and stirred for 2 hours. After the reaction was completed, water was added for dilution. The mixture was extracted with ethyl acetate (3 × 30 mL). The combined organic phases were washed with saturated brine, dried over anhydrous sodium sulfate, filtered, and then concentrated under reduced pressure. The obtained crude product was separated and purified by silica gel column chromatography (petroleum ether: ethyl acetate = 5:1) to give compound **A2S-d** as a yellow solid (670 mg, yield 96%). MS m/z 362.1 [M+H]⁻.

Compound **A2S-d** (600 mg, 1.66 mmol) and palladium on carbon catalyst (10%, 200 mg) were dissolved in methanol (10 mL). The reaction mixture was stirred at room temperature under a hydrogen atmosphere of 1 atmosphere for 60 minutes. TLC monitoring indicated the completion of the reaction. The reaction mixture was filtered through celite, and the filtrate was concentrated under reduced pressure to obtain the crude product. The obtained crude product was separated and purified by silica gel column chromatography (petroleum ether: ethyl acetate = 2:1) to give compound **A2S-e** as a pale yellow solid (500 mg, yield 90%). MS m/z 334.1 [M+H]⁺.

Compound **A2S-e** (120 mg, 0.36 mmol) and compound **A1R-d** (152 mg, 0.40 mmol) were dissolved in N,N-dimethylformamide (3 mL), followed by the addition of trifluoroacetic acid (82 mg, 0.72 mmol). The reaction mixture was stirred at 100 °C overnight. After the reaction was completed, water was added for dilution, and then triethylamine (1 mL) was added. The mixture was extracted with ethyl acetate (3 × 20 mL). The combined organic phases were washed with saturated brine, dried over anhydrous sodium sulfate, filtered, and then concentrated under reduced pressure. The obtained crude product was separated and purified by silica gel column chromatography (dichloromethane: methanol: ammonia water = 10:1:0.2) to give compound **A2S-f** as a yellow solid (80 mg, yield 40%). MS m/z 550.2 [M+H]⁺.

Compound **A2S-f** (20 mg, 0.04 mmol) and N-methyl-4-piperidone (14 mg, 0.12 mmol) were dissolved in dimethylacetamide (2 mL), followed by the addition of glacial acetic acid (0.1 mL). The reaction mixture was stirred at 60 °C for 30 minutes, then cooled to room temperature. Sodium triacetoxyborohydride (25 mg, 0.12 mmol) was added to the reaction mixture at room temperature. The reaction mixture was then heated to 75 °C and stirred at 75 °C for 60 minutes. After the reaction was completed, water was added for dilution. The mixture was extracted with ethyl acetate (3 × 10 mL). The combined organic phases were washed with saturated brine, dried over anhydrous sodium sulfate, filtered, and then concentrated under reduced pressure. The obtained crude product was separated and purified by silica gel column chromatography (dichloromethane: methanol = 15:1, with 2% ammonia water) to give compound **A2S** as a white solid (4 mg, yield 17%). ¹H NMR (500 MHz, DMSO-*d*₆) *δ* 8.58 - 8.49 (m, 1H), 8.03 (s, 1H), 6.98 - 6.90 (m, 1H), 6.72 - 6.60 (m, 2H), 4.19 (dd, *J =* 10.5, 2.3 Hz, 1H), 3.89 - 3.79 (m, 1H), 3.76 - 3.69 (m, 1H), 3.67 - 3.52 (m, 4H), 3.42 - 3.36 (m, 2H), 3.26 - 3.18 (m, 4H), 3.01 - 2.82 (m, 5H), 2.65 - 2.54 (m, 3H), 2.48 - 2.44 (m, 2H), 2.33 - 2.16 (m, 5H), 2.03 - 1.83 (m, 3H), 1.82 - 1.72 (m, 2H), 1.62 - 1.53 (m, 2H), 1.50 - 1.40 (m, 2H), 1.03 (t, *J =* 7.5 Hz, 3H). MS *m*/*z* 647.0 [M+H]⁺.

### Example 47: Kinase Activity Inhibition Assay

### 1. ULK Kinase Activity Inhibition Assay

The ADP-Glo method was used to evaluate the inhibitory activity of the compounds against ULK1 and ULK2 kinases. Prepare 1× kinase reaction buffer (final working concentrations: HEPES 50 mM, MgCl₂ 10 mM, Brij35 0.01%, EGTA 1 mM, DTT 2 mM). Prepare 2× kinase and 4× substrate solutions: ULK1 kinase at 0.5 nM; substrate mix containing ATP at 100 µM and MBP at 0.1 mg/mL. ULK2 kinase at 0.15 nM; substrate mix containing ATP at 6 µM and MBP at 0.1 mg/mL. Compounds were serially diluted in DMSO and shaken on a shaker for 20 minutes. Prepare 2× kinase using 1× kinase reaction buffer. Transfer 20 nL of compound into a 384-well plate. Add 2 µL of kinase to each well of the reaction plate. Seal the plate with sealing film, centrifuge at 1000 rpm for 60 seconds, and incubate at 25°C for 10 minutes. Prepare 2× ATP & MBP mixture using 1× kinase reaction buffer and add 2 µL to each well. Seal the plate, centrifuge at 1000 rpm for 60 seconds, and incubate at 25°C for 60 minutes. Add 4 µL ADP-Glo reagent to the 384-well plate, centrifuge at 1000 rpm for 1 minute, and incubate at 25°C for 40 minutes. Then, add 8 µL Detection Reagent, centrifuge at 1000 rpm for 1 minute, and incubate at 25°C for 40 minutes. Read the RLU (Relative Luminescence Unit) signal using a BMG plate reader to indicate kinase activity. Inhibition rate was calculated as: % Inhibition *=* 100% - [(Compound - Positive Control)/(Negative Control - Positive Control)] × 100%. IC₅₀ values (half-maximal inhibitory concentration) were calculated by nonlinear regression using GraphPad Prism 7.0. Y = Bottom + (Top - Bottom)/(1 + 10^((LogIC₅₀ - X) × Hill Slope)), where X is the log of compound concentration and Y is the % inhibition. IC₅₀ values are shown in Table 1.

### 2. FAK Kinase Activity Inhibition Assay

### MethodA:

The HTRF method was used to evaluate the inhibitory activity of the compounds against FAK kinase. Prepare 1× kinase reaction buffer (MgCl₂ 5 mM, SEB 25 nM, DTT 1 mM). The reaction conditions were: FAK kinase at 0.1 ng/µL; ATP 10 µM; substrate concentration 1 µM. Compounds were serially diluted in DMSO and shaken on a shaker for 20 minutes. Prepare 2× kinase using 1× reaction buffer. Transfer 25 nL compound to a 384-well plate using Echo 655. Add 2.5 µL kinase per well. Seal the plate with sealing film, centrifuge at 1000 rpm for 60 seconds, and incubate at 25°C for 12 hours. Prepare 2× ATP/substrate mix using 1× enzyme reaction buffer and add 2.5 µL 2× ATP/substrate mix per well. Seal the plate with sealing film, centrifuge at 1000 rpm for 60 seconds, and incubate at 25°C for 50 minutes. Prepare 2×XL665 & antibody solution using HTRF detection buffer, and add 5 µL per well. Incubate at 25°C for 60 minutes. Read fluorescence at 620 nm (Cryptate) and 665 nm (XL665). Calculate the ratio per well (Ratio_665/620 nm). % Inhibition = 100% - [(Compound Ratio - Positive Control Ratio)/ (Negative Control Ratio - Positive Control Ratio)] × 100%. IC₅₀ values (half-maximal inhibitory concentration) were calculated by nonlinear regression using GraphPad Prism 7.0. Y = Bottom + (Top - Bottom)/ (1 + 10^ ((LogIC₅₀ - X) × Hill Slope)), where X is the log of compound concentration and Y is the % inhibition. IC₅₀ values are shown in Tables 2, 3, and 4.

### Method B:

The Lantha screen assay was used to evaluate the FAK kinase inhibitory activity. Compounds were dissolved in 100% DMSO at 10 mM stock concentration. Prepare 1× kinase buffer (25 mM HEPES, pH 7.5, 0.01 mM Triton, 0.5 mM EGTA, 0.01% Brij-35). Dilute compounds in a gradient, and transfer 5 µL compound into the reaction plate. Prepare 2× kinase solution with 1× Kinase buffer. Add 5 µL kinase solution to compound and positive control wells, and 5 µL 1× Kinase buffer to negative control wells. Mix reaction plate by shaking. Prepare 4× ATP/Fluorescein-polyGT substrate mix with 1× Kinase buffer. Add 2.5 µL substrate solution to initiate the reaction. Shake and incubate at room temperature for 30 minutes. Prepare 2× detection reagent (2 nM antibody, 10 mM EDTA) and add 10 µL per well to stop the reaction. Briefly centrifuge, mix, and incubate for 60 minutes. Read fluorescence at 340 nm excitation and 520 nm/495 nm emission using EnVision. Collect data and calculate 520 nm/495 nm ratio. Calculate the inhibition percentage using the formula: % Inhibition = (Max - Sample Ratio)/(Max - Min) × 100, where min is the mean of negative control wells (no enzyme activity), and max is the mean of positive control wells (DMSO-inhibited conversion rate).Use XLFit Excel add-in version 5.4.0.8 to determine the IC₅₀ values of each compound against the enzyme activity: Y = Bottom + (Top - Bottom)/(1 + (IC₅₀/X)^HillSlope). IC₅₀ values are shown in Tables 3 and 4.

### 3. AXL, FLT3 Kinase Activity Inhibition Assay

Testing compound inhibitory activity against FLT3 and AXL kinases using Mobility shift assay. Compounds were dissolved in 100% DMSO to prepare 10 mM stock solutions. Prepare 1× Kinase Buffer. Test compounds were serially diluted in 5-fold gradients, and 250 nL of 100× final concentration compounds were transferred to destination plate 3573 using an Echo 550 liquid handler. A 2.5× final concentration kinase solution was prepared in 1× Kinase Buffer. Then, 10 µL of the 2.5× kinase solution was added to both compound wells and positive control wells, while 10 µL of 1× Kinase Buffer was added to negative control wells. The plate was centrifuged at 1000 rpm for 30 seconds, mixed by orbital shaking, and incubated at room temperature for 10 minutes. A 5/3× final concentration mixture of ATP and Kinase Substrate 25 was prepared in 1× Kinase Buffer. The reaction was initiated by adding 15 µL of the 5/3× ATP/substrate mixture. The 384-well plate was centrifuged at 1000 rpm for 30 seconds, mixed by shaking, and incubated at room temperature for 120 minutes. The kinase reaction was terminated by adding 30 µL stop solution, followed by centrifugation at 1000 rpm for 30 seconds and mixing. Conversion rates were measured using a Caliper EZ Reader. %Inhibition = (Conversion%_max - Conversion%_sample)/(Conversion%_max - Conversion%_min) × 100. Where: Conversion%_sample: Conversion rate reading of test samples; Conversion%_min: Mean value of negative control wells (representing conversion rate without enzyme activity); Conversion%_max: Mean value of positive control wells (representing conversion rate without compound inhibition). Dose-response curves were plotted with log(concentration) as the X-axis and percentage inhibition as the Y-axis. Curve fitting was performed using GraphPad Prism 5 software with the "log(inhibitor) vs. response - Variable slope" model to derive IC₅₀ values for each compound's enzyme inhibitory activity. The fitting formula was: Y = Bottom + (Top - Bottom)/(1 + 10^((LogIC₅₀ - X) × HillSlope)). Compound IC₅₀ values are shown in Tables 3 and 4.

### 4. ALK Kinase Activity Inhibition Assay

Measure compound inhibitory activity against ALK kinase using HTRF Assay.1× kinase reaction buffer was prepared. The kinase reaction conditions were as follows: kinase concentration at 0.04 ng/µL; ATP working concentration in substrate mixture solution at 2 µM; final substrate concentration at 1 µM. Compounds were serially diluted in DMSO and shaken on an orbital shaker for 20 minutes. 2× kinase solution was prepared using 1× kinase reaction buffer. 1 µL of compound solution was transferred to a 384-well plate. 2 µL of kinase solution was added to each well of the reaction plate. The plate was sealed with adhesive film, centrifuged at 1000 rpm for 60 seconds, and incubated at 25°C for 10 minutes. 2.5× ATP/substrate mixture was prepared using 1× kinase reaction buffer. 2 µL of 2.5× ATP/substrate mixture was added to the reaction plate. The plate was sealed with adhesive film, centrifuged at 1000 rpm for 60 seconds, and incubated at 25°C for 50 minutes. 4× Sa-XL 665 solution was prepared using HTRF detection buffer. 5 µL of Sa-XL 665 solution and 5 µL of TK-antibody-Cryptate solution were added to each well. The plate was centrifuged at 1000 rpm for 60 seconds and incubated at 25°C for 60 minutes. Fluorescence signals were measured at 615 nm (Cryptate) and 665 nm (XL665) using a BMG microplate reader. The ratio for each well was calculated as: Ratio_665/615nm. The inhibition percentage was calculated as follows: % inhibition = 100% - [(Compound Ratio - Positive Control Ratio)/(Negative Control Ratio - Positive Control Ratio)] × 100%. The IC₅₀ values (half-maximal inhibitory concentration) of compounds were determined by fitting the data to the following nonlinear regression equation using GraphPad Prism 7.0 software: Y = Bottom + (Top - Bottom)/(1 + 10^((LogIC₃₀ - X) × Hill Slope)) where: X = log(compound concentration), Y = % inhibition. Compound IC₅₀ values are shown in Tables 3 and 4.

### 5. HPK1 Kinase Activity Inhibition Assay

Measure compound inhibitory activity against HPK1 kinase using Mobility shift assay. Compounds were dissolved in 100% DMSO to prepare 10 mM stock solutions. 1× Kinase buffer was prepared. Test compounds were serially diluted in 5-fold gradients, and 250 nL of 100× final concentration compounds were transferred to destination plate 3573 using an Echo 550 liquid handler. A 2.5× final concentration kinase solution was prepared in 1× Kinase Buffer. 10 µL of the 2.5× kinase solution was added to both compound wells and positive control wells, while 10 µL of 1× Kinase buffer was added to negative control wells. The plate was centrifuged at 1000 rpm for 30 seconds, mixed by orbital shaking, and incubated at room temperature for 10 minutes. A 5/3× final concentration mixture of ATP and Kinase substrate 25 was prepared in 1× Kinase buffer. 15 µL of the 5/3× ATP/substrate mixture was added to initiate the reaction. The 384-well plate was centrifuged at 1000 rpm for 30 seconds, mixed by shaking, and incubated at room temperature for 120 minutes. The kinase reaction was terminated by adding 30 µL stop solution, followed by centrifugation at 1000 rpm for 30 seconds and mixing. Conversion rates were measured using a Caliper EZ Reader. The inhibition percentage was calculated as: %Inhibition = (Conversion%_max - Conversion%_sample)/(Conversion%_max - Conversion%_min) × 100. Where Conversion%_sample: Conversion rate reading of test samples; Conversion%_min: Mean value of negative control wells (representing conversion rate without enzyme activity); Conversion%_max: Mean value of positive control wells (representing conversion rate without compound inhibition). Dose-response curves were plotted with log(concentration) as the X-axis and percentage inhibition as the Y-axis. Curve fitting was performed using GraphPad Prism 5 software with the "log(inhibitor) vs. response - Variable slope" model to derive IC₅₀ values for each compound's enzyme inhibitory activity. The fitting formula was: Y = Bottom + (Top - Bottom)/(1 + 10^((LogIC₅₀ - X) × HillSlope)). Compound IC₅₀ values are shown in Table 4.

### 6. CDK7 Kinase Activity Inhibition Assay

Measure compound inhibitory activity against CDK7/CycH/MAT1 kinase using ADP-Glo assay. 1× kinase reaction buffer was prepared (working concentrations: HEPES 50 mM, MgCl₂ 10 mM, Brij35 0.01%, DTT 2 mM). The kinase reaction conditions were as follows: Kinase concentration: 3.5 ng/µL; Substrate mixture working concentrations: ATP 30 µM, CDK substrate 0.1 mg/ mL. Compounds were serially diluted in DMSO and shaken on an orbital shaker for 20 minutes. 2× kinase solution was prepared using 1× kinase buffer. 1 µL of compound solution was transferred to a 384-well plate. 2 µL of kinase solution was added to each well of the reaction plate. The plate was sealed with adhesive film, centrifuged at 1000 rpm for 60 seconds, and incubated at 25°C for 10 minutes. 4× ATP&substrate mixture was prepared using 1× kinase buffer. 1 µL of 4× ATP&substrate mixture was added to the reaction plate. The plate was sealed with adhesive film, centrifuged at 1000 rpm for 60 seconds, and incubated at 25°C for 60 minutes. 4 µL of ADP-Glo reagent was transferred to the 384-well reaction plate, centrifuged at 1000 rpm for 1 minute, and incubated at 25°C for 40 minutes. 8 µL of Detection solution was transferred to the 384-well reaction plate, centrifuged at 1000 rpm for 1 minute, and incubated at 25°C for 40 minutes. Relative luminescence units (RLU) were measured using a BMG microplate reader, with signal intensity representing kinase activity level. The inhibition percentage was calculated as follows: % inhibition = 100% - [(Compound RLU - Positive Control RLU)/(Negative Control RLU - Positive Control RLU)] × 100%. The IC₅₀ values (half-maximal inhibitory concentration) of compounds were determined by fitting the data to the following nonlinear regression equation using GraphPad Prism 7.0 software: Y = Bottom + (Top - Bottom)/(1 + 10^((LogIC₅₀ - X) × Hill Slope)), where: X = log(compound concentration), Y = % inhibition. Compound IC₅₀ values are shown in Table 4.

### 7. TNK1 Kinase Activity Inhibition Assay

Measure compound inhibitory activity against TNK1 kinase using ADP-Glo Assay. 1× kinase reaction buffer was prepared (working concentrations: HEPES 50 mM, MgCl₂ 10 mM, Brij35 0.01%, EGTA 1 mM, DTT 2 mM). 2× kinase and 4× substrate buffers were prepared with the following working concentrations: For TNK1 kinase: kinase solution at 5 nM; substrate mixture containing ATP at 45 µM and MBP substrate at 0.1 mg/ mL. For ULK2 kinase: kinase solution at 0.15 nM; substrate mixture containing ATP at 6 µM and MBP substrate at 0.1 mg/mL. Compounds were serially diluted in DMSO and shaken on an orbital shaker for 20 minutes. 2× kinase solution was prepared using 1× kinase reaction buffer. 20 nL of compound solution was transferred to a 384-well plate. 2 µL of kinase solution was added to each well of the reaction plate. The plate was sealed with adhesive film, centrifuged at 1000 rpm for 60 seconds, and incubated at 25°C for 10 minutes. 2× ATP&MBP mixture was prepared using 1× kinase reaction buffer. 2 µL of 2× ATP&substrate mixture was added to the reaction plate. The plate was sealed with adhesive film, centrifuged at 1000 rpm for 60 seconds, and incubated at 25°C for 60 minutes. 4 µL of ADP-Glo reagent was transferred to the 384-well reaction plate, centrifuged at 1000 rpm for 1 minute, and incubated at 25°C for 40 minutes. 8 µL of Detection solution was transferred to the 384-well reaction plate, centrifuged at 1000 rpm for 1 minute, and incubated at 25°C for 40 minutes. Relative luminescence units (RLU) were measured using a BMG microplate reader, with signal intensity representing kinase activity level. The inhibition percentage was calculated as follows: % inhibition = 100% - [(Compound RLU - Positive Control RLU)/(Negative Control RLU - Positive Control RLU)] × 100%. The IC₅₀ values (half-maximal inhibitory concentration) of compounds were determined by fitting the data to the following nonlinear regression equation using GraphPad Prism 7.0 software: Y = Bottom + (Top - Bottom)/(1 + 10^((LogIC₅₀ - X) × Hill Slope)), where: X = log(compound concentration), Y = % inhibition. Compound IC₅₀ values are shown in Tables 4 and 5.

**Table1: Inhibitory activity of compounds against ULK (IC₅₀, nM)**

| **Compounds** | **ULK1** | **ULK2** |
|---|---|---|
| **A1R** | < 3 | < 1 |
| **A2R** | < 3 | <5 |
| **A3R** | < 3 | < 1 |
| **A4R** | < 1 | < 1 |
| **A5R** | < 1 | < 1 |
| **A6R** | < 3 | < 5 |
| **A7R** | < 3 | < 1 |
| **A8R** | < 1 | < 1 |

**Table 2: Inhibitory activity of compounds against FAK (IC₅₀, nM)**

| **Compounds** | **FAK (method A)** |
|---|---|
| **B1R** | < 0.5 |
| **B2R** | < 0.5 |
| **B3R** | < 0.5 |
| **B4R** | < 0.5 |
| **B5R** | < 0.5 |
| **B6R** | < 1 |
| **B7R** | < 0.5 |
| **B8R** | < 0.5 |
| **B9R** | < 0.5 |
| **B10R** | < 0.5 |

**Table 3: Inhibitory activity of compounds against FAK, AXL, FLT3 and ALK (IC₅₀, nM)**

| **Compounds** | **FAK(method A and B)** | **AXL** | **FLT3** | **ALK** |
|---|---|---|---|---|
| **C1S** | < 1 | < 30 | < 30 | |
| **C2S** | < 1 | < 30 | < 30 | < 0.05 |
| **C3S** | < 5 | | | |

**Table 4: Inhibitory activity of compounds against FAK, ALK, FLT3, HPK1, CDK7 and TNK1 (IC₅₀, nM)**

| **Compounds** | **FAK(method A and method B)** | **FLT3** | **HPK1** | **ALK** | **CDK7** | **TNK1** |
|---|---|---|---|---|---|---|
| **D1S** | < 0.5 | | | | | |
| **D1R** | <3 | < 30 | < 30 | | | |
| **D2S** | < 0.5 | < 50 | < 30 | < 15 | < 15 | < 10 |
| **D2R** | < 1 | < 20 | < 30 | < 10 | < 15 | |
| **D3S** | < 1 | | | | | |
| D4S | < 1 | < 70 | < 20 | < 15 | < 10 | |
| D5S | < 1 | | | | | |
| D6S | < 3 | | | | | |

**Table 5: Inhibitory activity of compounds against TNK1 (IC₅₀, nM)**

| **Compounds** | **TNK1** |
|---|---|
| **E1R** | < 0.5 |
| **E2R** | < 0.5 |
| **E3R** | < 0.5 |
| **E4R** | < 0.5 |
| **E5R** | < 0.5 |
| **E6R** | < 1 |
| **E7R** | < 2 |
| **E8R** | < 2 |
| **E9R** | < 1 |
| **E10R** | < 0.5 |
| **E11R** | < 1 |

### Example 48: Pharmacokinetic Study in Rats

Instrument: XEVO TQ-S LC-MS/MS system from Waters was used. All measurement data were collected and processed by Masslynx V4.1 software. Data were calculated and processed by Microsoft Excel. Pharmacokinetic parameters were calculated by statistical moment method using WinNonLin 8.0 software, mainly including Tmax, T_{1/2}, Cmax and AUC₀₋₂₄ₕ. Chromatographic column: ACQUITY UPLC BEH C18 (2.1 mm×50 mm, 1.7 µm). Column temperature: 40 °C. Mobile phase A was water (0.1% formic acid), mobile phase B was acetonitrile. Flow rate: 0.350 mL/min. Gradient elution program: 0.50 min: 10% B; 1.50 min: 90% B; 2.50 min: 90% B; 2.51 min: 10% B; 3.50 min: stop. Injection volume: 1 µL.

Animals: Three male SD rats weighing 200-220 g were used. After purchase, they were housed in the laboratory animal center for 2 days before experiment. Food was withheld for 12 h before dosing and 4 h after dosing, with free access to water. Blood samples were collected at scheduled time points after oral gavage.

Vehicle: 0.4% ethanol + 0.4% Tween 80 + 99.2% (0.5% methylcellulose M450). Preparation of oral gavage solution: The compound was accurately weighed and dissolved in vehicle by ultrasonication for 5 min at room temperature to prepare 0.3 mg/mL drug solution.

Drug samples: Multiple structurally similar compounds (molecular weight difference ≥2 units) were accurately weighed and administered together (cassette PK) to simultaneously screen and compare their oral absorption rates. Single compound administration was also used to study pharmacokinetics in rats.

Blood sample collection: After oral gavage, blood samples were collected from orbital sinus at 0.25, 0.5, 1, 2, 4, 8, 10 and 24 h. 50 µL plasma was mixed with 200 µL acetonitrile (containing 2 ng/mL verapamil as internal standard). The mixture was vortexed for 3 min, then centrifuged at 20000 rcf for 10 min at 4 °C. The supernatant was analyzed by LC-MS/MS.

Standard curves were established by accurately weighing and preparing compounds at different concentrations for mass spectrometric quantification. Compound concentrations in plasma samples were determined based on standard curves to obtain concentration-time profiles. All measurement data were collected and processed by relevant software. Pharmacokinetic parameters (including Tmax, T_{1/2}, Cmax and AUC₀₋₂₄ₕ) were calculated by statistical moment method. Pharmacokinetic parameters of representative compounds are shown in Table 6.

**Table 6 Pharmacokinetic parameters in rats**

| **Compounds** | **Dosage (oral, mg/Kg)** | **T_{1/2} (h)** | **Tₘₐₓ (h)** | **Cₘₐₓ (ng/mL)** | **AUC₀₋₂₄ₕ (h*ng/mL)** |
|---|---|---|---|---|---|
| **A1R** | 3 | 1.5 | 1.2 | 74.2 | 325.6 |
| **A2R** | 3 | 8.8 | 8 | 149 | 1935 |
| **A2S** | 3 | 7.05 | 1.17 | 11.81 | 98.03 |
| **A3R** | 3 | 1.4 | 1.2 | 223.1 | 1039.5 |
| **A4R** | 3 | 2.1 | 1.2 | 217.4 | 879.7 |
| **A6R** | 3 | 8.3 | 6.7 | 77.3 | 1083.2 |
| **A8R** | 3 | 1 | 0.8 | 222.5 | 685.4 |
| **A12R** | 3 | 1.76 | 0.5 | 486.73 | 1461.07 |
| **BIR** | 3 | 2.9 | 2.7 | 386.8 | 2330.1 |
| **B2R** | 3 | 7.4 | 4.7 | 76.4 | 879.9 |
| **B3R** | 3 | 7.9 | 3.3 | 68.9 | 690.4 |
| **B4R** | 3 | 1.5 | 0.8 | 199.6 | 567.9 |
| **B5R** | 3 | 2.8 | 2 | 117.9 | 685 |
| **B6R** | 3 | 1.3 | 1 | 298.6 | 904.9 |
| **B7R** | 3 | 1.2 | 2 | 389.6 | 1426.9 |
| **B8R** | 3 | 1.5 | 1.7 | 39.3 | 132.7 |
| **B9R** | 3 | 2.6 | 3.3 | 61.8 | 368.5 |
| **C1S** | 3 | 1.78 | 0.83 | 61.82 | 136.41 |
| **C2S** | 3 | 9.2 | 10 | 135.3 | 1968.2 |
| **D1S** | 3 | 2.63 | 1.67 | 2320.6 | 11027.21 |
| **D2S** | 3 | 5.97 | 3.33 | 134.18 | 1472.74 |
| **D4S** | 3 | 3.03 | 5.33 | 240.82 | 1660.22 |
| **D6S** | 3 | 5.74 | 6.67 | 52.31 | 675.46 |
| **E2R** | 3 | 2.07 | 1 | 74.44 | 331.91 |
| **E4R** | 3 | 0.99 | 0.5 | 68.96 | 83.91 |
| **E5R** | 3 | 1.38 | 1 | 499.84 | 1555.13 |
| **E6R** | 3 | 1.05 | 0.5 | 352.11 | 580.72 |
| **E7R** | 3 | 1.08 | 0.5 | 292.19 | 472.92 |
| **E8R** | 3 | 3.37 | 2 | 411.24 | 3168.26 |
| **E9R** | 3 | 14.48 | 9.33 | 71.59 | 1200.68 |
| **E10R** | 3 | 9.69 | 3.67 | 132.76 | 1995.47 |
| **E11R** | 3 | 4.36 | 1.33 | 1085.94 | 8107.59 |

All documents cited in the present invention are incorporated herein by reference as if each such document were individually incorporated by reference. Furthermore, it should be understood that after reading the above teachings of the present invention, those skilled in the art may make various changes or modifications to the invention, and such equivalent forms likewise fall within the scope defined by the appended claims of the present application.

## Claims

1. A compound of formula (I), or an optical isomer, pharmaceutically acceptable salt, prodrug, deuterated derivative, hydrate, or solvate thereof: 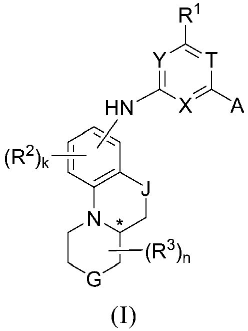
wherein the A is selected from Formula (IIa), Formula (IIb), Formula (IIc), Formula (IId) and Formula (IIe): 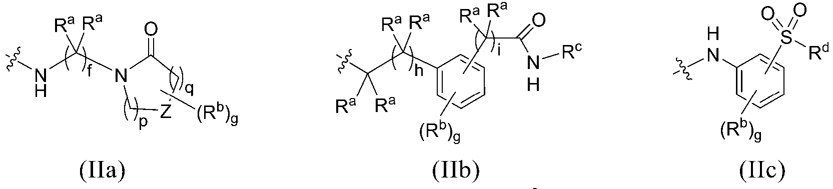 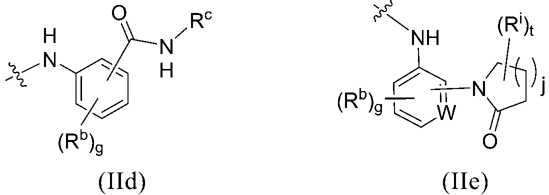
wherein in Formula (Ia), Formula (Ib), and Formula (Ic), " 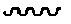 " represents the point which attach to other site of Formula (I);
"*" represents the chiral center;
X, Y and T are each independently N or CR¹;
each R¹ is independently selected from hydrogen, halogen, C₁₋₄ alkyl, C₁₋₄ haloalkyl, C₂₋₄ alkenyl, C₂₋₄ alkynyl, OR^{e} and CN;
each R² is independently selected from hydrogen, halogen, C₁₋₄ alkyl, C₁₋₄ haloalkyl, C₂₋₄ alkenyl, C₂₋₄ alkynyl, C₃₋₆ cycloalkyl, 3- to 6-membered heterocyclyl, OR^{e}, SR^{e}, NR^{e}R^{e}, CN and C(O)R^{h};
each R³ is independently selected from hydrogen and C₁₋₄ alkyl; or when two R³ are attached to the same carbon atom, said two R³ together with the carbon atom to which they are attached optionally form a carbonyl group (C=O);
J and G are each independently selected from NR^{f}, O, S, S(O), S(O)₂ and CR^{g}R^{g};
Z is selected from O, NR^{e} and CH₂;
W is N or CR^{b};
each R^{a} is hydrogen, halogen, or C₁₋₄ alkyl;
each R^{b} is independently selected from hydrogen, halogen, C₁₋₄ alkyl, C₁₋₄ haloalkyl, OR^{e}, SR^{e}, NR^{e}R^{e} and CN;
each R^{c} is independently selected from hydrogen, C₁₋₄ alkyl and C₃₋₆ cycloalkyl;
R^{d} is selected from C₁₋₄ alkyl, C₂₋₄ alkenyl, C₂₋₄ alkynyl, C₃₋₆ cycloalkyl, or 3- to 6-membered heterocyclyl;
each Rⁱ is independently selected from hydrogen, halogen, C₁₋₄ alkyl, C₁₋₄ haloalkyl, OR^{e}, SR^{e} and NR^{e}R^{e};
R^{f} is hydrogen, C₁₋₄ alkyl, C₁₋₄ haloalkyl, C₂₋₄ alkenyl, C₂₋₄ alkynyl, C₃₋₈ cycloalkyl, 3- to 12-membered heterocyclyl, aryl, heteroaryl, C(O)R^{h}, C(O)OR^{e}, C(O)NR^{e}R^{e}, S(O)₂R^{h} and S(O)₂NR^{e}R^{e}; wherein said alkyl, cycloalkyl, heterocyclyl, aryl, heteroaryl is optionally substituted by one or more substituents selected from the group consisting of halogen, C₁₋₄ alkyl, C₁₋₄ haloalkyl, C₃₋₆ cycloalkyl, 3- to 6-membered heterocyclyl, aryl, heteroaryl, OR^{e}, SR^{e}, NR^{e}R^{e}, CN, C(O)R^{h}, C(O)OR^{e}, C(O)NR^{e}R^{e}, NR^{e}C(O)R^{h}, S(O)₂R^{h}, S(O)₂NR^{e}R^{e} and NR^{e}S(O)₂R^{h};
each R^{g} is independently selected from the group consisting of hydrogen, halogen, C₁₋₄ alkyl, C₁₋₄ haloalkyl, OR^{e}, SR^{e}, NR^{e}R^{e}, CN, C(O)R^{h}, C(O)OR^{e}, C(O)NR^{e}R^{e}, NR^{e}C(O)R^{h}, or NR^{e}S(O)₂R^{h}; or two R^{g} attached to the same carbon atom together form a carbonyl group (C=O); or two R^{g} attached to the same carbon atom together form a 3- to 8-membered cyclic structure, said cyclic structure optionally containing 0, 1, or 2 heteroatoms selected from N, O and S;
each R^{e} is independently selected from hydrogen, C₁₋₄ alkyl, C₁₋₄ haloalkyl, C₃₋₆ cycloalkyl, or 3- to 6-membered heterocyclyl; or two R^{e} together with the nitrogen atom to which they are attached form a 3- to 8-membered heterocyclyl containing 1 or 2 N atoms and 0 or 1 heteroatom selected from O and S;
each R^{h} is independently selected from the group consisting of hydrogen, C₁₋₄ alkyl, C₁₋₄ haloalkyl, C₂₋₄ alkenyl, C₂₋₄ alkynyl, C₃₋₆ cycloalkyl, 3- to 6-membered heterocyclyl, aryl and heteroaryl;
k is 0, 1, 2, or 3;
n is 0, 1, 2, 3, or 4;
p and q are each independently 0, 1, 2, 3, 4, or 5;
f is 2, 3, 4, or 5;
g is 0, 1, 2, 3, or 4;
h is 0, 1, 2, or 3;
i is 0, 1, 2, or 3;
j is 0, 1, 2, 3, or 4;
t is 0, 1, 2, 3, or 4;
provided that when X is selected from CH, Y is selected from N, T is selected from CR¹, and A is selected from formula (IId), the structural fragment 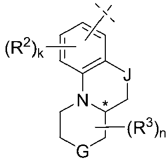
in formula (I) is selected from formula (IIf): 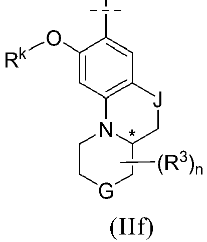
" ---" means the point in formula (IIf) which attach to the remaining part of formula (I);
R^{k} is selected from the group consisting of hydrogen, C₁₋₄ alkyl, C₁₋₄ haloalkyl, C₃₋₆ cycloalkyl and 3- to 6-membered heterocyclyl; the remaining groups in formula (IIf) are as defined above;
wherein, each of the aforementioned alkyl, alkenyl, alkynyl, cycloalkyl, heterocyclyl, cyclic structure, aryl, and heteroaryl groups is optionally and independently substituted with 1 to 3 substituents, each independently selected from the group consisting of: halogen, C₁₋₄ alkyl, C₁₋₄ haloalkyl, C₂₋₄ alkenyl, C₂₋₄ alkynyl, C₃₋₈ cycloalkyl, 3- to 12-membered heterocyclyl, aryl, heteroaryl, CN, NO₂, OR^{e}, SR^{e}, NR^{e}R^{e}, C(O)R^{h}, C(O)OR^{e}, C(O)NR^{e}R^{e}, NR^{e}C(O)R^{h}, S(O)₂R^{h}, and NR^{e}S(O)₂R^{h}, provided that the resulting chemical structure is stable and meaningful; wherein R^{e} and R^{h} are as defined above.
unless otherwise specified, the aforementioned aryl is an aromatic group containing 6-12 carbon atoms; heteroaryl is a 5- to 15-membered heteroaromatic group; cyclic structure is a saturated or unsaturated cyclic group, optionally containing heteroatoms.

2. The compound according to claim 1, **characterized in that** formula (I) is formula (IIIa) or formula (IIIb): 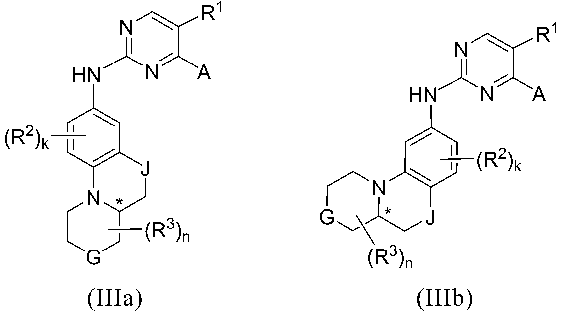 wherein the definitions of the groups in Formula (IIIa) and Formula (IIIb) are as defined in claim 1.

3. The compound according to any one of claims 1-2, **characterized in that** Formula (I) is Formula (IVa), Formula (IVb), Formula (IVc) or Formula (IVd): 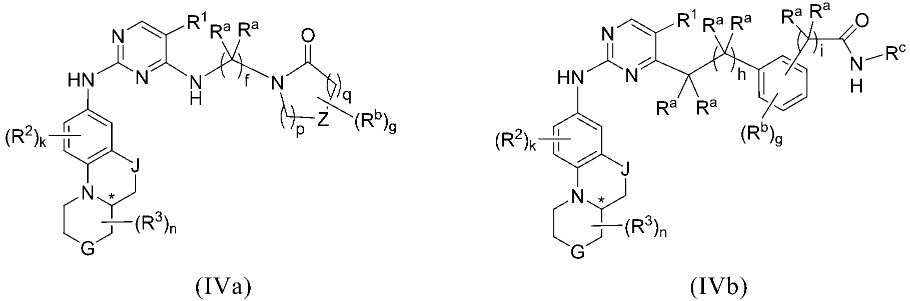 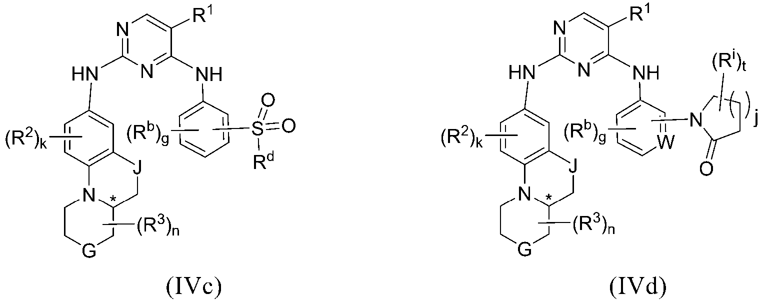 wherein the groups in Formula (IVa), Formula (IVb), Formula (IVc) and Formula (IVd) are as defined in claim 1.

4. The compound according to any one of claims 1-3, **characterized in that** Formula (I) is Formula (V): 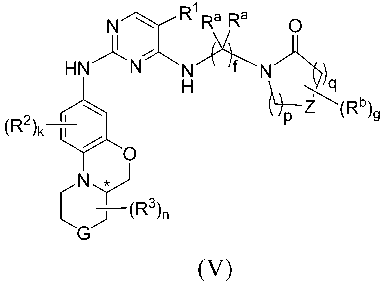 wherein the groups in Formula (V) are as defined in claim 1.

5. The compound according to any one of claims 1-4, **characterized in that** Formula (I) is Formula (VI): 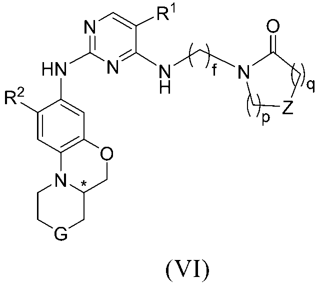
R¹ is selected from hydrogen, halogen, C₁₋₄ alkyl and C₁₋₄ haloalkyl;
R² is selected from hydrogen, halogen, C₁₋₄ alkyl, C₁₋₄ haloalkyl, C₃₋₆ cycloalkyl and O R^{e};
G is selected from NRf, O and CR^{g}R^{g};
Z is selected from O, NR^{e} and CH₂;
R^{f} is hydrogen, C₁₋₄ alkyl, C₃₋₈ cycloalkyl, 3- to 12-membered heterocyclyl (preferably 3- to 8-membered heterocyclyl), aryl, heteroaryl, C(O)R^{h}, C(O)OR^{e}, C(O)NR^{e}R^{e}, S(O)₂R^{h}, or S(O)₂NR^{e}R^{e}; wherein said alkyl, cycloalkyl, heterocyclyl, aryl, heteroaryl is optionally substituted by one or more substituents selected from the group consisting of halogen, C₁₋₄ alkyl, C₁₋₄ haloalkyl, C₃₋₆ cycloalkyl, 3- to 6-membered heterocyclyl, aryl, heteroaryl, OR^{e}, SR^{e}, NR^{e}R^{e}, CN, C(O)R^{h}, C(O)OR^{e}, C(O)NR^{e}R^{e}, NR^{e}C(O)R^{h}, S(O)₂R^{h}, S(O)₂NR^{e}R^{e} and NR^{e}S(O)₂R^{h};
each R^{g} is independently selected from the group consisting of hydrogen, halogen, C₁₋₄ alkyl, C₁₋₄ haloalkyl, OR^{e}, SR^{e}, NR^{e}R^{e}, CN, NR^{e}C(O)R^{h}, or NR^{e}S(O)₂R^{h};
each R^{e} is independently selected from hydrogen, C₁₋₄ alkyl, C₁₋₄ haloalkyl, C₃₋₆ cycloalkyl, or 3- to 6-membered heterocyclyl;
each R^{h} is independently selected from hydrogen, C₁₋₄ alkyl, C₁₋₄ haloalkyl, C₂₋₄ alkenyl, C₂₋₄ alkynyl, C₃₋₆ cycloalkyl, 3- to 6-membered heterocyclyl, aryl, heteroaryl;
p and q are each independently 0, 1, 2, 3 or 4;
f is 2, 3 or 4.

6. The compound according to any one of claims 1-3, **characterized in that** formula (I) is formula (VII): 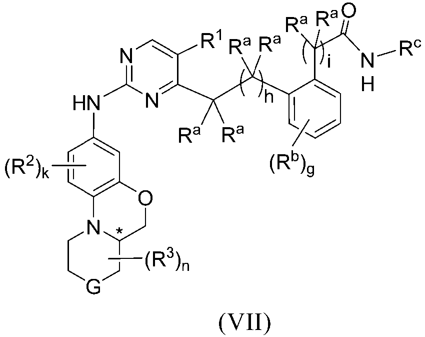 the groups in Formula (VII) are as defined in claim 1.

7. The compound according to any one of claims 1-3 and 6, **characterized in that** Formula (I) is Formula (VIII): 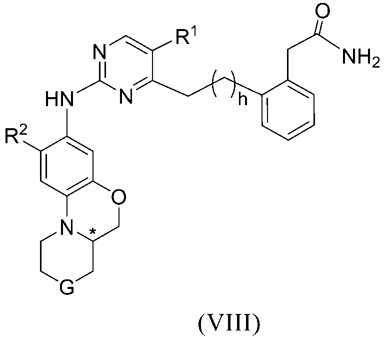
h is 0, 1, 2 or 3;
R¹, R² and G are defined as in claim 5.

8. The compound according to any one of claims 1-3, **characterized in that** Formula (I) is Formula (IX): 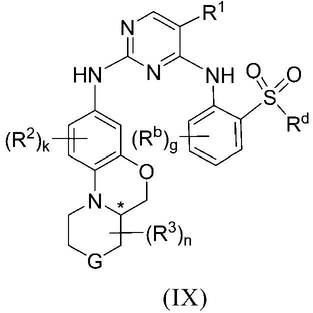 the groups in Formula (IX) are as defined in claim 1.

9. The compound according to any one of claims 1-3 and 8, **characterized in that** Formula (I) is Formula (X): 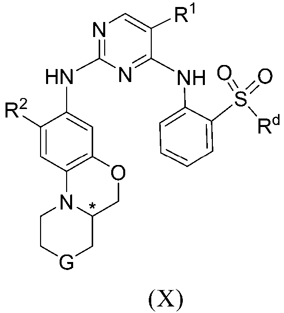
R^{d} is independently selected from C₂₋₄ alkenyl, C₂₋₄ alkynyl, C₃₋₆ cycloalkyl, and 3- to 6-membered heterocyclyl;
R¹, R² and G are as defined in claim 5.

10. The compound according to any one of claims 1-3, **characterized in that** Formula (I) is Formula (XI): 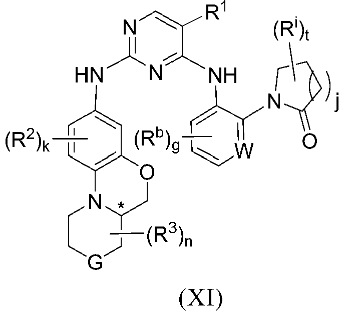 the groups in Formula (XI) are as defined in claim 1.

11. The compound according to any one of claims 1-3 and 10, **characterized in that** Formula (I) is Formula (XII): 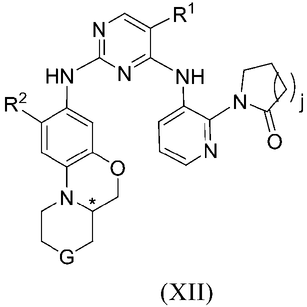
j is 0, 1, 2, 3 or 4;
R¹, R² and G are as defined in claim 5.

12. The compound according to claim 1, **characterized in that** Formula (I) is Formula (XIII): 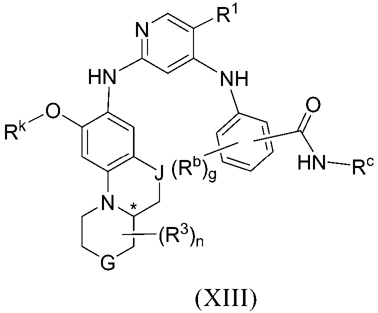 the groups in Formula (XIII) are as defined in claim 1.

13. The compound according to any one of claims 1 and 12, **characterized in that** Formula (I) is Formula (XIV): 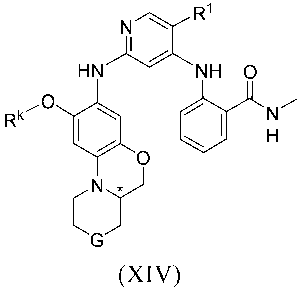
R^{k} is selected from hydrogen, C₁₋₄ alkyl, C₁₋₄ haloalkyl, C₃₋₆ cycloalkyl, or 3- to 6-membered heterocyclyl;
the R¹ and G are as defined in claim 5.

14. The compound according to claim 1, or an optical isomer, pharmaceutically acceptable salt, prodrug, deuterated derivative, hydrate, or solvate thereof, charactered in that the compound is selected from one of the following: 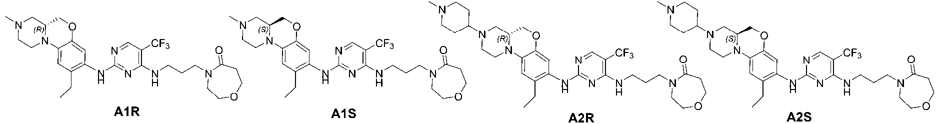 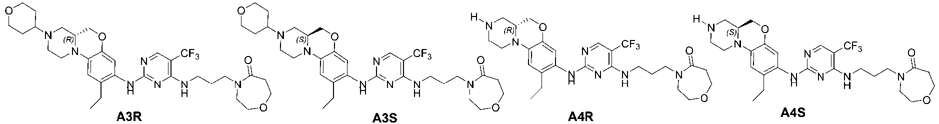 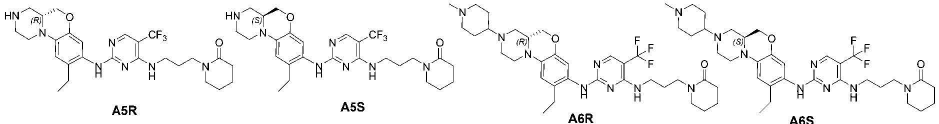 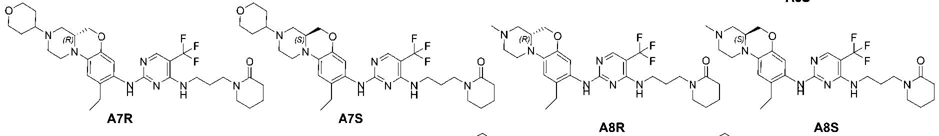 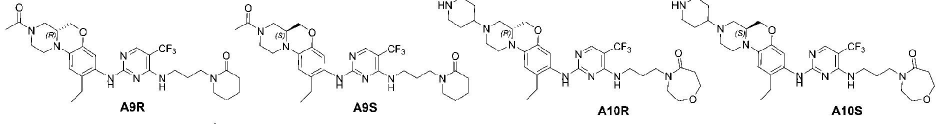 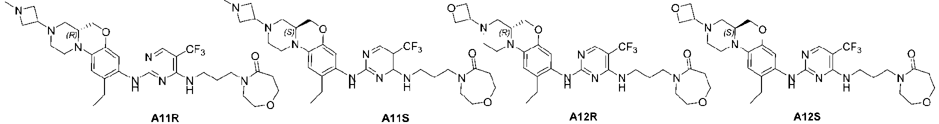 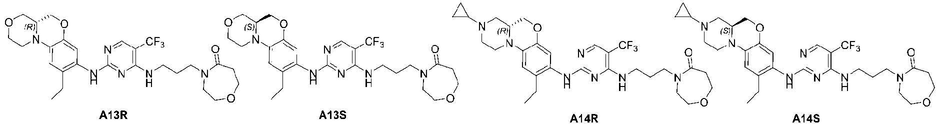 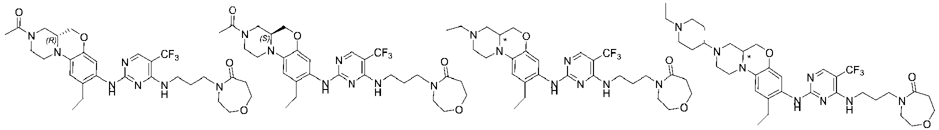 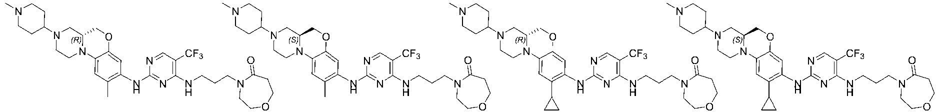 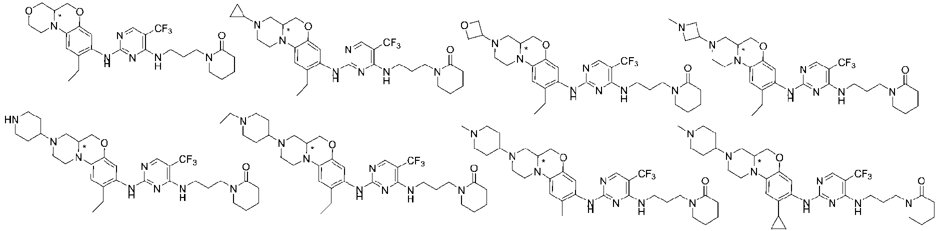 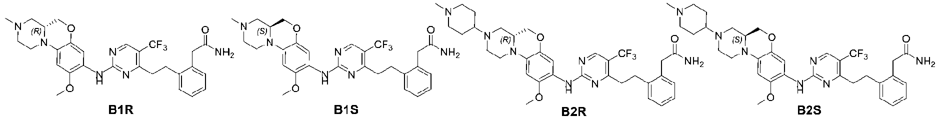 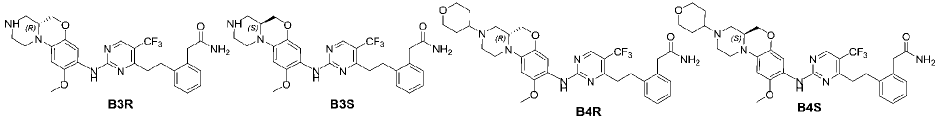 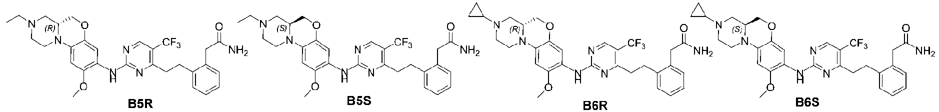 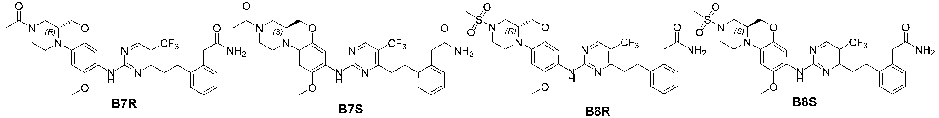 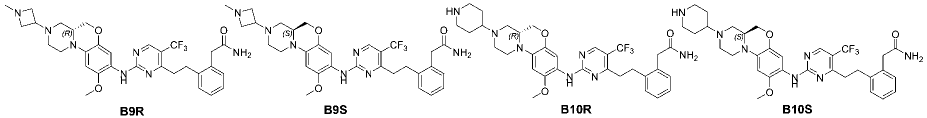 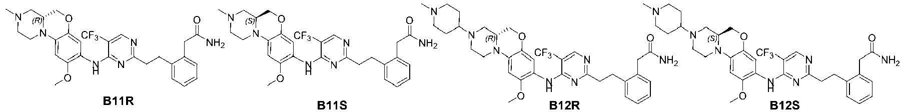 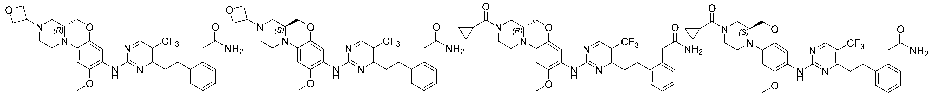 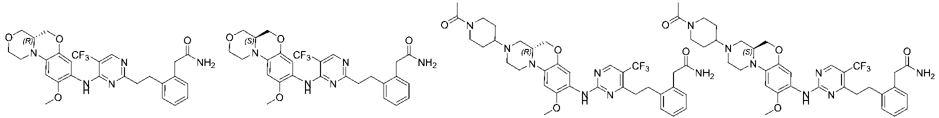 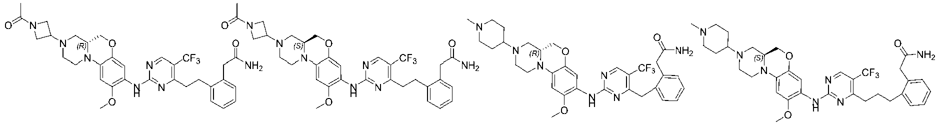 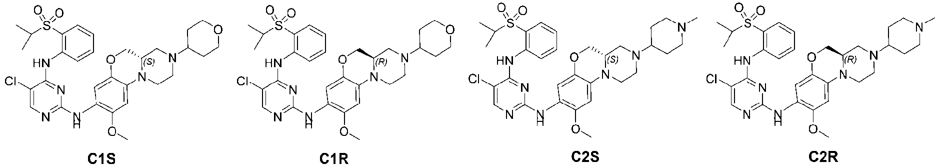 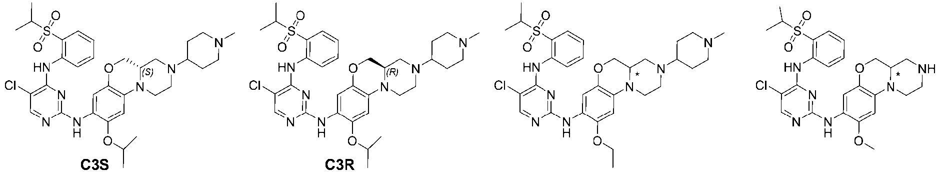 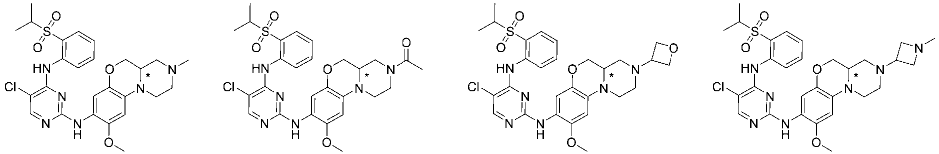 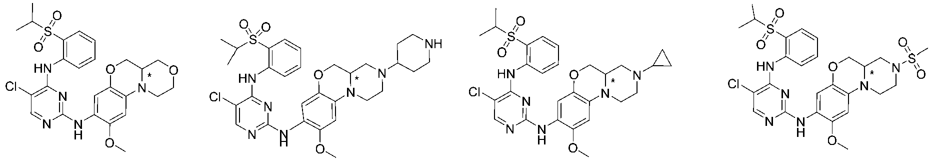 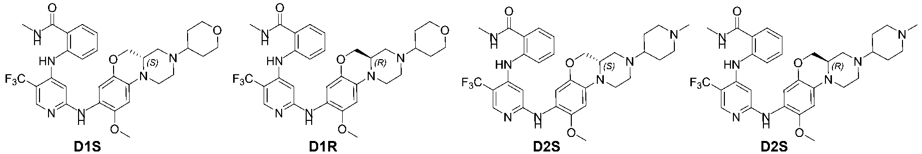 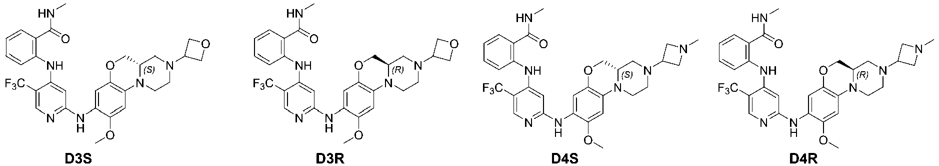 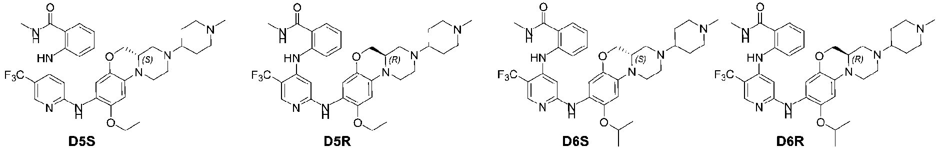 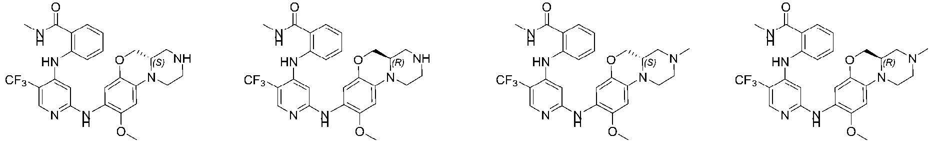 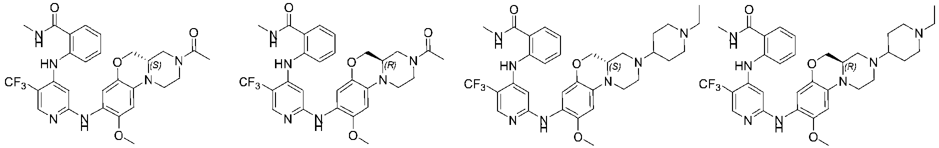 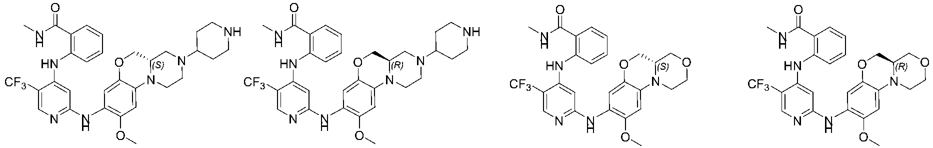 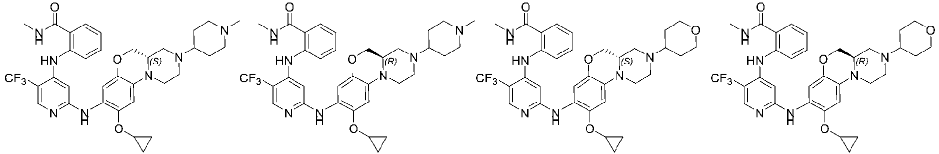 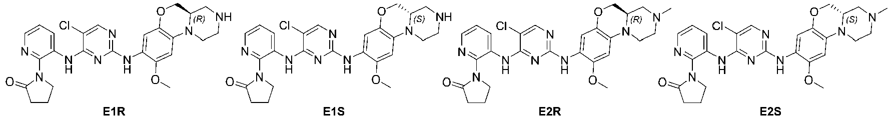 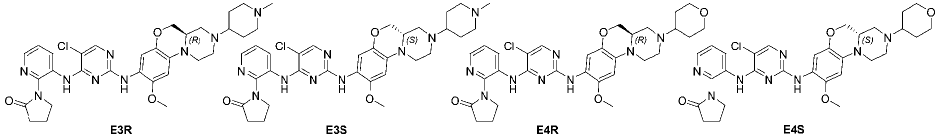 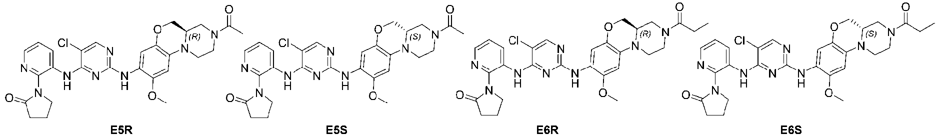 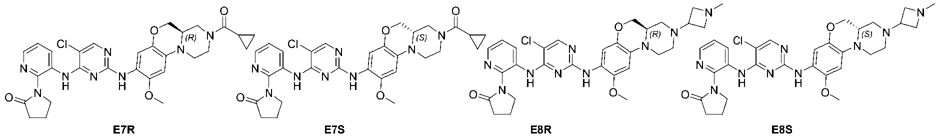 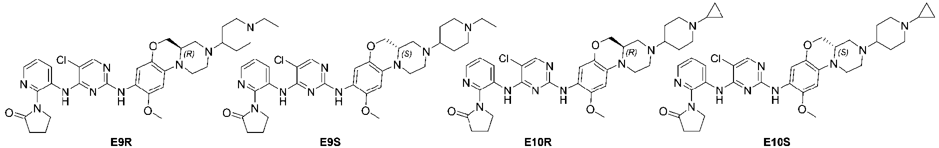 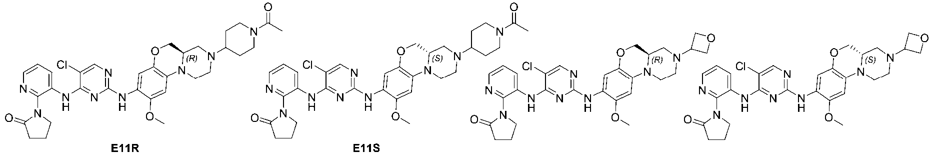 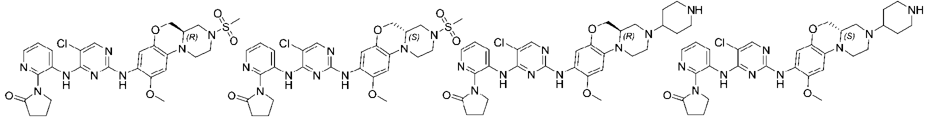 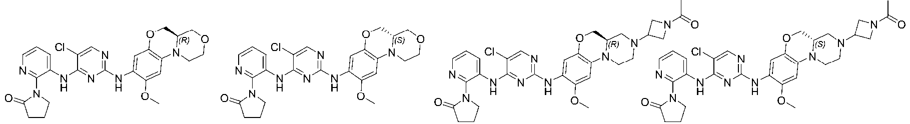 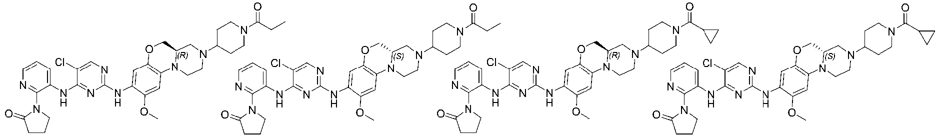 "*" denotes a chiral center in chemical structures.

15. A pharmaceutical composition comprising a compound according to any one of claims 1 to 14, or an optical isomer, pharmaceutically acceptable salt, prodrug, deuterated derivative, hydrate, or solvate thereof, and a pharmaceutically acceptable carrier.

16. A use of a compound according to any one of claims 1 to 14, or an optical isomer, pharmaceutically acceptable salt, prodrug, deuterated derivative, hydrate, or solvate thereof for use in the preparation of a pharmaceutical composition for treating a disease, disorder, or condition associated with kinase activity or expression level, including ULK, FAK, ALK, CDK7, HPK1, AXL, FLT3, TNK1, and the like.

17. The use according to claim 16, **characterized in that** said disease, disorder, or condition is selected from the group consisting of various other solid tumors and hematological malignancies, including breast cancer, non-small cell lung cancer (NSCLC), small cell lung cancer (SCLC), lung adenocarcinoma, lung squamous cell carcinoma, colon cancer, colorectal cancer, thyroid cancer, embryonal rhabdomyosarcoma, granular cell tumor of the skin, melanoma, liver cancer, rectal cancer, bladder cancer, laryngeal cancer, pancreatic cancer, prostate cancer, glioma, ovarian cancer, head and neck squamous cell carcinoma (HNSCC), cervical cancer, esophageal cancer, renal cancer, skin cancer, lymphoma, gastric cancer, mesothelioma, osteosarcoma, acute myeloid leukemia (AML), myelofibrosis, B-cell lymphoma, monocytic leukemia, splenomegalic polycythemia, hypereosinophilic syndrome, multiple myeloma; pulmonary fibrosis; DNA and RNA viral infections such as AIDS, herpesvirus infections, influenza virus infections, and the like.
